# EUROPÄISCHE PATENTSCHRIFT

(11) **EP 1 791 816 B1**
(45) Veröffentlichungstag und Bekanntmachung des Hinweises auf die Patenterteilung: **22.08.2012**
(21) Anmeldenummer: 05783004.4
(22) Anmeldetag: 13.09.2005
(51) Int. Cl.: C07D 207/36, C07D 209/54, C07D 491/10, C07D 495/10, C07D 307/60, C07D 307/94, C07D 493/10, C07C 233/52, C07C 233/47, C07D 311/00, C07C 255/29, C07D 487/04, C07D 237/04, C07C 57/58

(54) **JOD-PHENYLSUBSTITUIERTE CYCLISCHE KETOENOLE**
IODINE-PHENYL-SUBSTITUTED CYCLIC CETOENOLS
CETOENOLS CYCLIQUES A SUBSTITUTION IODE-PHENOL

(30) Priorität: 16.09.2004 DE 102004044827
(43) Veröffentlichungstag der Anmeldung: 06.06.2007
(73) Patentinhaber: Bayer CropScience AG, 40789 Monheim (DE)
(72) Erfinder: FISCHER, Reiner, 40789 Monheim (DE); BRETSCHNEIDER, Thomas, 53797 Lohmar (DE); ILG, Kerstin, 51061 Köln (DE); LEHR, Stefan, 65835 Liederbach (DE); FEUCHT, Dieter, 65760 Eschborn (DE); MALSAM, Olga, 51503 Rösrath (DE); RECKMANN, Udo, 50823 Köln (DE); BOJACK, Guido, 65207 Wiesbaden (DE); ARNOLD, Christian, 40764 Langenfeld (DE); AULER, Thomas, 42799 Leichlingen (DE); HILLS, Martin, Jeffrey, 65510 Idstein (DE); KEHNE, Heinz, 65719 Hofheim (DE); HEMPEL, Waltraud, 65835 Liederbach (DE); SANWALD, Erich, 24159 Kiel (DE)
(86) Internationale Anmeldenummer: PCT/EP2005/009807
(87) Internationale Veröffentlichungsnummer: WO 2006/029799

(56) Entgegenhaltungen:
- EP-A- 0 442 077
- EP-A- 0 668 267
- WO-A-00/47585
- WO-A-96/35664
- WO-A-97/02243
- WO-A-98/05638
- WO-A-98/25928
- WO-A-99/11605
- WO-A-99/24437
- WO-A-2004/000152
- WO-A-2004/064520
- WO-A-2004/065466
- WO-A-2004/080962
- WO-A-2004/111042
- BALTHAZOR T M; GODAR D E, STULTS B R: "Synthesis and Structure of Benzoindazoles" JOURNAL OF ORGANIC CHEMISTRY., Bd. 44, Nr. 9, 1979, Seiten 1447-1450, XP002355952 USAMERICAN CHEMICAL SOCIETY, WASHINGTON, DC.
- DATABASE CA [Online] CHEMICAL ABSTRACTS SERVICE, COLUMBUS, OHIO, US; DECKART, H. ET AL: "Pharmacokinetics of new radioiodine-labeled aromatic acids" XP002355954 gefunden im STN Database accession no. 1975:453382 & RADIOBIOLOGIA, RADIOTHERAPIA , 15(1), 27-38 CODEN: RDBGAT; ISSN: 0033-8184, 1974,
- CARSON J R ET AL: "2-ETHYNYLBENZENEALKANAMINES. A NEW CLASS OF CALCIUM ENTRY BLOCKERS" JOURNAL OF MEDICINAL CHEMISTRY, AMERICAN CHEMICAL SOCIETY. WASHINGTON, US, Bd. 31, 1988, Seiten 630-636, XP001091267 ISSN: 0022-2623
- BRESLOW R ET AL: "SELECTIVE HALOGENATION OF STEROIDS USING ATTACHED ARYL IODIDE TEMPLATES" JOURNAL OF THE AMERICAN CHEMICAL SOCIETY, AMERICAN CHEMICAL SOCIETY, WASHINGTON, DC, US, Bd. 99, Nr. 3, 1977, Seiten 905-915, XP001122053 ISSN: 0002-7863

## Beschreibung

Die vorliegende Erfindung betrifft neue Jod-phenylsubstituierte cyclische Ketoenole, mehrere Verfahren zu ihrer Herstellung und ihre Verwendung als Schädlingsbekämpfungsmittel und/oder Herbizide. Auch Gegenstand der Erfindung sind selektiv herbizide Mittel, die Jod-phenylsubstituierte cyclische Ketoenole einerseits und eine die Kulturpflanzenverträglichkeit verbessernde Verbindung andererseits enthalten.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869 und WO 99/55673, WO 01/17972, WO 01/23354, WO 01/74770, WO 03/013249, WO 04/007448, WO 04/024688, WO 04/065366, WO 04/080962, WO 04/111042, WO 05/044791, WO 05/044796, WO 05/048710, WO 05/049596, DE-A-04 001 433.

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354 und WO 01/74770, WO 03/013 249, WO 04/024 688, WO 04/080 962, WO 04/111 042 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673, WO 01/ 17972, WO 01/23354, WO 01/74770, WO 03/013249, WO 04/080 962, WO 04/111 042).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869, WO 99/55673, WO 01/17972, WO 01/74770, WO 03/013249, WO 04/080 962, WO 04/111 042 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869, WO 99/55673, WO 01/17972, WO 01/74770, WO 03/013249, WO 04/080 962, WO 04/111 042 beschrieben.

Es ist bekannt, dass bestimmte substituierte 2-Arylcyclopentandione herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673, WO 01/17972, WO 01/74770, WO 03/013249, WO 04/080 962, WO 04/111 042). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, dass bestimmte substituierte 2-Arylcyclohexandione herbizide, insektizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673, WO 01/17972, WO 01/74770, WO 03/013249, WO 04/080 962, WO 04/111 042).

Es ist bekannt, dass bestimmte substituierte 4-Aryl-pyrazolidin-3,5-dione akarizide, insektizide und herbizide Eigenschaften besitzen (vgl. z.B. WO 92/16 5 10, EP-A-508 126, WO 96/11 574, WO 96/21 652, WO 99/47525, WO 01/17351, WO 01/17 352, WO 01/17353, WO 01/17 972, WO 01/17 973, WO 03/028 466, WO 03/062 244, WO 04/080 962, WO 04/111 042, WO 05/005428, WO 05/016873).

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 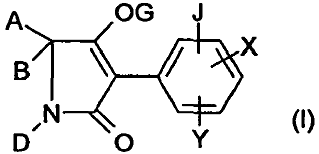 in welcher G für Wasserstoff steht und J, X, Y, D, A und B die in der Tabelle angegebenen Bedeutungen haben:

| **J** | **X** | **Y** | **D** | **A** | **B** |
|---|---|---|---|---|---|
| 4-J | 2-C₂H₅ | 6-CH₃ | H | -(CH₂)₂-CHOCH₃-(CH₂)₂- | |
| 2-J | 4-CH₃ | 6-CH₃ | H | -(CH₂)₂-CHCH₃-(CH₂)₂- | |
| 2-J | 4-Cl | 6-CH3 | H | -(CH₂)₂-CHOCH₃-(CH₂)₂- | |
| 2-J | 5-CH3 | H | H | -(CH₂)₂-CHOCH₃-(CH₂)₂- | |
| 2-J | 5-CH₃ | H | H | -(CH₂)₂-O-(CH₂)₂- | |
| 2-J | 4-Cl | 6-C₂H₅ | H | -(CH₂)₂-O-(CH₂)₂- | |
| 2-J | 4-Cl | 6-C₂H₅ | H | -(CH₂)₂-CHOCH₃-(CH₂)₂- | |
| 2-J | H | H | H | -(CH₂)₂-CHOCH₃-(CH₂)₂- | |
| 2-J | 4-CH₃ | 6-CH3 | H | -(CH₂)₂-CHCH₃-(CH₂)₂- | |
| 2-J | 4-Cl | 6-CH3 | H | -(CH₂)₂-O-(CH₂)₂- | |
| 3-J | 6-CH3 | H | H | -(CH₂)₂-CHOCH₃-(CH₂)₂- | |
| 4-J | 2-C₂H₅ | 6-CH3 | H | -(CH₂)₂-O-(CH₂)₂- | |
| 4-J | 2-C₂H₅ | 6-CH₃ | H | CH₃ | CH₃ |
| 4-J | 2-C₂H₅ | 6-CH3 | H | 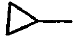 | CH₃ |
| 4-J | 2-C₂H₅ | 6-CH₃ | H | n-C₃H₇ | CH₃ |
| 4-J | 2-C₂H₅ | 6-CH₃ | 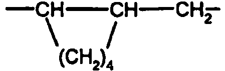 | | H |
| 4-J | 2-C₂H₅ | 6-CH₃ | H | -(CH₂)₅- | |
| 4-J | 2-C₂H₅ | 6-CH₃ | H | -(CH₂)₂-CHCH₃-(CH₂)₂- | |
| 4-J | 2-CH₃ | 6-CH₃ | H | -(CH₂)₂-CHOCH₃-(CH₂)₂- | |
| 4-J | 2-CH₃ | 6-CH₃ | H | -(CH₂)₂-O-(CH₂)₂- | |
| 4-J | 2-C₂H₅ | 6-Cl | H | -(CH₂)₂-O-(CH₂)₂- | |
| 4-J | 2-CH₃ | 6-CH₃ | H | -CH₂-CHOC₄H₉-(CH₂)₃- | |
| 4-J | 2-CH3 | 6-Cl | H | -(CH₂)₂-O-(CH₂)₂- | |
| 4-J | 2-C₂H₅ | 6-CH₃ | H | -CH₂-CHOC₄H₉-(CH₂)₃- | |
| 4-J | 2-C₂H₅ | 6-Cl | H | -(CH₂)₂-CHOCH₃-(CH₂)₂- | |
| 4-J | 2-CH₃ | 6-Cl | H | -(CH₂)₂-CHOCH₃-(CH₂)₂- | |
| 4-J | 2-C₂H₅ | 6-CH₃ | H | i-C₃H₇ | CH₃ |
| 4-J | 2-C₂H₅ | 6-CH₃ | H | i-C₄H₉ | CH₃ |

G für 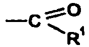 steht und J, X, Y, D, A, B und R¹ die in der Tabelle angegebenen Bedeutungen haben:

| **J** | **X** | **Y** | **D** | **A** | **B** | **R¹** |
|---|---|---|---|---|---|---|
| 2-J | 5-CH3 | H | H | -(CH₂)₂-CHOCH₃-(CH₂)₂- | | i-C₃H₇ |
| 2-J | 4-Cl | 6-C₂H₅ | H | -(CH₂)₂-CHOCH₃-(CH₂)₂- | | H₃C-O-CH₂- |
| 2-J | 4-Cl | 6-C₂H₅ | H | -(CH₂)₂-O-(CH₂)₂- | | H₃C-O-CH₂- |
| 4-J | 2-C₂H₅ | 6-CH₃ | H | -(CH₂)₂-O-(CH₂)₂- | | i-C₃H₇ |
| 4-J | 2-C₂H₅ | 6-CH₃ | H | C₃H₇ | CH₃ | i-C₃H₇ |
| 4-J | 2-CH₃ | 6-CH₃ | H | -CH₂-CH-OC₄H₉-(CH₂)₃- | | H₃C-O-CH₂- |
| 4-J | 2-CH₃ | 6-CH₃ | H | -CH₂-CHOC₄H₉-(CH₂)₃- | | 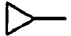 |
| 4-J | 2-CH₃ | 6-CH₃ | H | -CH₂-CHOC₄H₉-(CH₂)₃- | | Cl-CH₂- |
| 4-J | 2-CH₃ | 6-CH₃ | H | -CH₂-CHOC₄H₉-(CH₂)₃- | | 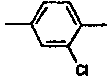 |
| 4-J | 2-C₂H₅ | 6-CH₃ | H | -(CH₂)₂-O-(CH₂)₂- | | H₃C-O-CH₂- |
| 4-J | 2-C₂H₅ | 6-Cl | H | -(CH₂)₂-O-(CH₂)₂- | | H₃C-O-CH₂- |
| 4-J | 2-C₂H₅ | 6-CH₃ | H | -CH₂-CHOC₄H₉-(CH₂)₃- | | i-C₃H₇ |
| 4-J | 2-C₂H₅ | 6-CH₃ | H | i-C₃H₇ | CH₃ | i-C₃H₇ |
| 4-J | 2-C₂H₅ | 6-CH₃ | H | -CH₂-CHOC₄H₉-(CH₂)₃- | | H₃C-O-CH₂- |
| 4-J | 2-C₂H₅ | 6-CH₃ | H | -(CH₂)₅- | | H₃C-O-CH₂- |
| 4-J | 2-C₂H₅ | 6-CH₃ | H | -(CH₂)₅- | | i-C₃H₇ |
| 4-J | 2-C₂H₅ | 6-CH₃ | H | CH₃ | CH₃ | i-C₃H₇ |
| 4-J | 2-C₂H₅ | 6-CH₃ | H | -(CH2)5- | | t-C₄H₉ |
| 4-J | 2-C₂H₅ | 6-CH₃ | H | i-C₃H₇ | CH₃ | H₃C-O-CH₂- |
| 4-J | 2-C₂H₅ | 6-CH₃ | H | CH₃ | CH₃ | H₃C-O-CH₂- |
| 4-J | 2-C₂H₅ | 6-CH₃ | H | i-C₃H₇ | CH₃ | i-C₃H₇ |
| 2-J | 5-CH3 | H | H | -(CH₂)₂-CHOCH₃-(CH₂)₂- | | 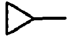 |

G für 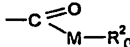 teht und J, X, Y, D, A, B M und R² die in der Tabelle angegebenen Bedeutungen haben:

| **J** | **X** | **Y** | **D** | **A** | **B** | **M** | **R²** |
|---|---|---|---|---|---|---|---|
| 2-J | 5-CH₃ | H | H | -(CH₂)₂-CHOCH₃-(CH₂)₂- | | O | C₆H₅-CH₂- |
| 2-J | 4-Cl | 6-CH₃ | H | -(CH₂)₂-CHOCH₃-(CH₂)₂- | | O | C₂H₅ |
| 2-J | 5-CH₃ | H | H | -(CH₂)₂-CHOCH₃-(CH₂)₂- | | O | C₂H₅ |
| 2-J | 4-Cl | 6-C₂H₅ | H | -(CH₂)₂-O-(CH₂)₂- | | O | C₂H₅ |
| 2-J | 4-Cl | 6-C₂H₅ | H | -(CH₂)₂-CHOCH₃-(CH₂)₂- | | O | C₂H₅ |
| 3-J | 6-CH₃ | H | H | -(CH₂)₂-CHOCH₃-(CH₂)₂- | | O | C₂H₅ |
| 4-J | 2-CH₃ | 6-CH₃ | H | -(CH₂)₂-CHOCH₃-(CH₂)₂- | | O | C₂H₅ |
| 4-J | 2-CH₃ | 6-Cl | H | -(CH₂)₂-O-(CH₂)₂- | | O | C₂H₅ |
| 4-J | 2-C₂H₅ | 6-CH₃ | H | n-C₃H₇ | CH₃ | O | C₂H₅ |
| 4-J | 2-C₂H₅ | 6-CH₃ | H | 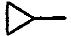 | CH₃ | O | C₂H₅ |
| 4-J | 2-C₂H₅ | 6-CH₃ | H | -(CH₂)₂-O-(CH₂)₂- | | O | C₂H₅ |
| 4-J | 2-C₂H₅ | 6-Cl | H | -(CH₂)₂-O-(CH₂)₂- | | O | C₂H₅ |
| 4-J | 2-CH₃ | 6-CH₃ | H | -CH₂-CHOC₄H₉-(CH₂)₃- | | O | C₆H₅-CH₂- |
| 4-J | 2-CH₃ | 6-CH₃ | H | -CH₂-CHOC₄H₉-(CH₂)₃- | | O | CH₂=CH-CH₂- |
| 4-J | 2-CH₃ | 6-CH₃ | H | -CH₂-CHOC₄H₉-(CH₂)₃- | | O | C₂H₅ |
| 4-J | 2-C₂H₅ | 6-CH₃ | H | -(CH₂)₅- | | O | C₂H₅ |
| 4-J | 2-C₂H₅ | 6-CH₃ | H | CH₃ | CH₃ | O | C₂H₅ |
| 4-J | 2-C₂H₅ | 6-CH₃ | H | i-C₄H₉ | CH₃ | O | C₂H₅ |
| 4-J | 2-CH₃ | 6-CH₃ | H | -(CH₂)₂-O-(CH₂)₂- | | O | C₂H₅ |
| 4-J | 2-C₂H₅ | 6-CH₃ | H | -(CH₂)₂-CHOCH₃-(CH₂)₂- | | O | C₂H₅ |

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), 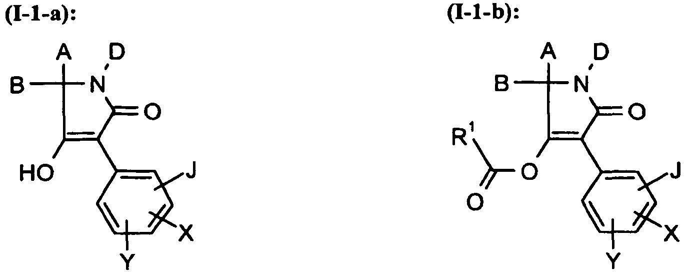 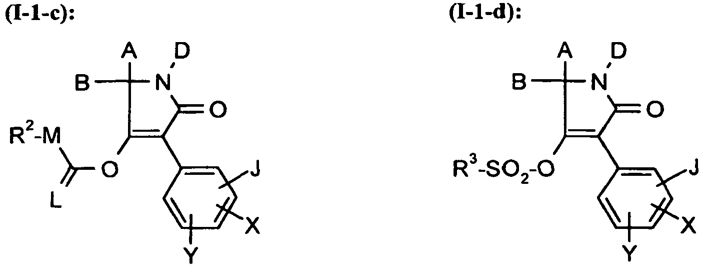 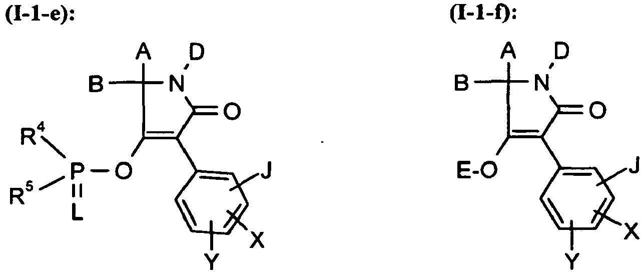 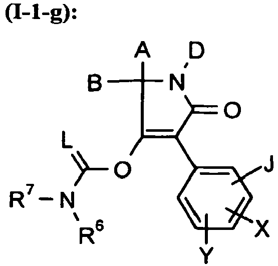 worin
A, B, D, E, J, L, M, X, Y, R¹, R², R³, R⁴, R⁵, R⁶ und R⁷ die oben angegebenen Bedeutungen besitzen.

Weiterhin wurde gefunden, dass man die neuen Verbindungen der Formel (I) nach einem der im folgenden beschriebenen Verfahren erhält:

### (A) Man erhält substituierte 3-Phenylpyrrolidin-2,4-dione bzw. deren Enole der Formel (I-1-a)

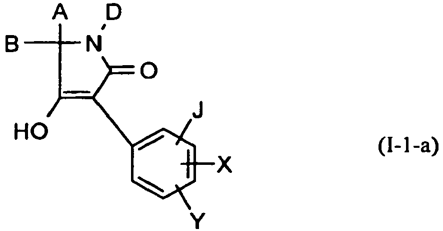

in welcher
- A, B, D, J, X und Y: die oben angegebenen Bedeutungen haben,
wenn man
N-Acylaminosäureester der Formel (II) 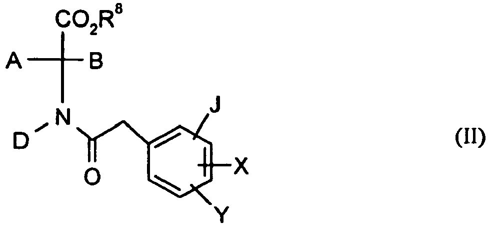 in welcher
- A, B, D, J, X und Y: die oben angegebenen Bedeutungen haben,
und
- R⁸: für Alkyl (bevorzugt C₁-C₆-Alkyl) steht,
in Gegenwart eines Verdünnungsmittels und in Gegenwart einer Base intramolekular kondensiert.

### Außerdem wurde gefunden

(I) dass man die Verbindungen der oben gezeigten Formel (I-1-b), in welchen A, B, D, J, R¹, X, und Y die oben angegebenen Bedeutungen haben, erhält, wenn man Verbindungen der oben gezeigten Formel (I-1-a), in welchen A, B, D, J, X und Y die oben angegebenen Bedeutungen haben, jeweils
(α) mit Säurehalogeniden der Formel (XIII) 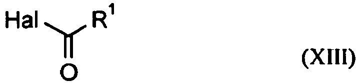 in welcher
   - R¹: die oben angegebene Bedeutung hat und
   - Hal: für Halogen (insbesondere Chlor oder Brom) steht
   oder
(β) mit Carbonsäureanhydriden der Formel (XIV)

   R¹-CO-O-CO-R¹ (XIV)

   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(J) dass man die Verbindungen der oben gezeigten Formel (I-1-c), in welchen A, B, D, J, R², M, X und Y die oben angegebenen Bedeutungen haben und L für Sauerstoff steht, erhält, wenn man Verbindungen der oben gezeigten Formel (I-1-a), in welchen A, B, D, J, X und Y die oben angegebenen Bedeutungen haben, jeweils is
   mit Chlorameisensäureestern oder Chlorameisensäurethioestern der Formel (XV)

   R²-M-CO-Cl (XV)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(P) dass man Verbindungen der oben gezeigten Formel (I-1-a) in welchen A, B, D, J, X und Y die oben angegebene Bedeutung haben, erhält, wenn man Verbindungen der Formel (I-1-a'), in welchen A, B, D, X und Y die oben genannte Bedeutung haben und W' bevorzugt für Brom steht 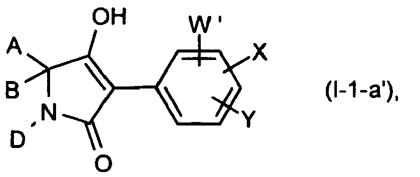
a) mit Metalliodiden (z.B. Natriumiodid oder Kaliumiodid) gegebenenfalls in Gegenwart eines Verdünnungsmittels, eines Cu-(I)-Salzes (z.B. CuBr, CuJ) und einer Base (z.B. N,N-Dimethylethylendiamin) umsetzt, oder
ß) mit Metallorganylen (z.B. n, s-, -Butyllithium) einen Halogenmetallaustausch durchführt und das entstehende Anion mit Jodierungsreagenzien (z.B. Iod, Iodmonochlorid) quencht.

Weiterhin wurde gefunden, dass die neuen Verbindungen der Formel (I) eine sehr gute Wirksamkeit als Schädlingsbekämpfungsmittel, vorzugsweise als Insektizide, Akarizide und/oder Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind auch selektiv-herbizide Mittel enthaltend einen wirksamen Gehalt an einer Wirkstoffkombination umfassend als Komponenten
(a') mindestens ein substituiertes, cyclisches Ketoenol der Formel (I), in welcher CKE, J, X und Y die oben angegebene Bedeutung haben
   und
(b') zumindest eine die Kulturpflanzen-Verträglichkeit verbessernde Verbindung aus der folgenden Gruppe von Verbindungen:
   4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyloxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlorphenoxy-essigsäure, 3,3'-Dimethyl-4-methoxy-benzophenon, 1-Brom-4-chlormethylsulfonylbenzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-hamstoff, 1-[4-(N-Naphthylsulfamoyl)-phenyl]-3,3-dimethyl-hamstoff, N-(2-Methoxy-5-methyl-benzoyl)-4-(cyclopropylaminocarbonyl)-benzolsulfonamid,
   und/oder eine der folgenden durch allgemeine Formeln definierten Verbindungen
   der allgemeinen Formel (IIa) 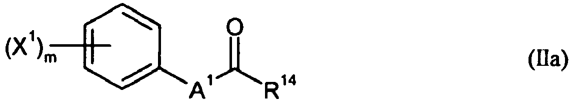 oder der allgemeinen Formel (IIb) 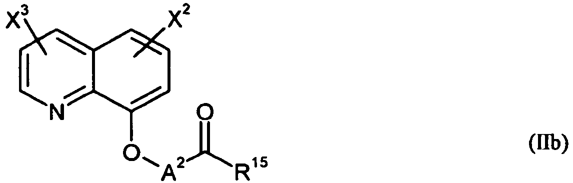 oder der Formel (IIc) 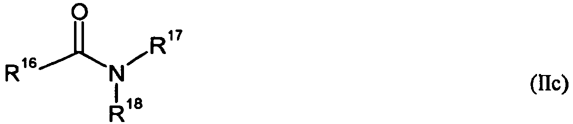 wobei
   - m: für eine Zahl 0, 1, 2, 3, 4 oder 5 steht,
   - A¹: für eine der nachstehend skizzierten divalenten heterocyclischen Gruppierungen steht, 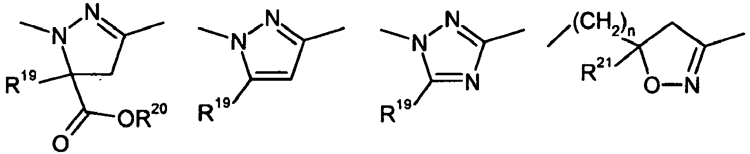
   - n: für eine Zahl 0, 1, 2, 3, 4 oder 5 steht,
   - A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
   - R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
   - R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
   - R¹⁶: für jeweils gegebenenfalls durch Fluor, Chlor und/oder Brom substituiertes C₁-C₄-Alkyl steht,
   - R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
   - R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
   - R¹⁷ und R¹⁸: auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Alkandiyl oder C₂-C₅-Oxaalkandiyl steht,
   - R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
   - R²⁰: für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
   - R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
   - X': für Nitro, Cyano, Halogen, C₁-C₄-Alkyl, C₁-C₄-Halogenalkyl, C₁-C₄-Alkoxy oder C₁-C₄-Halogenalkoxy steht,
   - X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
   - X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen
der allgemeinen Formel (IId) 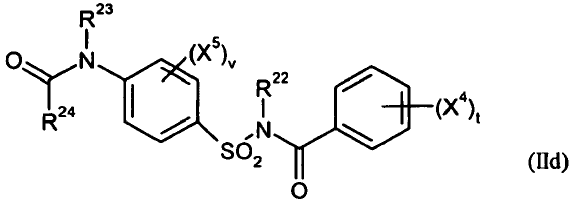 oder der allgemeinen Formel (IIe) 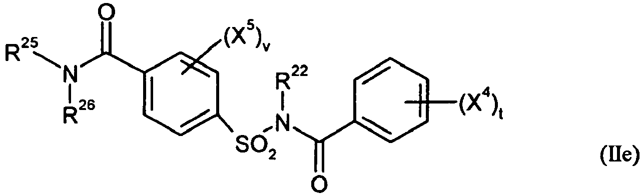 wobei
- t: für eine Zahl 0, 1, 2, 3, 4 oder 5 steht,
- v: für eine Zahl 0, 1, 2, 3, 4 oder 5 steht,
- R²²: für Wasserstoff oder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen beispielsweise die folgenden Verbindungen der Formel (I-1-a) genannt:

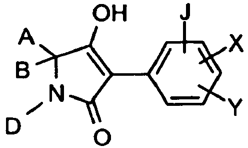

**Tabelle 1: 2 - J; X = H; Y = H.**

| | | |
|---|---|---|
| A | B | D |
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| S-C₄H₉ | CH₃ | H |
| t-C₄H₉ | CH₃ | H |
| C₂H₅ | C₂H₅ | H |
| C₃H₇ | C₃H₇ | H |
| 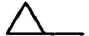 | CH₃ | H |

| A | B | D |
|---|---|---|
| 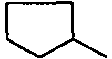 | CH₃ | H |
| 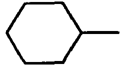 | CH₃ | H |
| -(CH₂)₂- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -CH₂-CHOCH₃-(CH₂)₃- | | H |
| -CH₂-CHOC₂H₅-(CH₂)₃- | | H |
| -CH₂-CHOC₃H₇-(CH₂)₃- | | H |
| -CH₂-CHOC₄H₉-(CH₂)₃- | | H |
| -CH₂-CHO-(CH₂)₂-OCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHO-i-C₃H₇-(CH₂)₂- | | H |
| -(CH₂)₂-C(CH₃)₂-(CH₂)₂- | | H |
| -CH₂-(CHCH₃)₂-(CH₂)₂- | | H |
| 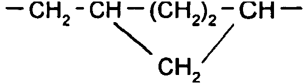 | | H |
| 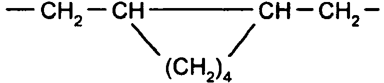 | | H |
| 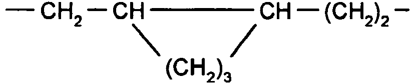 | | H |
| 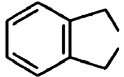 | | H |
| 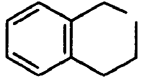 | | H |
| | | |

| A | D | B |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-CH(OCH₃)-CH₂- | | H |
| -CH₂-CH=CH-CH₂- | | H |
| 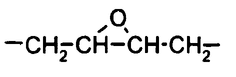 | | H |
| -CH₂-S-CH₂- | | H |
| -CH₂-S-(CH₂)₂- | | H |
| -(CH₂)₂-S-CH₂- | | H |
| 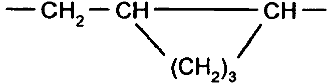 | | H |
| H | CH₃ | H |
| H | C₂H₅ | H |
| H | C₃H₇ | H |
| H | i-C₃H₇ | H |
| H | 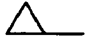 | H |
| H | 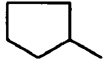 | H |
| H | 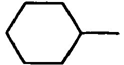 | H |
| CH₃ | CH₃ | H |
| CH₃ | C₂H₅ | H |
| CH₃ | C₃H₇ | H |
| CH₃ | i-C₃H₇ | H |
| CH₃ | 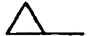 | H |
| CH₃ | 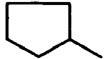 | H |
| CH₃ | 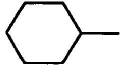 | H |
| C₂H₅ | CH₃ | H |
| C₂H₅ | C₂H₅ | H |

| | |
|---|---|
| **Tabelle 2:** | A, B und D wie in Tabelle 1 angegeben |
| | 2-J; X = 4-CH₃; Y = H |

| | |
|---|---|
| **Tabelle 3:** | A, B und D wie in Tabelle 1 angegeben |
| | 2-J; X = 6-CH₃; Y = H. |

| | |
|---|---|
| **Tabelle 4:** | A, B und D wie in Tabelle 1 angegeben |
| | 2 - J; X = 6-C₂H₅; Y = H. |

| | |
|---|---|
| **Tabelle 5:** | A, B und D wie in Tabelle 1 angegeben |
| | X = 2-CH₃; Y = H; 5 - J. |

| | |
|---|---|
| **Tabelle 6:** | A, B und D wie in Tabelle 1 angegeben |
| | X = 2-CH₃; Y = 4-CH₃; 5 - J. |

| | |
|---|---|
| **Tabelle 7:** | A, B und D wie in Tabelle 1 angegeben |
| | 2 - J; X = 4-CH₃; Y = 6-CH₃. |

| | |
|---|---|
| **Tabelle 8:** | A, B und D wie in Tabelle 1 angegeben |
| | 2 - J; X = 6-C₂H₅; Y = 4-CH₃. |

| | |
|---|---|
| **Tabelle 9:** | A, B und D wie in Tabelle 1 angegeben |
| | 2 - J; X = 6-CH₃; Y = 4-Cl. |
| **Tabelle 10:** | A, B und D wie in Tabelle 1 angegeben |
| | 2 - J; X = 6-C₂H₅; Y = 4-Cl. |

| | |
|---|---|
| **Tabelle 11:** | A, B und D wie in Tabelle 1 angegeben |
| | 2 - J; X = 6-Cl; Y = 4-CH₃. |

| | |
|---|---|
| **Tabelle 12:** | A, B und D wie in Tabelle 1 angegeben |
| | 2 - J; X = 5-CH₃; Y = 4-CH₃. |

| | |
|---|---|
| **Tabelle 13:** | A, B und D wie in Tabelle 1 angegeben |
| | X = 2-CH₃; 4 - J; Y = H. |

| | |
|---|---|
| **Tabelle 14:** | A, B und D wie in Tabelle 1 angegeben |
| | X = 2-C₂H₅; 4 - J; Y = H. |

| | |
|---|---|
| **Tabelle 15:** | A, B und D wie in Tabelle 1 angegeben |
| | X = 2-CH₃; 4 - J; Y = 6-CH₃. |

| | |
|---|---|
| **Tabelle 16:** | A, B und D wie in Tabelle 1 angegeben |
| | X = 2-C₂H₅; 4 - J; Y = 6- CH₃. |

| | |
|---|---|
| **Tabelle 17:** | A, B und D wie in Tabelle 1 angegeben |
| | X = 2-C₂H₅; 4 - J; Y = 6-C₂H₅. |

| | |
|---|---|
| **Tabelle 18:** | A, B und D wie in Tabelle 1 angegeben |
| | X = 2-Cl; 4 - J; Y = 6-CH₃. |

| | |
|---|---|
| **Tabelle 19:** | A, B und D wie in Tabelle 1 angegeben |
| | X = 2-Cl; 4 - J; Y = 6-C₂H₅. |

| | |
|---|---|
| **Tabelle 20:** | A, B und D wie in Tabelle 1 angegeben |
| | X = 2-CH₃; 4 - J; Y = 5-CH₃. |

| | |
|---|---|
| **Tabelle 21:** | A, B und D wie in Tabelle 1 angegeben |
| | X = 2-CH₃; 3 - J; Y = 6-CH₃. |

| | |
|---|---|
| **Tabelle 22:** | A, B und D wie in Tabelle 1 angegeben |
| | 2 - J; X = 5-CH₃; Y = H. |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen 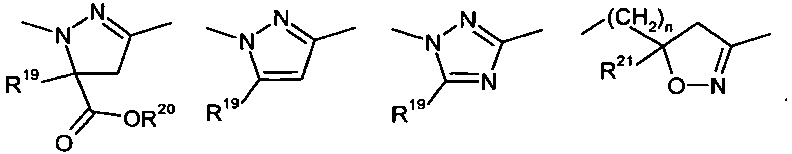
- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxy-carbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamine, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexaloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.
- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder zusammen mit R¹⁷ für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s-oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s-oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- x²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2 oder 3.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle Beispiele für die Verbindungen der Formel (IIa)**

| | | | |
|---|---|---|---|
| 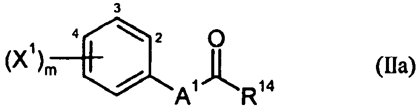 | | | |

| **Beispiel-Nr.** | **(Positionen) (X¹)ₘ,** | **A¹** | **R¹⁴** |
|---|---|---|---|
| IIa-1 | (2) Cl, (4) Cl | 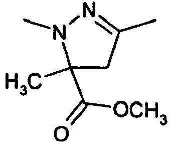 | OCH₃ |
| IIa-2 | (2) Cl, (4) Cl | 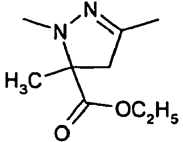 | OCH₃ |
| IIa-3 | (2) Cl, (4) CI | 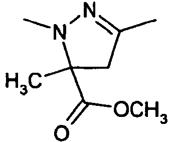 | OC₂H₅ |
| IIa-4 | (2) Cl, (4) Cl | 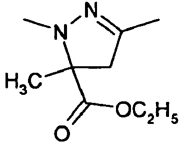 | 0C₂H₅ |
| IIa-5 | (2) Cl | 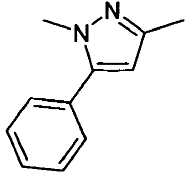 | OCH₃ |
| IIa-6 | (2) Cl, (4) Cl | 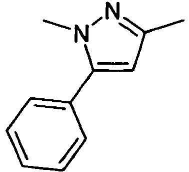 | OCH₃ |
| IIa-7 | (2) F | 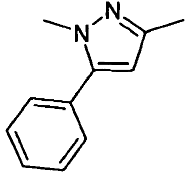 | OCH₃ |

| **Beispiel-Nr.** | **(Positionen) (X¹)ₘ** | **A¹** | **R¹⁴** |
|---|---|---|---|
| IIa-8 | (2) F | 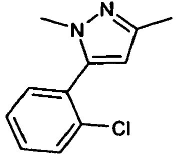 | OCH₃ |
| IIa-9 | (2) Cl, (4) Cl | 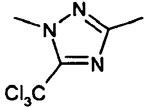 | OC₂H₅ |
| IIa-10 | (2) Cl, (4) CF₃ | 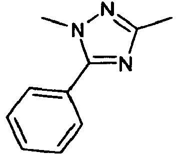 | OCH₃ |
| IIa-11 | (2) Cl | 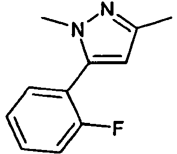 | OCH₃ |
| IIa-12 | - | 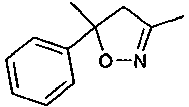 | OC₂H₅ |
| IIa-13 | (2) Cl, (4) Cl | 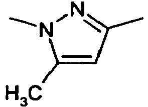 | OC₂H₅ |
| IIa-14 | (2) Cl, (4) Cl | 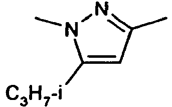 | OC₂H₅ |
| IIa-15 | (2) Cl, (4) Cl | 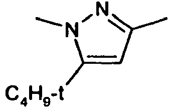 | OC₂H₅ |
| IIa-16 | (2) Cl, (4) Cl | 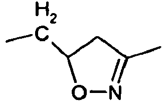 | OC₂H₅ |
| IIa-17 | (2) Cl, (4) Cl | 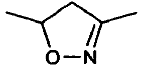 | OC₂H₅ |
| IIa-18 | - | 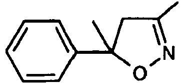 | OH |

Beispiele für die als erfindungsgemäße Herbizid-Safener ganz besonders bevorzugten Verbindungen der Formel (IIb) sind in der nachstehenden Tabelle aufgeführt.

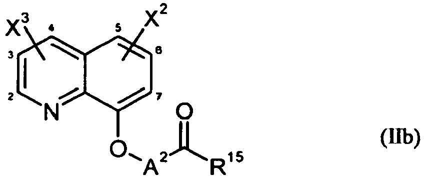

**Tabelle Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| Ilb-4 | (5) Cl | - | CH₂ | OC₃H₇-n |
| Ilb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH₌CH₂ |
| IIb-11 | (5) Cl | - | CH₂ | OC₄H₉-i |

| **Beispiel-Nr.** | **(Position) x²** | **(Position) X³** | **A²** | **R¹⁵** |
|---|---|---|---|---|
| IIb-12 | (5) Cl | - | CH₂ | 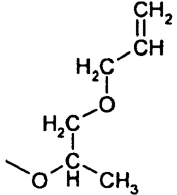 |
| IIb-13 | (5) Cl | - | 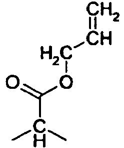 | OCH₂CH=CH₂ |
| IIb-14 | (5) Cl | - | 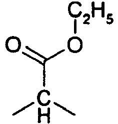 | OC₂H₅ |
| IIb-15 | (5) Cl | - | 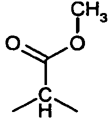 | OCH3 |

Beispiele für die als erfindungsgemäße Herbizid-Safener ganz besonders bevorzugten Verbindungen der Formel (IIc) sind in der nachstehenden Tabelle aufgeführt.

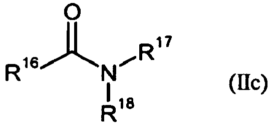

**Tabelle Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 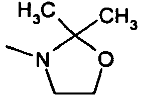 |
| IIc-3 | CHCl₂ | 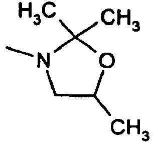 |
| IIc-4 | CHCl₂ | 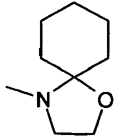 |
| IIc-5 | CHCl₂ | 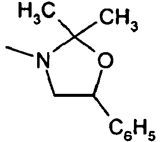 |
| IIc-6 | CHCl₂ | 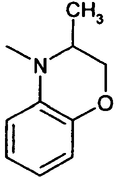 |
| IIc-7 | CHCl₂ | 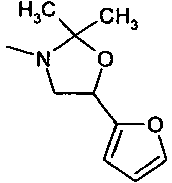 |

Beispiele für die als erfindungsgemäße Herbizid-Safener ganz besonders bevorzugten Verbindungen der Formel (IId) sind in der nachstehenden Tabelle aufgeführt.

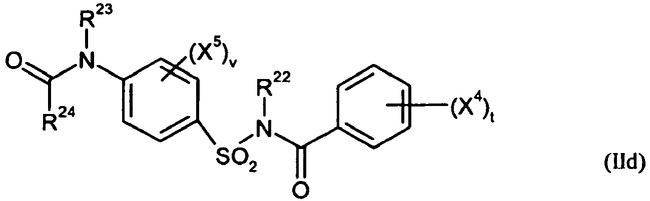

**Tabelle Beispiele für die Verbindungen der Formel (IId)**

| **Beispiel-Nr.** | **R²²** | **R²³** | **R²⁴** | **(Positionen) (X⁴),** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH₃ | - |
| IId-4 | H | H | C₃H₇-i | (2) OCH₃ | - |
| IId-5 | H | H | 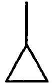 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇₋ₙ | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 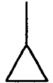 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 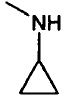 | (2) OCH₃ | - |
| | | | | (5) CH₃ | |
| IId-21 | H | H | NHCH₃ | (2) OCH₃ | - |

| **Beispiel-Nr.** | **R²²** | **R²³** | **R²⁴** | **(Positionen) (X⁴)ₜ** | **(Positionen) (X⁵)ᵥ** |
|---|---|---|---|---|---|
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

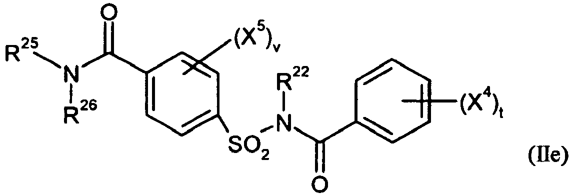

**Tabelle Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| Ile-4 | H | H | C₃H₇-i | (2) OCH₃ | - |
| IIe-5 | H | H | 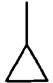 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| II-8 | H | H | C₂H₅ | (2) OCH₃ | - |
| | | | | (5) CH₃ | |
| IIe-9 | H | H | C₃H₇-n | (2) OCH₃ | - |
| | | | | (5) CH3 | |
| IIe-10 | H | H | C₃H₇-i | (2) OCH₃ | - |
| | | | | (5) CH₃ | |
| IIe-11 | H | H | 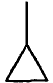 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522/US-A-6235680).

Die als Safener erfindungsgemäß zu verwendenden Verbindungen der allgemeinen Formel (IIe) sind bekannt und können nach an sich bekannten Verfahren hergestellt werden (vgl. WO-A-99/66795/US-A-6251827 A-6251827).

Beispiele für die erfindungsgemäßen selektiv herbiziden Kombinationen aus jeweils einem Wirkstoff der Formel (I) und jeweils einem der oben definierten Safener sind in der nachstehenden Tabelle auf geführt.

**Tabelle Beispiele für die erfindungsgemäßen Kombinationen**

| **Wirkstoffe der Formel (I)** | **Safener** |
|---|---|
| I-I | Cloquintocet-mexyl |
| I-1 | Fenchlorazole-ethyl |
| I-I | Isoxadifen-ethyl |
| I-I | Mefenpyr-diethyl |
| I-I | Furilazole |
| I-I | Fenclorim |
| I-I | Cumyluron |
| I-I | Daimuron /Dymron |
| I-I | Dimepiperate |
| I-I | IIe-11 |
| I-1 | IIe-5 |

Es wurde nun überraschend gefunden, dass die oben definierten Wirkstoffkombinationen aus lodphenyl substituierten cyclischen Ketoenole der allgemeinen Formel (I) und Safenem (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-(2,6-Dimethyl-4-iod-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

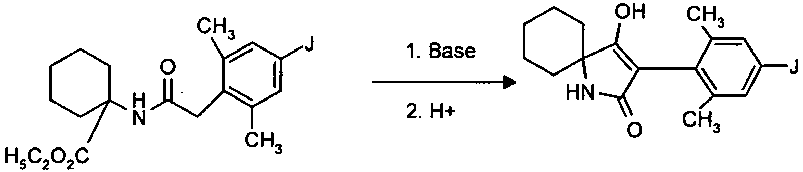

Verwendet man beispielsweise gemäß Verfahren (Iα) 3-(2-Methyl-4-iod-6-ethyl-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

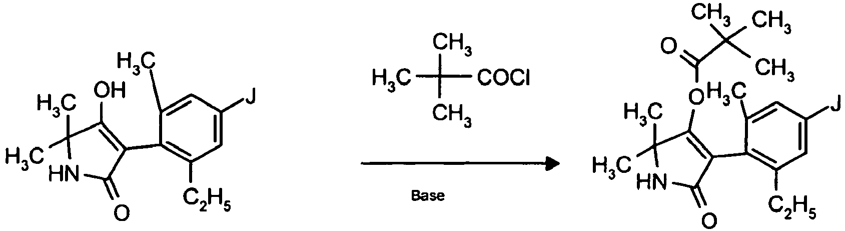

Verwendet man beispielsweise gemäß Verfahren (Iβ) 3-(2,6-Dimethyl-4-iod-phenyl)-5,5-dimethylpyrrolidin-2,4-dion und Acetanhydrid als Ausgangsverbindungen, so kann der Verlauf des erfindungsgemäßen Verfahrens durch folgendes Reaktionsschema wiedergegeben werden:

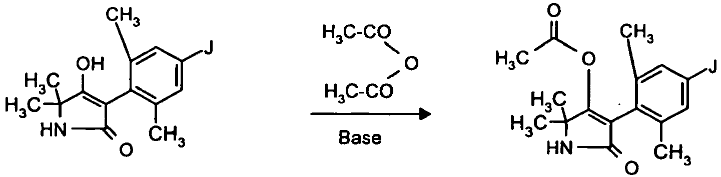

Verwendet man beispielsweise gemäß Verfahren (J) 8-[(2,6-Dimethyl-4-iod)-phenyl]-1-azabicyclo-(4,3,0^{1,6})-nonan-7,9-dion und Chlorameisensäureethylester als Ausgangsverbindungen, so kann der Verlauf des erfindungsgemäßen Verfahrens durch folgendes Reaktionsschema wiedergegeben werden:

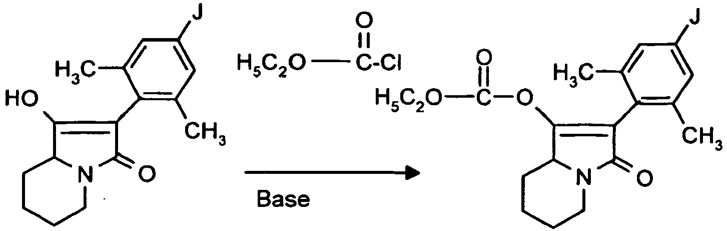

Verwendet man beispielsweise gemäß Verfahren (P) 3-(4-Brom-2,6-dimethyl-phenyl)-5,5-dimethyl-pyrrolidin-2,4-dion und Natriummethylat als Ausgangsstoffe, so kann der Reaktionsverlauf durch folgendes Schema wiedergegeben werden:

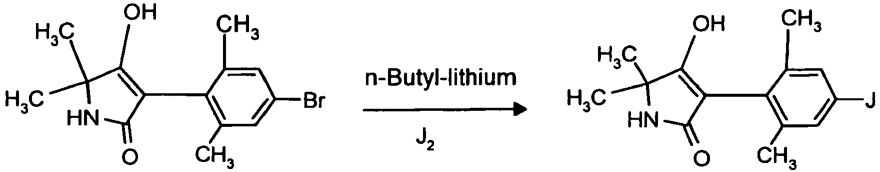

Die beim erfindungsgemäßen Verfahren (a) als Ausgangsstoffe benötigten Verbindungen der Formel (II) 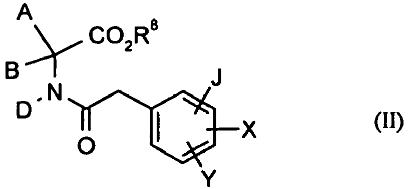 in welcher
A, B, D, J, X, Y und R⁸ die oben angegebenen Bedeutungen haben,
sind neu.
Man erhält die Acylaminosäureester der Formel (II) beispielsweise, wenn man Aminosäurederivate der Formel (XXIII) 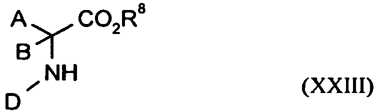 in welcher
A, B, R⁸ und D die oben angegebenen Bedeutungen haben,
mit substituierten Phenylessigsäurederivaten Formel (XXIV) 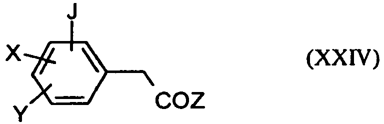 in welcher
- J, X und Y: die oben angegebenen Bedeutungen haben und
- Z: für eine durch Carbonsäureaktivierungsreagenzien wie Carbonyldiimidazol, Carbonyldiimide (wie z.B. Dicyclohexylcarbondiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968)
oder wenn man Acylaminosäuren der Formel (XXV) 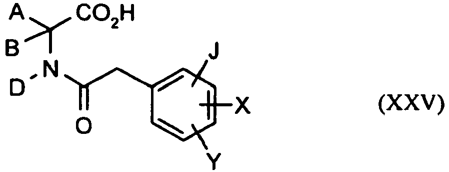 in welcher
- A, B, D, J, X und Y: die oben angegebenen Bedeutungen haben,
verestert (Chem. Ind. (London) 1568 (1968)).

Die Verbindungen der Formel (XXV) 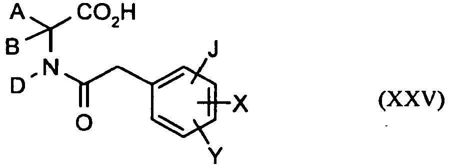 in welcher
- A, B, D, J, X und Y: die oben angegebenen Bedeutungen haben,
sind neu.

Man erhält die Verbindungen der Formel (XXV), wenn man Aminosäuren der Formel (XXVI) 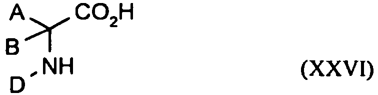 in welcher
- A, B und D: die oben angegebenen Bedeutungen haben,
mit substituierten Phenylessigsäurederivaten der Formel (XXIV) 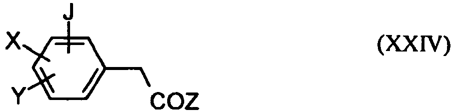 in welcher
- J, X und Y: die oben angegebenen Bedeutungen haben und
- Z: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXIV) sind neu. Sie lassen sich nach im Prinzip bekannten Verfahren und wie aus den Beispielen ersichtlich darstellen (s. z.B. H. Henecka, Houben-Weyl, Methoden der Organischen Chemie, Bd. 8, S. 467-469 (1952)).

Man erhält die Verbindungen der Formel (XXIV) beispielsweise, indem man substituierte Phenylessigsäuren der Formel (XXVII) 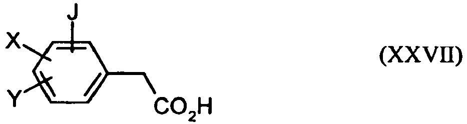 in welcher
- J, X und Y: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von -10°C bis 100°C, umsetzt.

Die Verbindungen der Formel (XXIII) und (XXVI) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXVI), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 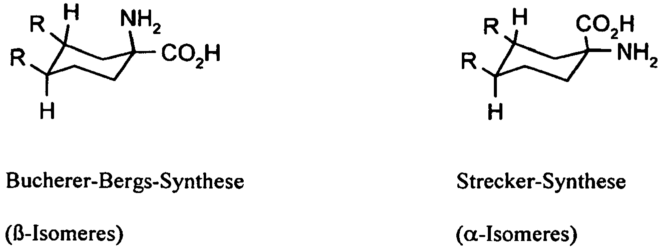 (L. Munday, J. Chem. Soc. 4372 (1961); J.T. Eward, C. Jitrangeri, Can. J. Chem. 53, 3339 (1975). Weiterhin lassen sich die bei dem obigen Verfahren (A) verwendeten Ausgangsstoffe der Formel (II) 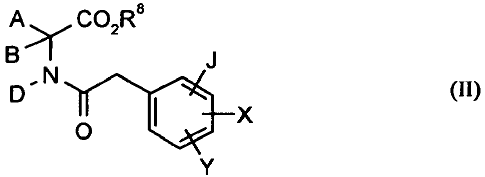 in welcher
A, B, D, J, X, Y und R⁸ die oben angegebenen Bedeutungen haben,
herstellen, wenn man Aminonitrile der Formel (XXVIII) 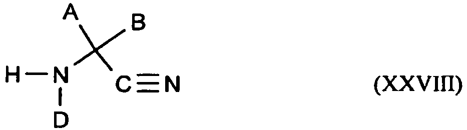 in welcher
- A, B und D: die oben angegebenen Bedeutungen haben,
mit substituierten Phenylessigsäurederivaten der Formel (XXIV) 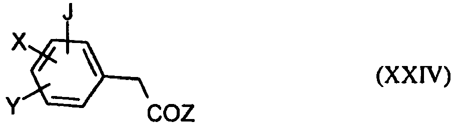 in welcher
- J, X, Y und Z: die oben angegebenen Bedeutungen haben,
zu Verbindungen der Formel (XXIX) 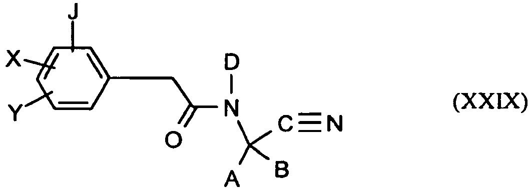 in welcher
- A, B, D, J, X und Y: die oben angegebenen Bedeutungen haben,
umsetzt,
und diese anschließend einer sauren Alkoholyse unterwirft.

Die Verbindungen der Formel (XXIX) sind ebenfalls neu.

Die Verbindungen der Formel (XXVII) sind teilweise käuflich, teilweise bekannt, aber auch teilweise neu.

Beispielsweise erhält man die Verbindungen der Formel (XXVII), 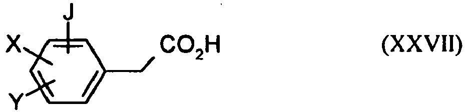 in welcher
- J, X und Y: die oben angegebenen Bedeutungen haben,
wenn man Phenylessigsäureester der Formel (XXXI) 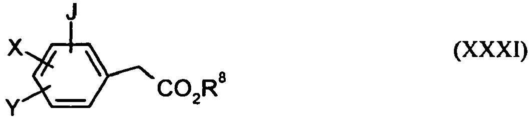 in welcher
J, X, Y und R⁸ die oben angegebene Bedeutung haben,
in Gegenwart von Säuren oder Basen, in Gegenwart eines Lösungsmittels unter allgemein bekannten Standardbedingungen verseift.

Die Verbindungen der Formel (XXXI) sind teilweise käuflich, teilweise bekannt z.B. aus WO 01/17973, aber auch teilweise neu.

Die Verbindungen der Formel (XXXI) 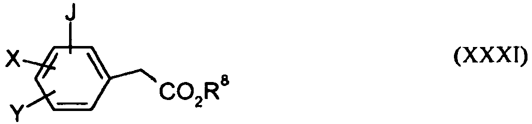 in welcher
J, X, Y und R⁸ die oben angegebene Bedeutung haben, erhält man außerdem nach dem in den Beispielen beschriebenen Verfahren (Q), wenn man Phenylessigsäureester der Formel (XXXI-a) 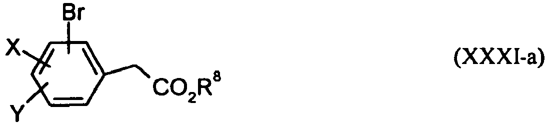 in welcher
- R⁸, X und Y: die oben angegebene Bedeutung haben,
in Gegenwart von Iodiden (bevorzugt Natriumiodid oder Kaliumiodid), in Gegenwart einer Base und gegebenenfalls in Gegenwart eines Katalysators (bevorzugt Kupfersalze wie z.B. Kupfer(I)iodid) umsetzt.

Die Phenylessigsäureester der Formel (XXXI-a) sind prinzipiell beispielsweise aus den Anmeldungen WO 96/35 664, WO 97/02243, WO 97/01535, WO 98/05638 und DE-A-10 301 804 bekannt und lassen sich nach den dort beschriebenen Verfahren herstellen.

Die zur Durchführung der erfindungsgemäßen Verfahren (I), (J), außerdem als Ausgangsstoffe benötigten Säurehalogenide der Formel (XIII), Carbonsäureanhydride der Formel (XIV), Chlorameisensäureester oder Chlorameisensäurethioester der Formel (XV), sind allgemein bekannte Verbindungen der Organischen bzw. Anorganischen Chemie.

Die Verbindungen der Formeln, (XIII) bis (XV), (XXIII), (XXVI), (XXVIII), sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, J, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (I-α) ist dadurch gekennzeichnet, dass man Verbindungen der Formel (1-1-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (I-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (I-α) innerhalb eines größeren Bereiches variiert werden. Im Allgemeinen arbeitet man bei Temperaturen zwischen - 20°C und +150°C, vorzugsweise zwischen 0°C und 100°C.

Bei der Durchführung des erfindungsgemäßen Verfahrens (I-α) werden die Ausgangsstoffe der Formel (1-1-a) und das Carbonsäurehalogenid der Formel (XIII) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (I-β) ist dadurch gekennzeichnet, dass man Verbindungen der Formel (I-I-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (1-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (I-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (I-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-β) werden die Ausgangsstoffe der Formel (I-1-a) und das Carbonsäureanhydrid der Formel (XIV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (J) ist dadurch gekennzeichnet, dass man Verbindungen der Formel (I-1-a) jeweils mit Chlorameisensäureestern oder Chlorameisensäurethiolestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (J) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (J) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (J) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (J) wird im Allgemeinen unter Normaldruck durchgeführt.

Bei der Durchführung des erfindungsgemäßen Verfahrens (J) werden die Ausgangsstoffe der Formel (I-1-a) und der entsprechende Chlorameisensäureester bzw. Chlorameisensäurethiolester der Formel (XIII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das Verfahren (Pα) ist dadurch gekennzeichnet, dass man Verbindungen der Formel (I-1-a') in welchen A, B, D, Q¹, Q², Q³, Q⁴, Q⁵, Q⁶, X und Y die oben angegebenen Bedeutungen haben und W' bevorzugt für Brom steht, mit Metalliodiden (z.B. Natriumiodid, Kaliumiodid), gegebenenfalls in Gegenwart einer Base und eines Cu-(I)-Salzes (z.B. CuBr oder CuJ) umsetzt.

Als Verdünnungsmittel können bei dem erfindungsgemäßen Verfahren (Pα) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, Ester wie Methylacetat, Ethylacetat, Propylacetat sowie Alkohole wie z.B. Methanol, Ethanol, Propanol, Iso-Propanol, Butanol und Iso-Butanol.

Als Base können bei der Durchführung des erfindungsgemäßen Verfahrens (Pa) vor allem organische Basen eingesetzt werden. Vorzugsweise verwendbar sind Amine wie z.B. N,N-Dimethylethylendiamin, 1,2-Diaminocyclohexan einsetzbar.

Die Reaktionstemperatur kann bei der Durchführung des erfindungsgemäßen Verfahrens (Pα) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (Pα) wird im Allgemeinen unter Normaldruck durchgerührt.

Bei der Durchführung des erfindungsgemäßen Verfahrens (Pα) setzt man die Reaktionskomponente der Formel (I-1-a') im Allgemeinen mit Überschüssen der Metalliodide bis zu 20 Mol, bevorzugt 1,1 bis 5 Mol um. Die Kupfer-I-Salze werden in der Regel katalytisch eingesetzt; 0,001 bis 0,5 Mol, bevorzugt 0,01 bis 0,2 Mol. Es ist jedoch auch möglich diese äquimolar einzusetzen.

Das Verfahren (Pβ) ist dadurch gekennzeichnet, dass man Verbindungen der Formel (I-1-a') in welchen A, B, D, Q¹, Q², Q³, Q⁴, Q⁵, Q⁶, X und Y die oben angegebenen Bedeutungen haben und W' bevorzugt für Brom steht, mit Metallorganylen einen Halogenmetallaustausch durchführt und das entstandene Anion mit lodierungsreagenzien umsetzt.

Als Verdünnungsmittel können bei dem erfindungsgemäßen Verfahren (Pβ) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Diethylether, Methyl-tert.-butylether, Dibutylether, Tetrahydrofuran, Dioxan, Glykoldimethylether und Diglykoldimethylether.

Als lodierungsreagenzien können bei der Durchfiihrung des Verfahrens (Pβ) gebräuchliche Reagenzien wie Iod, Iodmonochlorid, Iodmonobromid eingesetzt werden.

Für den Halogenmetallaustausch können bei der Durchführung des erfindungsgemäßen Verfahrens (Pβ) alle üblichen Metallorganyle eingesetzt werden. Vorzugsweise verwendbar sind n-Butyllithium, sek.-Butyllithium, tert.-Butyllithium, Phenyllithium.

Die Reaktionstemperatur kann bei der Durchführung des erfindungsgemäßen Verfahrens (Pβ) innerhalb eines größeren Bereiches variiert werden. Im Allgemeinen arbeitet man bei Temperaturen zwischen -120°C und 50°C, vorzugsweise zwischen -78°C und 30°C.

Das erfindungsgemäße Verfahren (Pβ) wird im Allgemeinen unter Normaldruck durchgeführt.

Bei der Durchführung des erfindungsgemäßen Verfahrens (Pβ) setzt man die Reaktionskomponente der Formel (I-1-a') im Allgemeinen mit Überschüssen der Metallorganyle und der Iodierungsreagenzien bis zu 20 Mol, bevorzugt 1,2 bis 5 Mol um.

Die erfindungsgemäßen Wirkstoffe/Wirkstoffkombinationen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp.

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp.

Aus der Ordnung der Chilopoda z.B. Geophilus spp., Scutigera spp.

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp.

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa.

Aus der Klasse der Gastropoda z.B. Arion spp., Biomphalaria spp., Bulinus spp., Deroceras spp., Galba spp., Lymnaea spp., Oncomelania spp., Succinea spp.

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp.

Aus der Ordnung der Isopoda z.B. Armadillidium vulgare, Oniscus asellus, Porcellio scaber.

Aus der Ordnung der Isoptera z.B. Reticulitermes spp.

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp.

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Rhipiphorothrips cruentatus, Scirtothrips spp., Taeniothrips cardamoni, Thrips spp.

Aus der Ordnung der Thysanura z.B. Lepisma saccharina.

Zu den pflanzenparasitären Nematoden gehören z.B. Aphelenchoides spp., Bursaphelenchus spp., Ditylenchus dipsaci, Globodera spp., Heterodera spp., Longidorus spp., Meloidogyne spp., Pratylenchus spp., Radopholus similis, Trichodorus spp., Tylenchulus semipenetrans, Xiphinema spp.

Die erfindungsgemäßen Verbindungen/Wirkstoffkombinationen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen/Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Samen, weiterhin durch ein- oder mehrschichtiges Umhüllen.

Die Wirkstoffe/Wirkstoffkombinationen können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe/Wirkstoffkombinationen mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Der erfindungsgemäße Wirkstoff/Wirkstoffkombinationen kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenem, Düngemitteln oder Semiochemicals vorliegen.

Besonders günstige Mischpartner sind z.B. die folgenden:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); lodocarb; Ipconazole; Iprobenfos; Iprodione; lprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothalisopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazole-carboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate; sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### Acetylcholinesterase (AChE) Inhibitoren

1.1 Carbamate,
   zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
1.2 Organophosphate,
   zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion

### Natrium-Kanal-Modulatoren / Spannungsabhängige Natrium-Kanal-Blocker

2.1 Pyrethroide,
   zum Beispiel Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (IR-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum)
   DDT
2.2 Oxadiazine,
   zum Beispiel Indoxacarb

### Acetylcholin-Rezeptor-Agonisten/-Antagonisten

3.1 Chloronicotinyle,
   zum Beispiel Acetamiprid, Clothianidin, Dinotefuran, Imidacloprid, Nitenpyram, Nithiazine, Thiacloprid, Thiamethoxam
3.2 Nicotine, Bensultap, Cartap

### Acetylcholin-Rezeptor-Modulatoren

4.1 Spinosyne,
   zum Beispiel Spinosad

### GABA-gesteuerte Chlorid-Kanal-Antagonisten

5.1 Organochlorine,
   zum Beispiel Camphechlor, Chlordane, Endosulfan, Gamma-HCH, HCH, Heptachlor, Lindane, Methoxychlor
5.2 Fiprole,
   zum Beispiel Acetoprole, Ethiprole, Fipronil, Pyrafluprole, Pyriprole, Vaniliprole

### Chlorid-Kanal-Aktivatoren

6.1 Mectine,
   zum Beispiel Avermectin, Emamectin, Emamectin-benzoate, Ivermectin, Milbemycin Juvenilhormon-Mimetika,
   zum Beispiel Diofenolan, Epofenonane, Fenoxycarb, Hydroprene, Kinoprene, Methoprene, Pyriproxifen, Triprene

### Ecdysonagonisten/disruptoren

8.1 Diacylhydrazine,
   zum Beispiel Chromafenozide, Halofenozide, Methoxyfenozide, Tebufenozide

### Inhibitoren der Chitinbiosynthese

9.1 Benzoylharnstoffe,
   zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
9.2 Buprofezin
9.3 Cyromazine

### Inhibitoren der oxidativen Phosphorylierung, ATP-Disruptoren

10.1 Diafenthiuron
10.2 Organozinnverbindungen,
   zum Beispiel Azocyclotin, Cyhexatin, Fenbutatin-oxide

### Entkoppler der oxidativen Phoshorylierung durch Unterbrechung des H-Protongradienten

11.1 Pyrrole,
   zum Beispiel Chlorfenapyr
11.2 Dinitrophenole,
   zum Beispiel Binapacyrl, Dinobuton, Dinocap, DNOC

### Seite-I-Elektronentransportinhibitoren

12.1 METl's,
   zum Beispiel Fenazaquin, Fenpyroximate, Pyrimidifen, Pyridaben, Tebufenpyrad, Tolfenpyrad
12.2 Hydramethylnon
12.3 Dicofol

### Seite-II-Elektronentransportinhibitoren

Rotenone

### Seite-III-Elektronentransportinhibitoren

Acequinocyl, Fluacrypyrim

### Mikrobielle Disruptoren der Insektendarmmembran

Bacillus thuringiensis-Stämme

### Inhibitoren der Fettsynthese

Tetronsäuren,
   zum Beispiel Spirodiclofen, Spiromesifen
Tetramsäuren,
   zum Beispiel Spirotetramat (CAS-Reg.-No.: 203313-25-1) und 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8)
Carboxamide,
   zum Beispiel Flonicamid
Oktopaminerge Agonisten,
   zum Beispiel Amitraz

### Inhibitoren der Magnesium-stimulierten ATPase,

Propargite
Benzoesäuredicarboxamide,
   zum Beispiel Flubendiamide
Nereistoxin-Analoge,
   zum Beispiel Thiocyclam hydrogen oxalate, Thiosultap-sodium

### Biologika, Hormone oder Pheromone

Azadirachtin, Bacillus spec., Beauveria spec., Codlemone, Metarrhizium spec., Paecilomyces spec., Thuringiensin, Verticillium spec.

### Wirkstoffe mit unbekannten oder nicht spezifischen Wirkmechanismen

23.1 Begasungsmittel,
   zum Beispiel Aluminium phosphide, Methyl bromide, Sulfuryl fluoride
23.2 Fraßhemmer,
   zum Beispiel Cryolite, Flonicamid, Pymetrozine
23.3 Milbenwachstumsinhibitoren,
   zum Beispiel Clofentezine, Etoxazole, Hexythiazox
23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Triarathene,Verbutin

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muss.

Die erfindungsgemäßen Wirkstoffe/Wirkstoffkombinationen können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepassten üblichen Weise.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel 1 bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe/Wirkstoffkombinationen wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Omithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe/Wirkstoffkombinationen der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe/Wirkstoffkombinationen geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feedthrough-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen/Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:
Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.%, vorzugsweise 0,001 bis 10 Gew.%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstofiharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.%, vorzugsweise 50 bis 68 Gew.%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester. Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfernaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
2-tert.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
Benzo[b]thiophencarbonsäurecyclohexylamid-S,S-dioxid, Dichlofluanid, Fluorfolpet, 3-Iod-2-propinyl-butylcarbamat, Tolylfluanid und Azole wie
Azaconazole, Cyproconazole, Epoxyconazole, Hexaconazole, Metconazole, Propiconazole und Tebuconazole;
Molluskizide wie
Fentinacetat, Metaldehyd, Methiocarb, Niclosamid, Thiodicarb und Trimethacarb, Fe-chelate;
oder herkömmliche Antifouling-Wirkstoffe wie
4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp.
Aus der Ordnung der Chilopoda z.B. Geophilus spp.
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe/Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe/Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe/Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe/Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zierund Sportrasen und Weideflächen sowie zur selektiven Unkrautbekämpfung in einjährigen Kulturen eingesetzt werden.

Die erfindungsgemäßen Verbindungen der Formel (I)/Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe/Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe/Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe/Wirkstoffkombinationen können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Aminopyralid, Anilofos, Asulam, Atrazine, Azafenidin, Azimsulfuron, Beflubutamid, Benazolin (-ethyl), Benfuresate, Bensulfuron (-methyl), Bentazon, Benzcarbazone, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, HOK-201, Imazamethabenz -methyl), Imazamethapyr, Imazamox, Imazapic, Imazapyr, Imazaquin, Imazethapyr, Imazosulfuron, Iodosulfuron (-methyl, -sodium), loxynil, Isopropalin, Isoproturon, Isouron, Isoxaben, Isoxachlortole, Isoxaflutole, Isoxapyrifop, KIH 485, Lactofen, Lenacil, Linuron, MCPA, Mecoprop, Mefenacet, Mesosulfurone, Mesotrione, Metamifop, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Orthosulfamuron, Oryzalin, Oxadiargyl, Oxadiazon, Oxasulfuron, Oxaziclomefone, Oxyfluorfen, Paraquat, Pelargonsäure, Pendimethalin, Pendralin, Penoxsulam, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrasulfotole, Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrimisulfan, Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tembotrione, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Topramezone, Tralkoxydim, Triallate, Triasulfuron, Tribenuron (-methyl), Triclopyr, Tridiphane, Trifluralin, Trifloxysulfuron, Triflusulfuron (-methyl), Tritosulfuron und

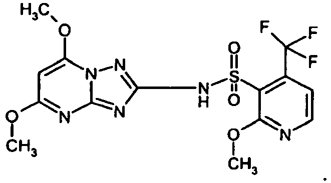

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im Allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im Allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im Allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Die Herstellung und die Verwendung der erfindungsgemäßen Wirkstoffe geht aus den nachfolgenden Beispielen hervor.

### Herstellungsbeispiele

### Beispiel I-1-a-1

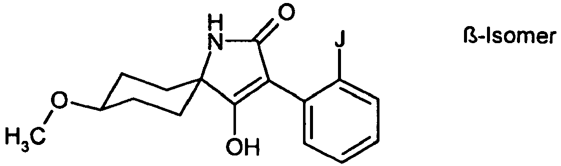

In einem 100 ml Dreihalskolben mit Thermometer und Rückflusskühler werden unter Argon 5,24 g Kalium-tert.-butylat -95 %ig- (44,4 mmol) in 10 ml Dimethylacetamid vorgelegt.

Bei 40 bis 50°C tropft man 8,7 g der Verbindung gemäß Beispiel II-1 (20,2 mmol) in 10 ml Dimethylacetamid zu. Unter dünnschichtchromatographischer Kontrolle rührt man 1 h bei 60°C.

Die Reaktionslösung wird dann in 100 ml Eiswasser eingerührt; mit konz. HCl auf pH 2 gestellt und der Niederschlag abgesaugt. Es folgt säulenchromatographische Reinigung an Kieselgel (Dichlormethan : Essigsäureethylester 5 : 3).

Ausbeute 7,8 g (94 % d. Theorie), Fp. 225,6°C.

### Beispiel I-1-a-3

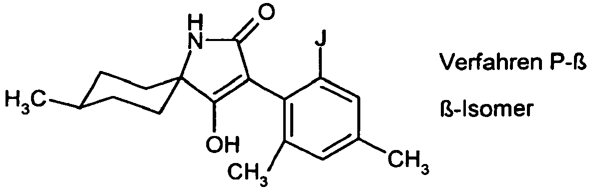

1.413 g (3 mmol) gemäß Beispiel I-1-c-4 aus WO 97/02243 werden in 30 ml wasserfreiem Tetrahydrofuran in einem 100 ml Dreihalskolben unter Argon vorgelegt. Dazu tropft man bei -78°C 2.64 ml n-Butyllithium (2,5 m in n-Hexan). Nach 15 Minuten rühren werden 0.761 g (3 mmol) Iod in 5 ml wasserfreiem Tetrahydrofuran bei -78°C zugetropft und der Ansatz langsam auf Raumtemperatur kommen lassen. Das Lösungsmittel wird abgedampft und der Rückstand durch Flashchromatographie an Kieselgel mit Methylenchlorid/Aceton 5:1 als Fließmittel vorgereinigt. Die das Produkt enthaltenen Fraktionen wurden vereinigt, das Lösungsmittel abgezogen und der Rückstand 250 mg mittels Reversed Phase Chromatographie mit Acetonitril/Wasser (Gradientprogram 70:30 → 10 : 90) gereinigt. Ausbeute: 40 mg (=̂ 2.7 % d. Theorie) Fp. 245°C.

In Analogie zu Beispiel (I-1-a-1) und gemäß den allgemeinen Angaben zur Herstellung erhält man folgende Verbindungen der Formel (I-1-a)

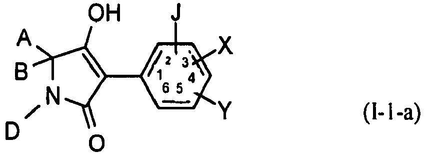

| **Bsp.-Nr.** | **J** | **X** | **Y** | **D** | **A** | **B** | **Fp °C** | **Isomer** |
|---|---|---|---|---|---|---|---|---|
| I-1-a-2 | 4-J | 2-C₂H₅ | 6-CH₃ | H | -(CH₂)₂-CHOCH₃-(CH₂)₂- | | 262 | ß |
| I-1-a-3 | 2-J | 4-CH3 | 6-CH3 | H | -(CH₂)₂-CHCH₃-(CH₂)₂- | | 245 | ß |
| I-1-a-4 | 2-J | 4-Cl | 6-CH₃ | H | -(CH₂)₂-CHOCH₃-(CH₂)₂- | | 295 | ß |
| I-1-a-5 | 2-J | 5-CH₃ | H | H | -(CH₂)₂-CHOCH₃-(CH₂)₂- | | 222 | ß |
| I-1-a-6 | 2-J | 5-CH₃ | H | H | -(CH₂)₂-O-(CH₂)₂- | | Zersetzung | - |
| I-1-a-7 | 2-J | 4-Cl | 6-C₂H₅ | H | -(CH₂)₂-O-(CH₂)₂- | | 324 | - |
| I-1-a-8 | 2-J | 4-Cl | 6-C₂H₅ | H | -(CH₂)₂-CHOCH₃-(CH₂)₂- | | *1.02 (t, 3H, ArCH₂CH₃), 7.31, (d. 1H, Ar-H), 7.74 (d, 1H, Ar-H), | ß |
| I-1-a-9 | 2-J | 4-Cl | 6-CH₃ | H | -(CH₂)₂-O-(CH₂)₂- | | *3.83 - 3.88 (m, 2H, O-CH₂), 7.35, (d, 1 H, Ar-H), 7.74 (d, 1H, Ar-H), | - |
| I-1-a-10 | 3-J | 6-CH₃ | H | H | -(CH₂)₂-CHOCH₃-(CH₂)₂- | | 248 | ß |
| I-1-a-11 | 4-J | 2-C₂H₅ | 6-CH3 | H | -(CH₂)₂-O-(CH₂)₂- | | 300 | - |
| I-1-a-12 | 4-J | 2-C₂H₅ | 6-CH₃ | H | CH₃ | CH₃ | 254 | - |
| I-1-a-13 | 4-J | 2-C₂H₅ | 6-CH₃ | H | 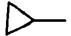 | CH₃ | *2.05 (d, 3H, ArCH₃), 1.20 (m, 1 H, CH (C₁-Cyclopropyl)) | - |
| I-1-a- 14 | 4-J | 2-C₂H₅ | 6-CH₃ | H | n-C₃H₇ | CH₃ | *2.05 (d, 3H, ArCH₃), 1.31 (d, 3H, CH₃ Tetramsäure) | - |
| I-1-a-15 | 4-J | 2-C₂H₅ | 6-CH₃ | 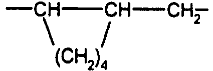 | | H | *2.03 (s, 3H, ArCH₃), 4.34 (dd, 1 H, N-CH Tetramsäure) | - |
| I-1-a-16 | 4-J | 2-C₂H₅ | 6-CH3 | H | -(CH₂)₅- | | *2.40 (m, 2H, Ar-CH₂), 2.05 (s, 3H, Ar-CH₃) 1.05 (t, 3H, ArCH₂CH₃) | - |
| I-1-a-17 | 4-J | 2-C₂H₅ | 6-CH₃ | H | -(CH₂)₂-CHCH₃-(CH₂)₂- | | *7.4 (s, br, 2H, Ar-H) 1.10 (tr, 3H, Ar CH₂-CH₃) 0.95 ("tr", 3H, CHCH₃) | - |
| I-1-a-18 | 4-J | 2-CH₃ | 6-CH₃ | H | -(CH₂)₂-CHOCH₃-(CH₂)₂- | | 295 | ß |
| I-1-a-19 | 4-J | 2-CH₃ | 6-CH₃ | H | -(CH₂)₂-O-(CH₂)₂- | | > 300 | - |
| I-1-a-20 | 4-J | 2-C₂H₅ | 6-Cl | H | -(CH₂)₂-O-(CH₂)₂- | | 274 | - |
| I-1-a-21 | 4-J | 2-CH3 | 6-CH₃ | H | -CH₂-CHOC₄H₉-(CH₂)₃- | | 182 | ß |
| I-1-a-22 | 4-J | 2-CH₃ | 6-Cl | H | -(CH₂)₂-O-(CH₂)₂- | | > 300 | - |
| I-1-a-23 | 4-J | 2-C₂H₅ | 6-CH₃ | H | -CH₂-CHOC₄H₉-(CH₂)₃- | | 166 | ß |
| I-1-a-24 | 4-J | 2-C₂H₅ | 6-Cl | H | -(CH₂)₂-CHOCH₃-(CH₂)₂- | | > 300 | ß |
| I-1-a-25 | 4-J | 2-CH₃ | 6-Cl | H | -(CH₂)₂-CHOCH₃-(CH₂)₂- | | 299 | ß |
| I-1-a-26 | 4-J | 2-C₂H₅ | 6-CH₃ | H | i-C₃H₇ | CH₃ | 80 | - |
| I-1-a-27 | 4-J | 2-C₂H₅ | 6-CH₃ | H | i-C₄H₉ | CH₃ | 225 | - |

| | | | | | | | | |
|---|---|---|---|---|---|---|---|---|
| * ¹H-NMR (400 MHZ, d₆-DMSO):Verschiebungen δ in ppm. | | | | | | | | |

### Beispiel I-1-b-1

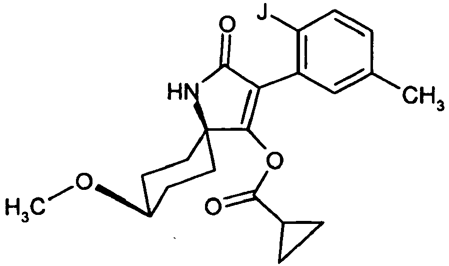

0,48 g der Verbindung gemäß Beispiel I-1-a-5 werden in 30 ml Essigsäureethylester unter Schutzgasatmosphäre vorgelegt, 0,15 ml Triethylamin und 10 mg Steglichbase zugegeben und unter Rückfluss 0,115 g Cyclopropylcarbonsäurechlorid in 5 ml Essigsäureethylester zugetropft und unter Rückfluss weitergerührt.

Nach Reaktionsende (dünnschichtchromatographische Kontrolle) erfolgt Reinigung über Flashsäulentrennung an Kieselgel (Essigsäureethylester als Laufmittel)

Ausbeute: 0,4 g (75 % d. Theorie), Fp. 167°C

In Analogie zu Beispiel (I-1-b-1) und gemäß den allgemeinen Angaben zur Herstellung erhält man folgende Verbindungen der Formel (I-1-b)

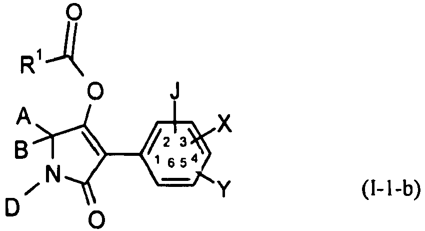

| **Bsp.-Nr.** | **J** | **x** | **Y** | **D** | **A** | **B** | **R¹** | **Fp °C** | **Isomer** |
|---|---|---|---|---|---|---|---|---|---|
| I-1-b-2 | 2-J | 5-CH3 | H | H | -(CH₂)₂-CHOCH₃-(CH₂)₂- | | i-C₃H₇ | **0.95, 1.01 (2s, 6H, CH(CH₃)₂) 2.67, (m, 1 H, CH (CH₃)₂) 7.71, (d, 1 H, ArH), | ß |
| I-1-b-3 | 2-J | 4-Cl | 6-C₂H₅ | H | -(CH₂)₂-CHOCH₃-(CH₂)₂- | | H₃C-O-CH₂- | * 1.20 (t, 3H, Ar-CH₂CH₃), 3.25, (m, 1H, CH-OCH₃), 7.25, 7.70, (2d, 2H, Ar-H), | ß |
| I-1-b-4 | 2-J | 4-Cl | 6-C₂H₅ | H | -(CH₂)₂-O-(CH₂)₂- | | H₃C-O-CH₂- | *4.11 (q, 2H, OCH₂), 3.64, (dt, 2H, OCH₂ (tetrahydropyran)), 2.61 (m, 2H, Ar-CH₂), | - |
| I-1-b-5 | 4-J | 2-C₂H₅ | 6-CH₃ | H | -(CH₂)₂-O-(CH₂)₂- | | i-C₃H₇ | *1.15 (t, 3H, Ar-CHCH₃), 2.55, (s, 1 H, CH (CH₃)₂) 4.00 (m, 2H, O-CH₂) | - |
| I-1-b-6 | 4-J | 2-C₂H₅ | 6-CH₃ | H | C₃H₇ | CH₃ | i-C₃H₇ | *1.40 (s, 3H, 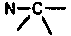 CH₃), 2.55, (m, 1H, CH (CH₃)₂) 7.4, 7.43 (2s, 2H, Ar-H) | - |
| I-1-b-7 | 4-J | 2-CH₃ | 6-CH3 | H | -CH₂-CH-OC₄H₉-(CH₂)₃- | | H₃C-O-CH₂- | 168-170 | ß |
| I-1-b-8 | 4-J | 2-CH3 | 6-CH3 | H | -CH₂-CHOC₄H₉-(CH₂)₃- | | 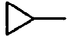 | *3.43 (m, 3H, OCH, OCH₂), 2.21 (s, 3 H, Ar-CH₃) 0,83 (m, 4H, Cyclopropyl H) | ß |
| I-1-b-9 | 4-J | 2-CH3 | 6-CH3 | H | -CH₂-CHOC₄H₉-(CH₂)₃- | | Cl-CH₂- | 185 | ß |
| I-1-b-10 | 4-J | 2-CH₃ | 6-CH₃ | H | -CH₂-CHOC₄H₉-(CH₂)₃- | | 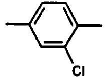 | 245 | ß |
| I-1-b-11 | 4-J | 2-C₂H₅ | 6-CH₃ | H | -(CH₂)₂-O-(CH₂)₂- | | H₃C-O-CH₂- | *4.04 (m, 4H, 2 x OCH₂), 2.18 (s, 3 H, Ar-CH₃) | - |
| I-1-b-12 | 4-J | 2-C₂H₅ | 6-Cl | H | -(CH₂)₂-O-(CH₂)₂- | | H₃C-O-CH₂- | *4.13 (q, 2H, OCH₂), 2.64 (q, 2H, Ar-CH₂) | - |
| I-1-b-13 | 4-J | 2-C₂H₅ | 6-CH3 | H | -CH₂-CHOC₄H₉-(CH₂)₃- | | i-C₃H₇ | *3.45 (m, 3H, OCH, OCH₂), 2.52 (sept, 1H, CH(CH₃)₂) | ß |
| I-1-b-14 | 4-J | 2-C₂H₅ | 6-CH₃ | H | i-C₃H₇ | CH₃ | i-C₃H₇ | 130 | - |
| I-1-b-15 | 4-J | 2-C₂H₅ | 6-CH3 | H | -CH₂-CHOC₄H₉-(CH₂)₃- | | H₃C-O-CH₂- | *3.98 (m, 2H, OCH₂), 3.45 (m, 3H, OCH, OCH₂) 2.20 (s, 3H, Ar-CH₃) | ß |
| I-1-b-16 | 4-J | 2-C₂H₅ | 6-CH₃ | H | -(CH₂)₅- | | H₃C-O-CH₂- | 201 | - |
| I-1-b-17 | 4-J | 2-C₂H₅ | 6-CH₃ | H | -(CH2)5- | | i-C₃H₇ | 217 | - |
| I-1-b-18 | 4-J | 2-C₂H₅ | 6-CH₃ | H | CH₃ | CH₃ | i-C₃H₇ | 137 | - |
| I-1-b-19 | 4-J | 2-C₂H₅ | 6-CH3 | H | -(CH₂)₅- | | t-C₄H₉ | 206 | - |
| I-1-b-20 | 4-J | 2-C₂H₅ | 6-CH₃ | H | i-C₃H₇ | CH₃ | H₃C-O-CH₂- | *4.05 (dd, 2H, OCH₂) 3.20 (s, 3H, OCH₃) 1.50 (d, 3H, CH₃) | - |
| I-1-b-21 | 4-J | 2-C₂H₅ | 6-CH₃ | H | CH₃ | CH₃ | H₃C-O-CH₂- | 165 | - |
| I-1-b-22 | 4-J | 2-C₂H₅ | 6-CH3 | H | i-C₃H₇ | CH₃ | i-C₃H₇ | 130 | - |

| | | | | | | | | | |
|---|---|---|---|---|---|---|---|---|---|
| * 1H-NMR (300 MHz/400 MHz, CDCl₃):Verschiebungen δ in ppm. ** ¹H-NMR (400 MHz, d₆-DMSO):Verschiebungen δ in ppm | | | | | | | | | |

### Beispiel I-1-c-1

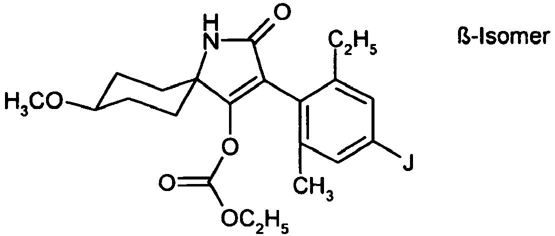

In einem 100 ml Dreihalskolben werden 0,66 g (1,5 mmol) gemäß Beispiel I-1-a-2 in 20 ml wasserfreiem Methylenchlorid unter Argon vorgelegt, mit 0,21 ml (1,5 mmol) Triethylamin versetzt und bei 20°C 0,14 ml (1,5 mmol) Chlorameisensäureethylester zugetropft. Es wird 4 Stunden nachgerührt, das Lösungsmittel im Vakuum abgedampft und der Rückstand an Kieselgel mit Methylenchlorid/Essigsäureethylester 10:1 als Fließmittel chromatographiert.

Ausbeute: 0,4 g (=̂ 43 % d. Theorie) Fp. 198°C

In Analogie zu Beispiel (I-1-c-1) und gemäß den allgemeinen Angaben zur Herstellung erhält man folgende Verbindungen der Formel (I-1-c)

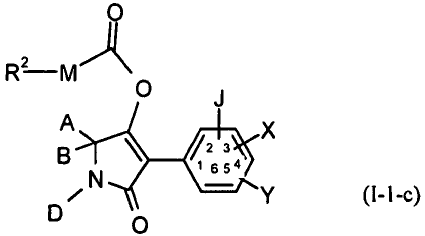

| **Bsp.-Nr.** | **J** | **x** | **Y** | **D** | **A** | **B** | **M** | **R²** | **Fp °C** | **Isomer** |
|---|---|---|---|---|---|---|---|---|---|---|
| I-1-c-2 | 2-J | 5-CH₃ | H | H | -(CH₂)₂-CHOCH₃-(CH₂)₂- | | O | C₆H₅-CH₂- | 152 | ß |
| I-1-c-3 | 2-J | 4-Cl | 6-CH₃ | H | -(CH₂)₂-CHOCH₃-(CH₂)₂- | | O | C₂H₅ | 186 | ß |
| I-1-c-4 | 2-J | 5-CH3 | H | H | -(CH₂)₂-CHOCH₃-(CH₂)₂- | | O | C₂H₅ | Wachs | ß |
| I-1-c-5 | 2-J | 4-Cl | 6-C₂H₅ | H | -(CH₂)₂-O-(CH₂)₂- | | O | C₂H₅ | *4.11 (q, 2H, O-CH₂), 4.03, (m, 2H, OCH₂) 2.20 (s, 3H, Ar-CH₃) | - |
| I-1-c-6 | 2-J | 4-Cl | 6-C₂H₅ | H | -(CH₂)₂-CHOCH₃-(CH₂)₂- | | O | C₂H₅ | *4.11 (q, 2H, O-CH₂), 3.39, (s, 3H, OCH₃) 2.61 (q, 2H, Ar-CH₂) | ß |
| I-1-c-7 | 3-J | 6-CH3 | H | H | -(CH₂)₂-CHOCH₃-(CH₂)₂- | | O | C₂H₅ | Wachs | ß |
| I-1-c-8 | 4-J | 2-CH₃ | 6-CH₃ | H | -(CH₂)₂-CHOCH₃-(CH₂)₂- | | O | C₂H₅ | 208 | ß |
| I-1-c-9 | 4-J | 2-CH3 | 6-Cl | H | -(CH₂)₂-O-(CH₂)₂- | | O | C₂H₅ | 214 | - |
| 1-1-c-10 | 4-J | 2-C₂H₅ | 6-CH₃ | H | n-C₃H₇ | CH₃ | O | C₂H₅ | *4.02 (m, 2H, OCH₂), 2.21 (d, 3H, ArCH₃) 1.46 (s, 3H, CH₃) | - |
| I-1-c-1 | 4-J | 2-C₂H₅ | 6-CH3 | H | 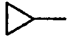 | CH₃ | O | C₂H₅ | *4.05 (m, 2H, OCH₂), 2.21 (d, 3H, ArCH₃) 1.51 (s, 3H, CH₃) | - |
| I-1-c-12 | 4-J | 2-C₂H₅ | 6-CH₃ | H | -(CH₂)₂-O-(CH₂)₂- | | O | C₂H₅ | *4.07 (q, 2H, OCH₂), 4.00 (m, 2H, OCH₂) 2.47 (q, 2H, Ar-CH₂ | - |
| I-1-c-13 | 4-J | 2-C₂H₅ | 6-Cl | H | -(CH₂)₂-O-(CH₂)₂- | | O | C₂H₅ | *4.11 (q, 2H, OCH₂), 4.03 (m, 2H, OCH₂) 2.61 (q, 2H, Ar-CH₂) | - |
| I-1-c-14 | 4-J | 2-CH₃ | 6-CH₃ | H | -CH₂-CHOC₄H₉-(CH₂)₃- | | O | C₆H₅-CH₂- | *5.00 (q, 2H, OCH₂), 3.43 (m, 3H, OCH, OCH₂) 2.14 (s, 3H, Ar-CH₃) | ß |
| I-1-c-15 | 4-J | 2-CH₃ | 6-CH₃ | H | -CH₂-CHOC₄H₉-(CH₂)₃- | | O | CH₂=CH-CH₂- | *4.38 (d, 2H, OCH₂), 3.37 (m, 3H, OCH, OCH₂) 2.13 (s, 3H, Ar-CH₃) | ß |
| I-1-c-16 | 4-J | 2-CH3 | 6-CH₃ | H | -CH₂-CHOC₄H₉-(CH₂)₃- | | O | C₂H₅ | *4.04 (q, 2H, O-CH₂), 3.43 (m, 3H, OCH, OCH₂) 2.17 (s, 3H, Ar-CH₃)- | ß |
| I-1-c-17 | 4-J | 2-C₂H₅ | 6-CH₃ | H | -(CH₂)₅- | | O | C₂H₅ | 188 | - |
| I-1-c-18 | 4-J | 2-C₂H₅ | 6-CH3 | H | CH₃ | CH₃ | O | C₂H₅ | *4.05 (q, 2H, O-CH₂), 2.20 (s, 3H, ArCH₃) 1.50 (2s, 6H, 2 x CH₃)- | - |
| I-1-c-19 | 4-J | 2-C₂H₅ | 6-CH₃ | H | i-C₄H₉ | CH₃ | O | C₂H₅ | *4.00 (m, 2H, O-CH₂), 2.50 (m, 2H, ArCH₂) 2.20 (d, 3H, Ar-CH₃)- | - |
| I-1-c-20 | 4-J | 2-CH₃ | 6-CH₃ | H | -(CH₂)₂-O-(CH₂)₂- | | O | C₂H₅ | 216 | - |

| | | | | | | | | | | |
|---|---|---|---|---|---|---|---|---|---|---|
| *¹H-NMR (400 MHz, CDCl₃):Verschiebungen δ in ppm. | | | | | | | | | | |

### Beispiel II-1

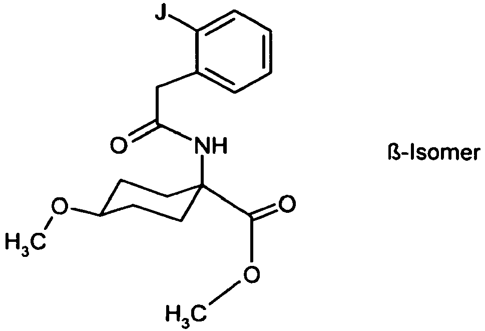

In einem 100 ml Dreihalskolben mit Thermometer und Rückflusskühler werden unter Argon 5,1 g cis-1-Amino-4-methoxy-cyclohexancarbonsäuremethylester-hydrochlorid (0.0226 mol) in 50 ml wasserfreiem Tetrahydrofuran vorgelegt. Bei 20°C werden 6,3 ml (0.0452 mol) Triethylamin zugetropft. Man rührt 5 min nach und versetzt bei 20°C mit 5,4 g 2-Iodphenylessigsäure (0.0205 mol). Nach 15 min tropft man 4,3 ml Triethylamin (0.0308 mol) dazu und sofort danach 1,15 ml Phosphoroxychlorid; die Lösung soll mäßig sieden. 30 min unter Rückfluss nachrühren. Nach Abkühlen und Abziehen des Lösungsmittels erfolgt eine säulenchromatographische Reinigung an Kieselgel (Dichlormethan : Essigsäureethylester 3 : 1)

Ausbeute: 8,7 g (96 % d. Theorie), Fp. 152°C

### Beispiel II-25

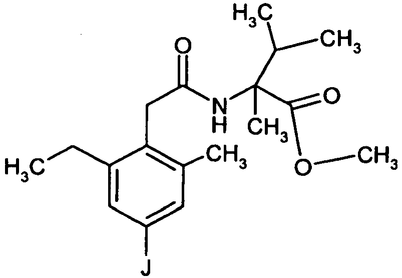

Zu 1,4 ml konzentrierte Schwefelsäure werden 1,8 g (4,5 mmol) der Verbindung gemäß Beispiel XXIX-1 in 20 ml Methylenchlorid zugetropft und 2 Stunden bei 30 - 40°C Außentemperatur gerührt. Anschließend tropft man 3.3 ml Methanol zu, rührt 4 Stunden bei 40 - 70°C Außentemperatur nach, lässt über Nacht stehen und rührt weitere 3 Stunden bei 40 bis 70°C nach. Dann wird die Reaktionslösung auf Eis/H₂O gegeben, mit Dichlormethan extrahiert, mit ges. NaHCO₃-Lösung gewaschen, getrocknet und einrotiert.

¹H-NMR (CDCl₃ 300 MHz): δ = 7.40 (s, 2H, Ar-H), 3.65 (s, 3H, OCH₃), 3.55 (s, 2H, CH₂) 1.90 (sept, 1 H, CH(CH₃)₂) ppm.

In Analogie zu den Beispielen (II-1) und (II-25) und gemäß den allgemeinen Angaben zur Herstellung erhält man folgende Verbindungen der Formel (II)

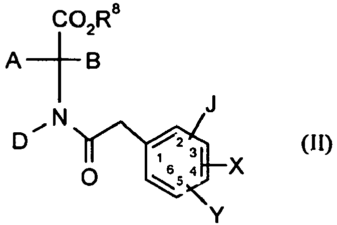

| **Bsp.-Nr.** | **J** | **X** | **Y** | **D** | **A** | **B** | **R⁸** | **Fp °C** | **Isomer** |
|---|---|---|---|---|---|---|---|---|---|
| II-2 | 4-J | 2-C₂H₅ | 6-CH₃ | H | -(CH₂)₂-CHOCH₃-(CH₂)₂- | | CH₃ | 138 | ß |
| II-3 | 2-J | 4-Cl | 6-CH₃ | H | -(CH₂)₂-CHOCH₃-(CH₂)₂- | | CH₃ | 151 | ß |
| II-4 | 2-J | 4-Cl | 6-C₂H₅ | H | -(CH₂)₂-CHOCH₃-(CH₂)₂- | | CH₃ | 147 | ß |
| II-5 | 2-J | 5-CH₃ | H | H | -(CH₂)₂-CHOCH₃-(CH₂)₂- | | CH₃ | 157 | ß |
| II-6 | 2-J | 4-Cl | 6-CH₃ | H | -(CH₂)₂-O-(CH₂)₂- | | CH₃ | 187 | - |
| II-7 | 2-J | 4-Cl | 6-C₂H₅ | H | -(CH₂)₂-O-(CH₂)₂- | | CH₃ | 164 | - |
| II-8 | 2-J | 5-CH₃ | H | H | -(CH₂)₂-O-(CH₂)₂- | | CH₃ | 154 | - |
| II-9 | 2-J | 4-Cl | 6-CH₃ | H | -CH₂-CHOC₄H₉-(CH₂)₃- | | CH₃ | 131 | ß |
| II-10 | 3-J | 6-CH₃ | H | H | -(CH₂)₂-CHOCH₃-(CH₂)₂- | | CH₃ | 138 | ß |
| II-11 | 4-J | 2-C₂H₅ | 6-CH₃ | H | -(CH₂)₂-O-(CH₂)₂- | | CH₃ | 163 | - |
| II-12 | 4-J | 2-C₂H₅ | 6-CH₃ | H | CH₃ | CH₃ | CH₃ | 169 | - |
| II-13 | 4-J | 2-C₂H₅ | Cl | H | -(CH₂)₂-CHOCH₃-(CH₂)₂- | | CH₃ | 134 | ß |
| II-14 | 4-J | 2-C₂H₅ | 6-CH₃ | H | -(CH₂)₂-CHOCH₃-(CH₂)₂- | | CH₃ | 169 | ß |
| II-15 | 4-J | 2-C₂H₅ | 6-Cl | H | -(CH₂)₂-O-(CH₂)₂- | | CH₃ | 165 | - |
| II-16 | 4-J | 2-C₂H₅ | 6-CH₃ | H | -(CH₂)₂-O-(CH₂)₂- | | CH₃ | 173 | - |
| II-17 | 4-J | 2-CH₃ | 6-CH₃ | H | -CH₂CHOC₄H₉-(CH₂)₃- | | CH₃ | 172 | ß |
| II-18 | 4-J | 2-CH3 | 6-Cl | H | -(CH₂)₂-O-(CH₂)₂- | | CH₃ | 173 | - |
| II-19 | 4-J | 2-CH₃ | 6-Cl | H | -(CH₂)₂-CHOCH₃-(CH₂)₂- | | CH₃ | 157 | ß |
| II-20 | 4-J | 2-C₂H₅ | 6-CH₃ | H | -CH₂-CHOC₄H₉-(CH₂)₃- | | CH₃ | 131 | ß |
| II-21 | 4-J | 2-CH₃ | 6-Cl | H | -CH₂-CHOC₄H₉-(CH₂)₃- | | CH₃ | 163 | ß |
| II-22 | 4-J | 2-C₂H₅ | 6-Cl | H | -CH₂-CHOC₄H₉-(CH₂)₃- | | CH₃ | 119 | ß |
| II-23 | 4-J | 2-C₂H₅ | 6-CH₃ | 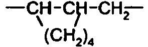 | | H | CH₃ | *4.24 (dd, 1H, N-CH), 3.57 (s, 3H, OCH₃), 2.11 (s, 3H, Ar-CH₃) | Gemisch |
| II-24 | 4-J | 2-C₂H₅ | 6-CH₃ | H | -(CH₂)₅- | | CH₃ | 144 | - |
| II-25 | 4-J | 2-C₂H₅ | 6-CH₃ | H | i-C₃H₇ | CH₃ | CH₃ | Öl | - |
| II-26 | 4-J | 2-C₂H₅ | 6-CH₃ | H | -(CH₂)₂-CHCH₃-(CH₂)₂- | | CH₃ | 130 | ß |
| II-27 | 4-J | 2-C₂H₅ | 6-CH₃ | H | i-C₄H₉ | CH₃ | CH₃ | 143 | - |

### Beispiel XXIX-1

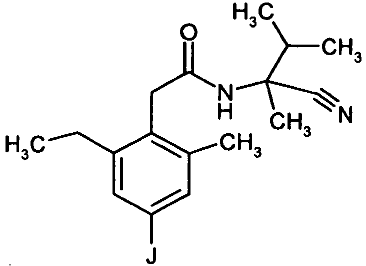

1,7 g (5,59 mmol) 4-Jod-2-ethyl-6-methyl-phenylessigsäure werden in 25 ml Tetrahydrofuran vorgelegt, 2 ml Triethylamin und 0,627 g (5,59 mmol) 2-Amino-2-methyl-isobutyronitril dazugegeben, 15 Minuten bei Raumtemperatur gerührt, mit 1 ml Triethylamin versetzt und danach 0,6 ml Phosphoroxychlorid so zugetropft, dass der Ansatz mäßig siedet. 30 Minuten unter Rückfluss rühren, einrotieren, mit Essigsäureethylester/Wasser aufarbeiten, organische Phase mit Natriumsulfat trocknen, mit Kieslegel rühren, abfiltrieren, einrotieren.

Ausbeute: 1,82 g (74 % d. Theorie), Fp. 186°C

In Analogie zu Beispiel (XXIX-1) und gemäß den allgemeinen Angaben zur Herstellung erhält man folgende Verbindungen der Formel (XXIX)

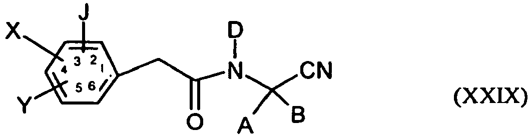

| **Bsp: Nr.** | **J** | **X** | **Y** | **A** | **B** | **¹H-NMR(300 MHz (CDCl₃) Verschiebung δ in ppm** |
|---|---|---|---|---|---|---|
| XXIX-2 | 4-J | 2-C₂H₅ | 6-CH3 | n-C₃H₇ | CH₃ | 0.90 (tr, 3H, (CH₂)₂-CH₃) |
| | | | | | | 1.60 (s, 3H, 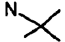 CH₃, |
| | | | | | | 3.6 (s, 2H, Ar-CH₂-CO) |
| XXIX-3 | 4-J | 2-C₂H₅ | 6-CH₃ | 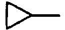 | CH₃ | 0.60-0.7 (m, 2H, 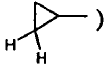 |
| | | | | | | 1.70 (s, 3H, 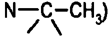 |
| | | | | | | 2.60 (q, 2H, Ar-CH₂-CH₃) |
| | | | | | | 3.60 (s, 2H, Ar-CH₂-CO) |

### Verfahren Q

### Beispiel XXXI-1

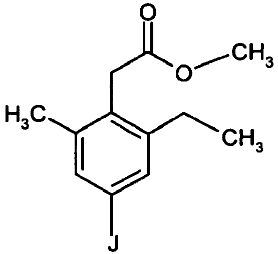

Eine Lösung von 10 g (4-Brom-2-methyl-6-ethylphenyl)-essigsäuremethylester, 11.056 g Natriumiodid, 7.023 g Kupfer(I)iodid und 3.172 g N,N'-Dimethylethylendiamin in 250 ml Dioxan wird unter Argon 18 h auf 110°C erhitzt. Nach beendeter Reaktion wird die Reaktionsmischung filtriert, die Mutterlauge mit 300 ml Wasser verdünnt und mit 2 mal 200 ml Dichlormethan extrahiert. Die organische Phase wird mit 25 %iger Ammoniaklösung gewaschen, über Natriumsulfat getrocknet und vom Lösungsmittel befreit. Ausbeute an (2-Ethyl-4-iod-6-methylphenyl)-essigsäuremethylester: 7,8 g, 65 %. ¹H-NMR (300MHz, d₆-DMSO): δ = 7.42 (d, 1 H), 7.39 (d, 1 H), 3.68 (s, 2 H), 3.61 (s, 3 H), 2.55 (q, 2 H), 219 (s, 3 H), 1.10 (t, 3 H) ppm.

### Beispiel XXVII-1

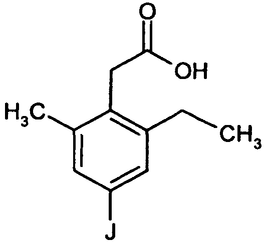

Eine Lösung von 7.7 g (2-Ethyl-4-iod-6-methylphenyl)-essigsäuremethylester in 50 ml THF wird mit einer Lösung von 0.696 g LiOH in 50 ml Wasser versetzt und 18 h bei Raumtemperatur gerührt. Anschließend wird zur Trockne einrotiert und der Rückstand mit je 50 ml Ethylacetat und Wasser versetzt. Die Phasen werden getrennt und die Ethylacetatphase mit Wasser gewaschen. Die vereinigten wässrigen Phasen werden mit HCl auf einen pH-Wert = 1 eingestellt, der ausgefallene Feststoff wird abgesaugt, mit Wasser gewaschen und im Vakuum getrocknet. Ausbeute an (2-Ethyl-4-iod-6-methylphenyl)-essigsäure: 6 g, 78 %.

¹H-NMR (300MHz, d₆-DMSO): δ = 12.5 (s(br), 1 H), 7.40 (d, 1 H), 7.38 (d, 1 H), 3.57 (s, 2 H), 2.56 (q, 2 H), 1.09 (t, 3 H) ppm.

### Beispiel XXXI-2

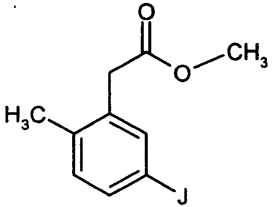

Eine Lösung von 20 g (5-Brom-2-methylphenyl)-essigsäuremethylester, 24.663 g Natriumiodid, 15.668 g Kupfer(I)iodid und 7.075 g N,N'-Dimethylethylendiamin in 500 ml Dioxan wird unter Argon 3 Tage auf 110°C erhitzt. Dann werden nochmals 8 g Natrium, 5.5 g Kupfer(I)iodid und 3.3 g N,N'-Dimethylethylendiamin zugegeben. Nach weiteren 3 Tagen Erhitzen auf 110°C wird die Reaktionsmischung filtriert, die Mutterlauge mit 300 ml Wasser verdünnt und mit 2 x 200 ml Dichlormethan extrahiert. Die organische Phase wird mit 25 %iger Ammoniaklösung gewaschen, über Na₂SO₄ getrocknet und vom Lösungsmittel befreit. Ausbeute an (5-Iod-2-methylphenyl)-essigsäuremethylester: 11.6 g, 30 %.

¹H-NMR (300MHz, d₆-DMSO): δ = 7.56 (d, 1 H), 7.51 (d, 1 H), 6.99 (d, 1 H), 3.62 (s, 2 H), 3.57 (s, 3 H), 2.16 (s, 3 H) ppm.

### Beispiel XXVII-2

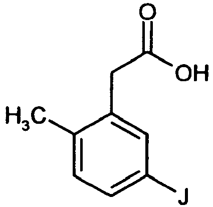

Eine Lösung von 10.7 g (5-Iod-2-methylphenyl)-essigsäuremethylester in 75 ml THF wird mit einer Lösung von 1.048 g LiOH in 75 ml Wasser versetzt und 18 h bei Raumtemperatur gerührt. Anschließend wird zur Trockne einrotiert und der Rückstand mit je 75 ml Ethylacetat und Wasser versetzt. Die Phasen werden getrennt und die organische Phase mit Wasser gewaschen. Die vereinigten wässrigen Phasen werden mit HCl auf einen pH-Wert = 1 eingestellt, der ausgefallene Feststoff wird nacheinander mit Dichlormethan und Ethylacetat ausgerührt, abgesaugt und im Vakuum getrocknet. Zur weiteren Reinigung wird er mit Diethylether verrührt und abfiltriert. Ausbeute an (5-Iod-2-methylphenyl)-essigsäure: 5.5 g, 44 %.

¹H-NMR (300MHz, d₆-DMSO): δ = 12.2 (s, (br) 1 H), 7.55 (d, 1 H), 7.49 (d, 1 H), 6.98 (d, 1 H), 3.56 (s, 2 H), 2.17 (s, 3 H) ppm.

In Analogie zu den Beispielen (XXXI-1) und (XXXI-2) erhält man folgende Verbindungen der Formel (XXXI)

| **Struktur** | **Bsp.Nr.** | **¹H-NMR (400 MHz, d₆ DMSO)** |
|---|---|---|
| 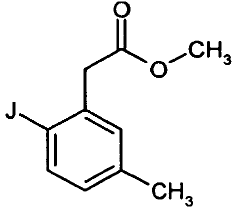 | XXXI-3 | δ = 7.70 (d, 1 H), 7.19 (d, 1 H), 6.85 (dd, 1 H), 3.76 (s, 2H), 3.63 (s, 3H), 2.25 (s, 2H) ppm. |
| 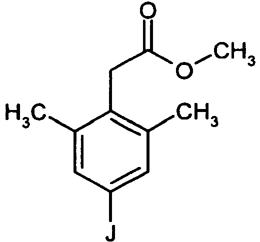 | XXXI-4 | δ = 7.41 (s, 2H), 3.66 (s, 2H), 3.60 (s, 3 H), 2.20 (s, 6H) ppm. |
| 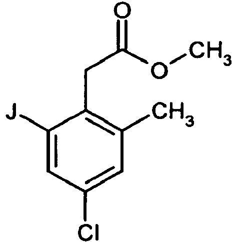 | XXXI-5 | δ = 7.76 (d, 1H), 7.35 (d, 1 H), 3.90 (s, 2 H), 3.64 (s, 3H), 2.30 (s, 3H), ppm. |
| 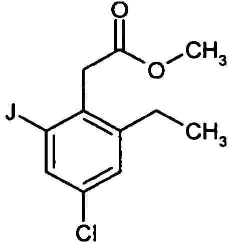 | XXXI-6 | δ = 7.79 (d, 1 H), 7.34 (d, 1 H), 3.91 (s, 2 H), 3.64 (s, 3H), 2.64 (q, 2H), 1,11 (t, 3H) ppm. |
| 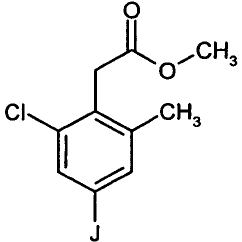 | XXXI-7 | Nur GC zur Reaktionskontrolle gemessen, direkt weiter umgesetzt. |
| 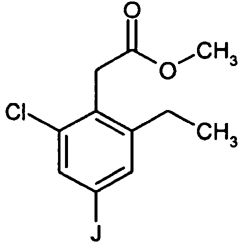 | XXXI-8 | Nur GC zur Reaktionskontrolle gemessen, direkt weiter umgesetzt. |

In Analogie zu Beispielen (XXVII-1) und (XXVII-2) erhält man folgende Verbindungen der Formel (XXVII)

| **Struktur** | **Bsp.Nr.** | **¹H-NMR (400 MHz, d₆ DMSO)** |
|---|---|---|
| 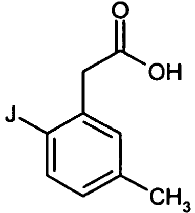 | XXVII-3 | δ = 12.31 (s (br), 1H), 7.69 (d, 1H), 7.18 (d, 1 H), 6.83 (dd, 1H), 3.66 (s, 2H), 2.24 (s, 2H) ppm. |
| 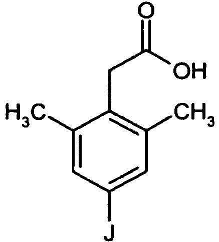 | XXVII-4 | δ = 12.3 (s (br), 1H), 7.40 (s, 2H), 3.55 (s, 2 H), 2.21 (s, 6H) ppm. |
| 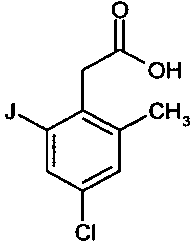 | XXVII-5 | δ = 12.46 (s (br), 1H), 7.75 (d, 1H), 7.33 (d, 1H), 3.80 (s, 2H), 2.31 (s, 3H), ppm. |
| 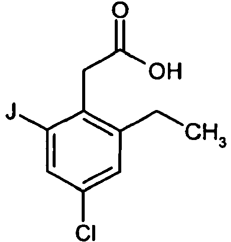 | XXXI-6 | δ = 12.47 (s (br), 1 H), 7.76 (d, 1 H), 7.31 (d, 1 H), 3.81 (s, 2H), 2.64 (q, 2H), 1,11 (t, 3H) ppm. |
| 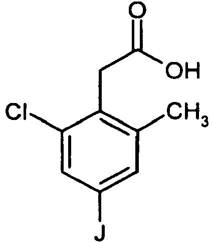 | XXVII-7 | δ = 12.50 (s (br), 1 H), 7.65 (d, 1 H), 7.58 (d, 1H), 3.71 (s, 2H), 2.25 (s, 3H), ppm. |
| 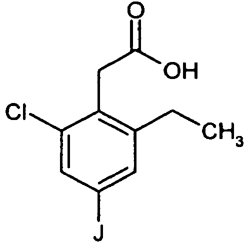 | XXVII-8 | δ = 12.41 (s (br), 1H), 7.66 (d, 1H), 7.55 (d, 1H), 3.72 (s, 2H), 2.63 (q, 2H), 1.11 (t, 3H) ppm. |

| | | |
|---|---|---|
| * Die Bestimmung der in den voran stehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C. | | |

Die Bestimmung erfolgt im sauren Bereich bei pH 2.3 mit 0,1 % wässriger Phosphorsäure und Acetonitril als Eluenten; linearer Gradient von 10 % Acetonitril bis 95 % Acetonitril.

Die Bestimmung mit der LC-MS im sauren Bereich erfolgt bei pH 2,7 mit 0,1 % wässriger Ameisensäure und Acetonitril (enthält 0,1 % Ameisensäure) als Eluenten; linearer Gradient von 10 % Acetonitril bis 95 % Acetonitril.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigtem Alkan-2-onen (mit 3 bis 16 Kohlenstoffatomen), deren logP-Werte bekannt sind (Bestimung der logP-Werte anhand der Retentionszeiten durch lineare Interpolation zwischen zwei aufeinander folgenden Alkanonen).

Die lambda-max-Werte wurde an Hand der UV-Spektren von 200 nm bis 400 nm in den Maxima der chromatographischen Signale ermittelt.

### Anwendungsbeispiele

### Beispiel A

### Phaedon-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele mit einer Aufwandmenge von 500 g/ha eine Wirksamkeit von ≥ 80 %.

I-1-a-1, I-1-a-2, I-1-a-3, I-1-a-4, I-1-a-5, I-1-a-11, I-1-a-12, I-1-a-13, 1-1 -a- 14, I-1-a-18, I-1-a-19, I-1-a-21, I-1-a-23, I-1-b-1, I-1-b-7, I-1-b-11, I-1-b-18, I-1-c-4, I-1-c-7, I-1-c-8, I-1-c-9, I-1-c-I 0, I-1-c-11, I-1-c-17, I-1-c-20,1.

### Beispiel B

### Myzus-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele mit einer Aufwandmenge von 500 g/ha eine Wirksamkeit von ≥ 80 %.

I-1-a-1, I-1-a-2, I-1-a-3, I-1-a-4, I-1-a-5, I-1-a-7, I-1-a-8, I-1-a-9, I-1-a-11, I-1-a-18, I-1-a-19, I-1-a-20, I-1-a-22, I-1-a-24, I-1-b-1, I-1-b-2, I-1-b-9, I-1-b-11, I-1-c-1, I-1-c-2, I-1-c-3, I-1-c-4, I-1-c-6, I-1-c-7, I-1-c-8, I-1-c-9, I-1-c-12, I-1-c-20.

### Beispiel D

### Tetranychus-Test; OP-resistent/Spritzbehandlung (TETRUR)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele mit einer Aufwandmenge von 100 g/ha eine Wirksamkeit von ≥ 80 %:
I-1-a-2, I-1-a-4, I-1-a-5, I-1-a-7, I-1-a-11, I-1-a-12, I-1-a-18, I-1-a-19, I-1-a-21, I-1-a-21, I-1-a-22, I-1-b-2, I-1-b-18, I-1-c-1, I-1-c-4, I-1-c-5, I-1-c-20,

Bei diesem Test zeigt z.B. die folgende Verbindung des Herstellungsbeispieles mit einer Aufwandmenge von 500 g/ha eine Wirksamkeit von ≥ 80 %:
I-1-a-14.

### Beispiel E

### Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 l/ha unter Zusatz von 0,2 % Netzmittel auf die Oberfläche der Abdeckerde appliziert.

Nach Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

Dabei wurden Avena sativa, Lolium multiflorum und Setaria viridis bei einer Aufwandmenge von 320 g/ha z.B. von folgenden Verbindungen mit ≥ 70 % Wirkung erfasst:
I-1-a-8, I-1-a-14, I-1-a-21, I-1-b-7.

### Beispiel F

### Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten Testverbindungen werden dann als wässrige Suspension mit einer Wasseraufwandmenge von umgerechnet 800 l/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

Dabei wurden Avena sativa, Echinochloa crus-galli, Lolium multiflorum und Setaria viridis bei einer Aufwandmenge von 320 g/ha z. B. von folgenden Verbindungen mit ≥ 70 % Wirkung erfasst:
I-1-a-8, I-1-a-11, I-1-a-12, I-1-a-13, I-1-a-14, I-1-a-21, I-1-c-1, I-1-c-6, I-1-c-11, I-1-c-12.

### Beispiel G

### Herbizide Wirkung im Nachauflauf

Samen von mono- bzw. dikotylen Unkraut- bzw. Kulturpflanzen werden in Holzfasertöpfen oder in Plastiktöpfen in sandigem Lehmboden ausgelegt, mit Erde abgedeckt und im Gewächshaus, während der Vegetationsperiode auch im Freien ausserhalb des Gewächshauses, unter guten Wachstumsbedingungen angezogen. 2 bis 3 Wochen nach der Aussaat werden die Versuchspflanzen im Ein- bis Dreiblattstadium behandelt. Die als Spritzpulver (WP) oder Flüssigkeit (EC) formulierten Testverbindungen werden in verschiedenen Dosierungen mit einer Wasseraufwandmenge von umgerechnet 300 l/ha unter Zusatz von Netzmittel (0,2 bis 0,3 %) auf die Pflanzen und die Bodenoberfläche gespritzt. 3 bis 4 Wochen nach Behandlung der Versuchspflanzen wird die Wirkung der Präparate visuell im Vergleich zu behandelten Kontrollen bonitiert (herbizide Wirkung in Prozent (%): 100 % Wirkung = Pflanzen sind abgestorben, 0 % Wirkung = wie Kontrollpflanzen).

### Verwendung von Safenem

Soll zusätzlich getestet werden, ob Safener die Pflanzenverträglichkeit von Testsubstanzen bei den Kulturpflanzen verbessern können, werden folgende Möglichkeiten für die Anwendung des Safeners verwendet:
- Samen der Kulturpflanzen werden vor der Aussaat mit der Safenersubstanz gebeizt (Angabe der Safenermenge in Prozent bezogen auf das Samengewicht)
- Kulturpflanzen werden vor Anwendung der Testsubstanzen mit dem Safener mit einer bestimmten Hektaraufwandmenge gespritzt (üblicherweise 1 Tag vor Anwendung der Prüfsubstanzen)
- der Safener wird zusammen mit der Testsubstanz als Tankmischung appliziert (Angabe der Safenermenge in g/ha oder als Verhältnis zum Herbizid).

Durch Vergleich der Wirkung von Testsubstanzen auf Kulturpflanzen, welche ohne und mit Safener behandelt wurden, kann die Wirkung der Safenersubstanz beurteilt werden.

### Gefäßversuche mit Getreide im Gewächshaus

### Mefenpyr 1 Tag vor Herbizidapplikation

| 10 Tage nach Applikation | | |
|---|---|---|
| | Aufwandmenge g ai/ha | Sommerweizen beobachtet (%) |
| Beispiel I-1-a-3 | 100 | 40 |
| | 50 | 30 |
| | 25 | 20 |
| | 12,5 | 10 |
| Beispiel I-1-a-3 + Mefenpyr | 100 + 100 | 20 |
| | 50 + 1 00 | 10 |
| | 25 + 100 | 5 |
| | 12,5 + 100 | 0 |

| 10 Tage nach Applikation | | |
|---|---|---|
| | Aufwandmenge g ai/ha | Sommerweizen beobachtet (%) |
| Beispiel- I-1-c-1 | 12,5 | 50 |
| Beispiel I-1-c-1 + Mefenpyr | 12,5 + 100 | 15 |

### Beispiel H

### Grenzkonzentrations-Test / Bodeninsekten - Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel I

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 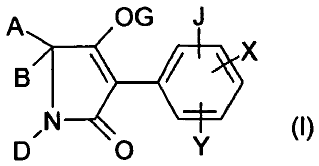 in welcher G für Wasserstoff steht und J, X, Y, D, A und B die in der Tabelle angegebenen Bedeutungen haben:
| **J** | **X** | **Y** | **D** | **A** | **B** |
|---|---|---|---|---|---|
| 4-J | 2-C₂H₅ | 6-CH₃ | H | -(CH₂)₂-CHOCH₃-(CH₂)₂- | |
| 2-J | 4-CH₃ | 6-CH₃ | H | -(CH₂)₂-CHCH₃-(CH₂)₂- | |
| 2-J | 4-Cl | 6-CH3 | H | -(CH₂)₂-CHOCH₃-(CH₂)₂- | |
| 2-J | 5-CH3 | H | H | -(CH₂)₂-CHOCH₃-(CH₂)₂- | |
| 2-J | 5-CH₃ | H | H | -(CH₂)₂-O-(CH₂)₂- | |
| 2-J | 4-Cl | 6-C₂H₅ | H | -(CH₂)₂-O-(CH₂)₂- | |
| 2-J | 4-Cl | 6-C₂H₅ | H | -(CH₂)₂-CHOCH₃-(CH₂)₂- | |
| 2-J | H | H | H | -(CH₂)₂-CHOCH₃-(CH₂)₂- | |
| 2-J | 4-CH3 | 6-CH3 | H | -(CH₂)₂-CHCH₃-(CH₂)₂- | |
| 2-J | 4-Cl | 6-CH₃ | H | -(CH₂)₂-O-(CH₂)₂- | |
| 3-J | 6-CH₃ | H | H | -(CH₂)₂-CHOCH₃-(CH₂)₂- | |
| 4-J | 2-C₂H₅ | 6-CH₃ | H | -(CH₂)₂-O-(CH₂)₂- | |
| 4-J | 2-C₂H₅ | 6-CH₃ | H | CH₃ | CH₃ |
| 4-J | 2-C₂H₅ | 6-CH3 | H | 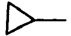 | CH₃ |
| 4-J | 2-C₂H₅ | 6-CH3 | H | n-C₃H₇ | CH₃ |
| 4-J | 2-C₂H₅ | 6-CH₃ | 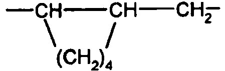 | | H |
| 4-J | 2-C₂H₅ | 6-CH₃ | H | -(CH2)5- | |
| 4-J | 2-C₂H₅ | 6-CH₃ | H | -(CH₂)₂-CHCH₃-(CH₂)₂- | |
| 4-J | 2-CH₃ | 6-CH₃ | H | -(CH₂)₂-CHOCH₃-(CH₂)₂- | |
| 4-J | 2-CH₃ | 6-CH₃ | H | -(CH₂)₂-O-(CH₂)₂- | |
| 4-J | 2-C₂H₅ | 6-Cl | H | -(CH₂)₂-O-(CH2)₂- | |
| 4-J | 2-CH₃ | 6-CH₃ | H | -CH₂-CHOC₄H₉-(CH₂)₃- | |
| 4-J | 2-CH₃ | 6-Cl | H | -(CH₂)₂-O-(CH₂)₂- | |
| 4-J | 2-C₂H₅ | 6-CH₃ | H | -CH₂-CHOC₄H₉-(CH₂)₃- | |
| 4-J | 2-C₂H₅ | 6-Cl | H | -(CH₂)₂-CHOCH₃-(CH₂)₂- | |
| 4-J | 2-CH₃ | 6-Cl | H | -(CH₂)₂-CHOCH₃-(CH₂)₂- | |
| 4-J | 2-C₂H₅ | 6-CH₃ | H | i-C3H₇ | CH₃ |
| 4-J | 2-C₂H₅ | 6-CH₃ | H | i-C₄H₉ | CH₃ |
G für 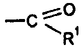 steht und J, X, Y, D, A, B und R¹ die in der Tabelle angegebenen Bedeutungen haben:
| **J** | **X** | **Y** | **D** | **A** | **B** | **R¹** |
|---|---|---|---|---|---|---|
| 2-J | 5-CH₃ | H | H | -(CH₂)₂-CHOCH₃-(CH₂)₂- | | i-C₃H₇ |
| 2-J | 4-Cl | 6-C₂H₅ | H | -(CH₂)₂-CHOCH₃-(CH₂)₂- | | H₃C-O-CH₂- |
| 2-J | 4-Cl | 6-C₂H₅ | H | -(CH₂)₂-O-(CH₂)₂- | | H₃C-O-CH₂- |
| 4-J | 2-C₂H₅ | 6-CH₃ | H | -(CH₂)₂-O-(CH₂)₂- | | i-C₃H₇ |
| 4-J | 2-C₂H₅ | 6-CH₃ | H | C₃H₇ | CH₃ | i-C₃H₇ |
| 4-J | 2-CH₃ | 6-CH₃ | H | -CH₂-CH-OC₄H₉-(CH₂)₃- | | H₃C-O-CH₂- |
| 4-J | 2-CH₃ | 6-CH₃ | H | -CH₂-CHOC₄H₉-(CH₂)₃- | | 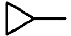 |
| 4-J | 2-CH₃ | 6-CH₃ | H | -CH₂-CHOC₄H₉-(CH₂)₃- | | Cl-CH₂- |
| 4-J | 2-CH3 | 6-CH₃ | H | -CH₂-CHOC₄H₉-(CH₂)₃- | | 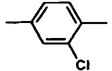 |
| 4-J | 2-C₂H₅ | 6-CH₃ | H | -(CH₂)₂-O-(CH₂)₂- | | H₃C-O-CH₂- |
| 4-J | 2-C₂H₅ | 6-Cl | H | -(CH₂)₂-O-(CH₂)₂- | | H₃C-O-CH₂- |
| 4-J | 2-C₂H₅ | 6-CH₃ | H | -CH₂-CHOC₄H₉-(CH₂)₃- | | i-C₃H₇ |
| 4-J | 2-C₂H₅ | 6-CH₃ | H | i-C₃H₇ | CH3 | i-C₃H₇ |
| 4-J | 2-C₂H₅ | 6-CH₃ | H | -CH₂-CHOC₄H₉-(CH₂)₃- | | H₃C-O-CH₂- |
| 4-J | 2-C₂H₅ | 6-CH₃ | H | -(CH₂)₅- | | H₃C-O-CH₂- |
| 4-J | 2-C₂H₅ | 6-CH₃ | H | -(CH₂)₅- | | i-C₃H₇ |
| 4-J | 2-C₂H₅ | 6-CH₃ | H | CH₃ | CH₃ | i-C₃H₇ |
| 4-J | 2-C₂H₅ | 6-CH₃ | H | -(CH₂)₅- | | t-C₄H₉ |
| 4-J | 2-C₂H₅ | 6-CH₃ | H | i-C₃H₇ | CH₃ | H₃C-O-CH₂- |
| 4-J | 2-C₂H₅ | 6-CH₃ | H | CH₃ | CH₃ | H₃C-O-CH₂- |
| 4-J | 2-C₂H₅ | 6-CH₃ | H | i-C₃H₇ | CH₃ | i-C₃H₇ |
| 2-J | 5-CH₃ | H | H | -(CH₂)₂-CHOCH₃-(CH₂)₂- | | 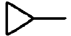 |
G für 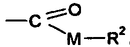 steht und J, X, Y, D, A, B M und R² die in der Tabelle angegebenen Bedeutungen haben:
| **J** | **X** | **Y** | **D** | **A** | **B** | **M** | **R²** |
|---|---|---|---|---|---|---|---|
| 2-J | 5-CH₃ | H | H | -(CH₂)₂-CHOCH₃-(CH₂)₂- | | O | C₆H₅-CH₂- |
| 2-J | 4-Cl | 6-CH₃ | H | -(CH₂)₂-CHOCH₃-(CH₂)₂- | | O | C₂H₅ |
| 2-J | 5-CH₃ | H | H | -(CH₂)₂-CHOCH₃-(CH₂)₂- | | O | C₂H₅ |
| 2-J | 4-Cl | 6-C₂H₅ | H | -(CH₂)₂-O-(CH₂)₂- | | O | C₂H₅ |
| 2-J | 4-Cl | 6-C₂H₅ | H | -(CH₂)₂-CHOCH₃-(CH₂)₂- | | O | C₂H₅ |
| 3-J | 6-CH₃ | H | H | -(CH₂)₂-CHOCH₃-(CH₂)₂- | | O | C₂H₅ |
| 4-J | 2-CH₃ | 6-CH₃ | H | -(CH₂)₂-CHOCH₃-(CH₂)₂- | | O | C₂H₅ |
| 4-J | 2-CH₃ | 6-Cl | H | -(CH₂)₂-O-(CH₂)₂- | | O | C₂H₅ |
| 4-J | 2-C₂H₅ | 6-CH₃ | H | n-C₃H₇ | CH₃ | O | C₂H₅ |
| 4-J | 2-C₂H₅ | 6-CH₃ | H | 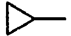 | CH₃ | O | C₂H₅ |
| 4-J | 2-C₂H₅ | 6-CH₃ | H | -(CH₂)₂-O-(CH₂)₂- | | O | C₂H₅ |
| 4-J | 2-C₂H₅ | 6-Cl | H | -(CH₂)₂-O-(CH₂)₂- | | O | C₂H₅ |
| 4-J | 2-CH₃ | 6-CH₃ | H | -CH₂-CHOC₄H₉-(CH₂)₃- | | O | C₆H₅-CH₂- |
| 4-J | 2-CH₃ | 6-CH₃ | H | -CH₂-CHOC₄H₉-(CH₂)₃- | | O | CH₂=CH-CH₂- |
| 4-J | 2-CH₃ | 6-CH₃ | H | -CH₂-CHOC₄H₉-(CH₂)₃- | | O | C₂H₅ |
| 4-J | 2-C₂H₅ | 6-CH₃ | H | -(CH₂)₅- | | O | C₂H₅ |
| 4-J | 2-C₂H₅ | 6-CH₃ | H | CH₃ | CH₃ | O | C₂H₅ |
| 4-J | 2-C₂H₅ | 6-CH₃ | H | i-C₄H₉ | CH₃ | O | C₂H₅ |
| 4-J | 2-CH₃ | 6-CH₃ | H | -(CH₂)₂-O-(CH₂)₂- | | O | C₂H₅ |
| 4-J | 2-C₂H₅ | 6-CH₃ | H | -(CH₂)₂-CHOCH₃ -(CH₂)₂- | | O | C₂H₅ |

2. Verfahren zur Herstellung von Verbindungen der Formel (I) gemäß Anspruch 1 **dadurch gekennzeichnet, dass** man zum Erhalt von
A) Verbindungen der Formel (I-1-a) 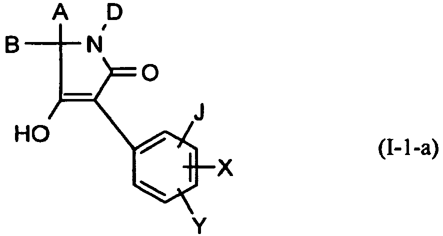 in welcher
A, B, D, J, X und Y die oben angegebenen Bedeutungen haben,
Verbindungen der Formel (II) 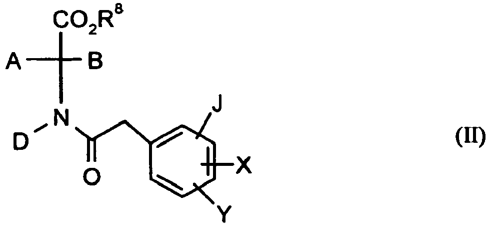 in welcher
A, B, D, J, X und Y die oben angegebenen Bedeutungen haben,
und
R⁸ für Alkyl steht,
in Gegenwart eines Verdünnungsmittels und in Gegenwart einer Base intramolekular kondensiert,
(I) Verbindungen der oben gezeigten Formel (I-1-b), in welchen A, B, D, J, R¹, X, und Y die oben angegebenen Bedeutungen haben, Verbindungen der oben gezeigten Formel (I-1-a), in welchen A, B, D, J, X und Y die oben angegebenen Bedeutungen haben, jeweils
(α) mit Säurehalogeniden der Formel (XIII) 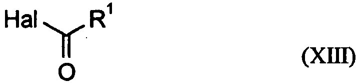 in welcher
R¹ die oben angegebene Bedeutung hat und
Hal für Halogen steht
oder
(ß) mit Carbonsäureanhydriden der Formel (XIV)
R¹-CO-O-CO-R¹ (XIV)
in welcher
R¹ die oben angegebene Bedeutung hat,
gegebenenfalls in Gegenwart eines Verdünnungsmittels und gegebenenfalls in Gegenwart eines Säurebindemittels umsetzt,
(J) Verbindungen der oben gezeigten Formel (I-1-c), in welchen A, B, D, J, R², M, X und Y die oben angegebenen Bedeutungen haben und L für Sauerstoff steht, Verbindungen der oben gezeigten Formel (I-1-a), in welchen A, B, D, J, X und Y die oben angegebenen Bedeutungen haben, jeweils
mit Chlorameisensäureestern oder Chlorameisensäurethioestern der Formel (XV)
R²-M-CO-Cl (XV)
in welcher
R² und M die oben angegebenen Bedeutungen haben,
gegebenenfalls in Gegenwart eines Verdünnungsmittels und gegebenenfalls in Gegenwart eines Säurebindemittels umsetzt,
(P) Verbindungen der oben gezeigten Formel (I-I-a), in welchen A, B, D, J, X und Y die oben angegebene Bedeutung haben, Verbindungen der Formel (I-1-a'), in welchen A, B, D, X und Y die oben genannte Bedeutung haben und W' für Brom steht 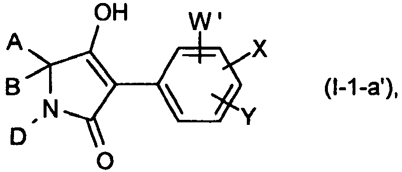
α) mit Metalliodiden gegebenenfalls in Gegenwart eines Verdünnungsmittels, eines Cu-(I)-Salzes und einer Base umsetzt, oder
β) mit Metallorganylen einen Halogenmetallaustausch durchführt und das entstehende Anion mit Jodierungsreagenzien quencht.

3. Verwendung von Verbindungen der Formel (I) gemäß Anspruch 1 zur Herstellung von Schädlingsbekämpfungsmitteln und/oder Herbiziden.

4. Schädlingsbekämpfungsmittel und/oder Herbizide, **gekennzeichnet durch** einen Gehalt an mindestens einer Verbindung der Formel (I) gemäß Anspruch 1.

5. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge und/oder ihren Lebensraum einwirken lässt, ausgenommen sind therapeutische Verfahren.

6. Verwendung von Verbindungen der Formel (I) gemäß Anspruch 1 zur Bekämpfung von tierischen Schädlingen und/oder unerwünschtem Pflanzenbewuchs, ausgenommen die Verwendung in therapeutischen Verfahren.

7. Verfahren zur Herstellung von Schädlingsbekämpfungsmitteln und/oder Herbiziden, **dadurch gekennzeichnet, dass** man Verbindungen der Formel (I) gemäß Anspruch 1 mit Streckmitteln und/oder oberflächenaktiven Stoffen vermischt.

8. Mittel enthaltend einen wirksamen Gehalt an einer Wirkstoffkombination umfassend als Komponenten
(a') mindestens ein substituiertes, cyclisches Ketoenol der Formel (I), in welcher A, B, D, G, J, X und Y die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thio-carbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-a-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxazolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluorphenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxymalonsäure-diethylester, 5-Chlor-chinoxalin-8-oxy-malonsäure-diallylester, 5-Chlorchinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlorphenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxy-benzoyl)-4-[(methylamino-carbonyl)amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)phenyl]-3,3-dimethylharnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen
der allgemeinen Formel (IIa) 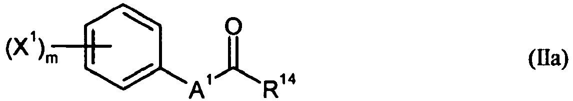 oder der allgemeinen Formel (IIb) 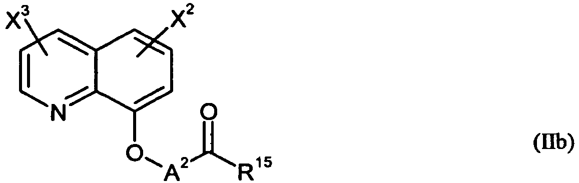 oder der Formel (IIc) 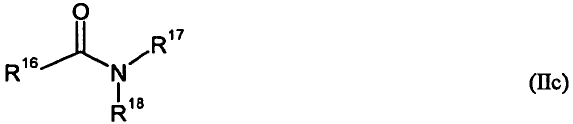 wobei
m für eine Zahl 0, 1, 2, 3, 4 oder 5 steht,
A¹ für eine der nachstehend skizzierten divalenten heterocyclischen Gruppierungen steht, 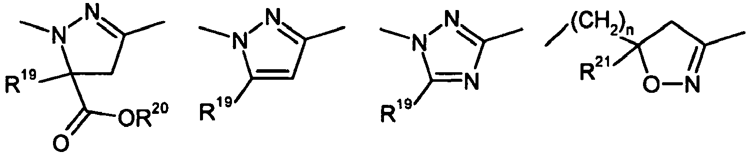
n für eine Zahl 0, 1, 2, 3, 4 oder 5 steht,
A² für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)amino steht,
R¹⁵ für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
R¹⁶ für jeweils gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carbocyclus bilden, substituiertes C₃-C₆-Alkandiyl oder C₂-C₅-Oxaalkandiyl steht,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenancyl, C₁-C₄-Alkoxy oder C₁-C₄-Halogenalkoxy steht,
und/oder die folgenden durch allgemeine Formeln definierten Verbindungen
der allgemeinen Formel (IId) 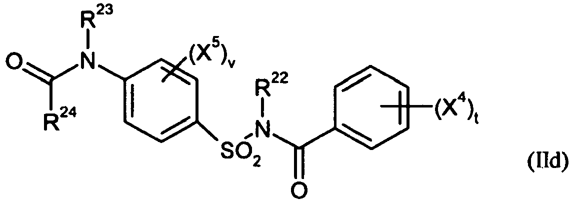 oder der allgemeinen Formel (IIe) 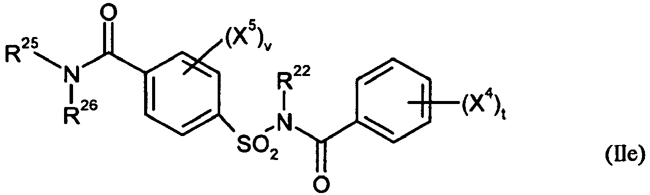 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

9. Mittel nach Anspruch 8, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen Ile-5 oder Ile-11.

10. Mittel nach Anspruch 8 bei dem die Kulturpflanzen-Verträglichkeit verbessernde Verbindung Mefenpyr-diethyl ist.

11. Verfahren zum Bekämpfen von unerwünschtem Pflanzenwuchs, **dadurch gekennzeichnet, dass** man ein Mittel gemäß Anspruch 8 auf die Pflanzen oder ihre Umgebung einwirken lässt.

12. Verwendung eines Mittels gemäß Anspruch 8 zum Bekämpfen von unerwünschten Pflanzenwuchs.

13. Verfahren zur Bekämpfung von unerwünschten Pflanzenbewuchs, **dadurch gekennzeichnet, dass** man eine Verbindung der Formel (I) gemäß Anspruch 1 und eine die Kulturpflanzenverträglichkeit verbessernde Verbindung gemäß Anspruch 8 in zeitlich naher Abfolge getrennt oder in Mischung auf die Pflanzen oder ihre Umgebung einwirken lässt.

14. Verbindungen der Formel (II) 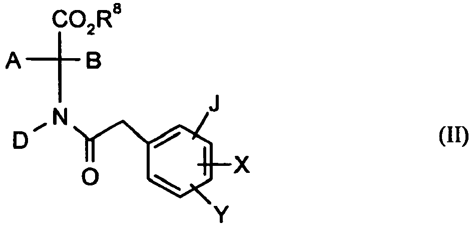 in welcher
A, B, D, J, X und Y die oben angegebenen Bedeutungen haben,
und
R⁸ für Alkyl steht.

15. Verbindungen der Formel (XXV) 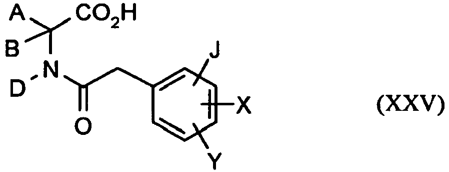 in welcher
A, B, D, J, X und Y die oben angegebenen Bedeutungen haben.

16. Verbindungen der Formel (XXIV) 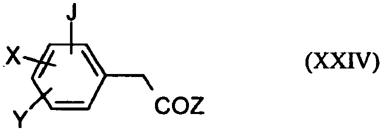 in welcher
J, X und Y die oben angegebenen Bedeutungen haben,
und Z für eine durch Carbonsäureaktivierungsreagenzien, Phosphorylierungsreagenzen, Halogenierungsmittel, Phosgen oder Chlorameisensäsureester eingeführte Abgangsgruppe steht,
ausgenommen 2-Jodo-5-methylphenylessigsäurechlorid.

17. Verbindungen der Formel (XXIX) 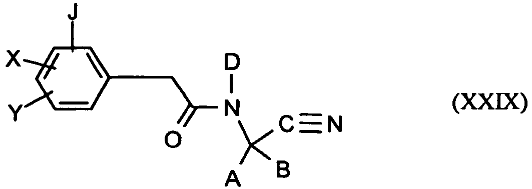 in welcher
A, B, D, J, X und Y die oben angegebenen Bedeutungen haben.

18. Verbindungen der Formel (XXVII) 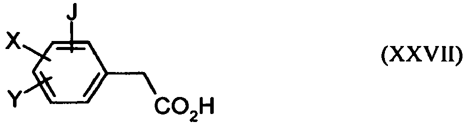 in welcher
J, X und Y die in der Tabelle angegebenen Bedeutungen haben:
| J | X | Y |
|---|---|---|
| 4-J | 2- C₂H₅ | 6- CH₃ |
| 4-J | 2-CH3 | 6-CH3 |
| 2-J | 4-Cl | 6-CH₃ |
| 2-J | 4-Cl | 6-C₂H₅ |
| 4-J | 2-Cl | 6-CH₃ |
| 4-J | 2-Cl | 6-C₂H₅ |

19. Verbindungen der Formel (XXXI) 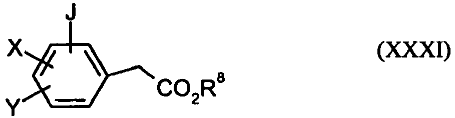 in welcher
J, X und Y die in der Tabelle angegebenen Bedeutungen haben:
| **J** | **X** | **Y** | **R⁸** |
|---|---|---|---|
| 4-J | 2- C₂H₅ | 6- CH₃ | CH₃ |
| 4-J | 2-CH₃ | 6-CH₃ | CH₃ |
| 2-J | 4-Cl | 6-CH₃ | CH₃ |
| 2-J | 4-Cl | 6-C₂H₅ | CH₃ |
| 4-J | 2-Cl | 6-CH₃ | CH₃ |
| 4-J | 2-Cl | 6-C₂H₅ | CH₃ |
und R⁸ für C₁-C₄-Alkyl steht.

## Claims

1. A compound of the formula (I) 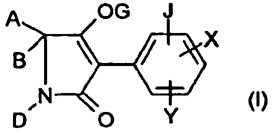 in which G represents hydrogen and J, X, Y, D, A and B have the meanings given in the table:
| **J** | **X** | **Y** | **D** | **A** | **B** |
|---|---|---|---|---|---|
| 4-1 | 2-C₂H₅ | 6-CH₃ | H | -(CH₂)₂-CHOCH₃-(CH₂)₂- | |
| 2-1 | 4-CH₃ | 6-CH₃ | H | -(CH₂)₂-CHCH₃-(CH₂)₂- | |
| 2-I | 4-Cl | 6-CH₃ | H | -(CH₂)₂-CHOCH₃-(CH₂)₂- | |
| 2-1 | 5-CH₃ | H | H | -(CH₂)₂-CHOCH₃-(CH₂)₂- | |
| 2-1 | 5-CH₃ | H | H | -(CH₂)₂-O-(CH₂)₂- | |
| 2-1 | 4-Cl | 6-C₂H₅ | H | -(CH₂)₂-O-(CH₂)₂- | |
| 2-I | 4-Cl | 6-C₂H₅ | H | -(CH₂)₂-CHOCH₃-(CH₂)₂- | |
| 2-1 | H | H | H | -(CH₂)₂-CHOCH₃-(CH₂)₂- | |
| 2-I | 4-CH₃ | 6-CH₃ | H | -(CH₂)₂-CHCH₃-(CH₂)₂- | |
| 2-1 | 4-Cl | 6-CH₃ | H | -(CH₂)₂-O-(CH₂)₂- | |
| 3-I | 6-CH₃ | H | H | -(CH₂)₂-CHOCH₃-(CH₂)₂- | |
| 4-1 | 2-C₂H₅ | 6-CH₃ | H | -(CH₂)₂-O-(CH₂)₂- | |
| 4-1 | 2-C₂H₅ | 6-CH₃ | H | CH₃ | CH₃ |
| 4-1 | 2-C₂H₅ | 6-CH₃ | H | 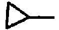 | CH₃ |
| 4-I | 2-C₂H₅ | 6-CH₃ | H | n-C₃H₇ | CH₃ |
| 4-I | 2-C₂H₅ | 6-CH₃ | 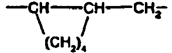 | | H |
| 4.I | 2-C₂H₅ | 6-CH₃ | H | -(CH₂)₅- | |
| 4-I | 2-C₂H₅ | 6-CH3 | H | -(CH₂)₂-CHCH₃-(CH₂)₂- | |
| 4-I | 2-CH₃ | 6-CH₃ | H | -(CH₂)₂-CHOCH₃-(CH₂)₂- | |
| 4-1 | 2-CH₃ | 6-CH₃ | H | -(CH₂)₂-O-(CH₂)₂- | |
| 4-I | 2-C₂H₅ | 6-Cl | H | -(CH₂)₂-O-(CH₂)₂- | |
| 4-I | 2-CH₃ | 6-CH₃ | H | -CH₂-CHOC₄H₉-(CH₂)₃₋ | |
| 4-I | 2-CH₃ | 6-Cl | H | -(CH₂)₂-O-(CH₂)₂- | |
| 4-I | 2-C₂H₅ | 6-CH₃ | H | -CH₂-CHOC₄H₉-(CH₂)₃- | |
| 4-1 | 2-C₂H₅ | 6-Cl | H | -(CH₂)₂-CHOCH₃-(CH₂)₂- | |
| 4-1 | 2-CH₃ | 6-Cl | H | -(CH₂)₂-CHOCH₃-(CH₂)₂- | |
| 4-I | 2-C₂H₅ | 6-CH₃ | H | i-C₃H₇ | CH₃ |
| 4-I | 2-C₂H₅ | 6-CH₃ | H | i-C₄H₉ | CH₃ |
G represents 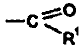 and J, X, Y, D, A, B and R¹ have the meanings given in the table:
| **J** | **X** | **Y** | **D** | **A** | **B** | **R¹** |
|---|---|---|---|---|---|---|
| 2-1 | 5-CH₃ | H | H | -(CH₂)₂-CHOCH₃-(CH₂)₂- | | i-C₃H₇ |
| 2-1 | 4-Cl | 6-C₂H₅ | H | -(CH₂)₂-CHOCH₃-(CH₂)₂- | | H₃C-O-CH₂- |
| 2-1 | 4-Cl | 6-C₂H₅ | H | -(CH₂)₂-O-CH₂)₂- | | H₃C-OCH₂- |
| 4-I | 2-C₂H₅ | 6-CH₃ | H | -(CH₂)₂-O-(CH₂)₂- | | i-C₃H₇ |
| 4-I | 2-C₂H₅ | 6-CH₃ | H | C₃H₇ | CH₃ | i-C₃H₇ |
| 4-1 | 2-CH₃ | 6-CH₃ | H | -CH₂-CH-OC₄H₉-(CH₂)₃- | | H₃C-O-CH₂- |
| 4-1 | 2-CH₃ | 6-CH₃ | H | -CH₂-CHOC₄H₉-(CH₂)₃- | | 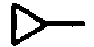 |
| 4-1 | 2-CH₃ | 6-CH₃ | H | -CH₂-CHOC₄C₄H₉-(CH₂)₃- | | Cl-CH₂- |
| 4-I | 2-CH₃ | 6-CH₃ | H | -CH₂-CHOC₄H₉-(CH₂)₃- | | 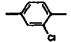 |
| 4-I | 2-C₂H₅ | 6-CH₃ | H | -(CH₂)₂-O-(CH₂)₂- | | H₃CO-CH₂- |
| 4-I | 2-C₂H₅ | 6-Cl | H | -(CH₂)₂-O-(CH₂)₂- | | H₃C-O-OH₂- |
| 4-I | 2-C₂H₅ | 6-CH₃ | H | -CH₂-CHOC₄H₉-(CH₂)₃- | | i-C₃H₇ |
| 4-I | 2-C₂H₅ | 6-CH₃ | H | i-C₃H₇ | CH₃ | i-C₃H₇ |
| 4-I | 2-C₂H₅ | 6-CH₃ | H | -CH₂-CHOC₄H₉-(CH₂)₃- | | H₃C-O-CH₂- |
| 4-I | 2-C₂H₅ | 6-CH₃ | H | -(CH₂)₅- | | H₃C-O-CH₂- |
| 4-I | 2-C₂H₅ | 6-CH₃ | H | -(CH₂)₅- | | i-C₃H₇ |
| 4-1 | 2-C₂H₅ | 6-CH₃ | H | CH₃ | CH₃ | i-C₃H₇ |
| 4-I | 2-C₂H₅ | 6-CH₃ | H | -(CH₂)₅- | | t-C₄H₉ |
| 4-1 | 2-C₂H₅ | 6-CH₃ | H | i-C₃H₇ | CH₃ | H₃C-O-CH₂- |
| 4-1 | 2-C₂H₅ | 6-CH₃ | H | CH₃ | CH₃ | H₃C-O-CH₂- |
| 4-1 | 2-C₂H₅ | 6-CH₃ | H | i-C₃H₇ | CH₃ | i-C₃H₇ |
| 2-I | 5-CH₃ | H | H | -(CH₂)₂-CHOCH₃-(CH₂)₂- | | 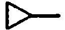 |
G represents 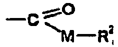 and J, X, Y, D, A, B M and R² have the meanings given in the table:
| **J** | **X** | **Y** | **D** | **A** | **B** | **M** | **R²** |
|---|---|---|---|---|---|---|---|
| 2-I | 5-CH₃ | H | H | -(CH₂)₂-CHOCH₃-(CH₂)₂- | | O | C₆H₅-CH₂- |
| 2-I | 4-Cl | 6-CH₃ | H | -(CH₂)₂-CHOCH₃-(CH₂)₂- | | O | C₂H₅ |
| 2-I | 5-CH₃ | H | H | -(CH₂)₂-CHOCH₃-(CH₂)₂- | | O | C₂H₅ |
| 2-I | 4-Cl | 6-C₂H₅ | H | -(CH₂)₂-O-(CH₂)₂- | | O | C₂H₅ |
| 2-I | 4-Cl | 6-C₂H₅ | H | -(CH₂)₂-CHOCH₃-(CH₂)₂- | | O | C₂H₅ |
| 3-I | 6-CH₃ | H | H | -(CH₂)₂-CHOCH₃-(CH₂)₂- | | O | C₂H₅ |
| 4-I | 2-CH₃ | 6-CH₃ | H | -(CH₂)₂-CHOCH₃-(CH₂)₂- | | O | C₂H₅ |
| 4-I | 2-CH₃ | 6-Cl | H | -(CH₂)₂-O-(CH₂)₂- | | O | C₂H₅ |
| 4-I | 2-C₂H₅ | 6-CH₃ | H | n-C₃H₇ | CH₃ | O | C₂H₅ |
| 4-I | 2-C₂H₅ | 6-CH₃ | H | 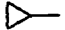 | CH₃ | O | C₂H₅ |
| 4-1 | 2-C₂H₅ | 6-CH₃ | H | -(CH₂)₂-O-(CH₂)₂- | | O | C₂H₅ |
| 4-I | 2-C₂H₅ | 6-Cl | H | -(CH₂)₂-O-(CH₂)₂- | | O | C₂H₅ |
| 4-I | 2-CH₃ | 6-CH₃ | H | -CH₂-CHOC₄H₉-(CH₂)₃- | | O | C₆H₅-CH₂- |
| 4-I | 2-CH₃ | 6-CH₃ | H | -CH₂-CHOC₄H₉-(CH₂)₃- | | O | CH₂=CH-CH₂- |
| 4-I | 2-CH₃ | 6-CH₃ | H | -CH₂-CHOC₄H₉-(CH₂)₃- | | O | C₂H₅ |
| 4-I | 2-C₂H₅ | 6-CH₃ | H | -(CH₂)₅- | | O | C₂H₅ |
| | | | | | | | |
| **J** | **X** | **Y** | **D** | **A** | **B** | **M** | **R²** |
|---|---|---|---|---|---|---|---|
| 4-I | 2-C₂H₅ | 6-CH₃ | H | CH₃ | CH₃ | O | C₂H₅ |
| 4-1 | 2-C₂H₅ | 6-CH₃ | H | i-C₄H₉ | CH₃ | O | C₂H₅ |
| 4-1 | 2-CH₃ | 6-CH₃ | H | -(CH₂)₂-O-(CH₂)₂- | | O | C₂H₅ |
| 4-1 | 2-C₂H₅ | 6-CH₃ | H | -(CH₂)₂-CHOCH₃ -(CH₂)₂- | | O | C₂H₅ |

2. A process for preparing compounds of the formula (I) as claimed in claim 1, wherein, to obtain
A) compounds of the formula (I-1-a) 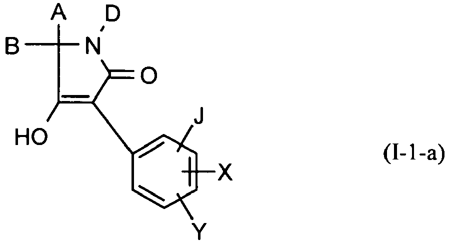 in which
A, B, D, J, X and Y are as defined above,
compounds of the formula (II) 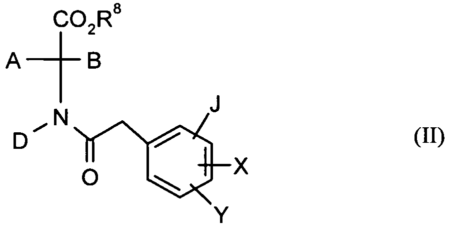 in which
A, B, D, J, X and Y are as defined above
and
R⁸ represents alkyl
are condensed intramolecularly in the presence of a diluent and in the presence of a base,
(I) compounds of the formula (I-1-b) shown above in which A, B, D, J, R¹, X, and Y are as defined above, compounds of the formula (I-1-a) shown above in which A, B, D, J, X and Y are as defined above are in each case
(α) reacted with acid halides of the formula (XIII) 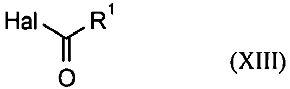 in which
R¹ is as defined above and
Hal represents halogen
or
(β) reacted with carboxylic anhydrides of the formula (XIV)
R¹-CO-O-CO-R¹ (XIV)
in which
R¹ is as defined above,
if appropriate in the presence of a diluent and if appropriate in the presence of an acid binder,
(J) compounds of the formula (I-1-c) to shown above in which A, B, D, J, R², M, X and Y are as defined above and L represents oxygen, compounds of the formula (I-1-a) shown above in which A, B, D, J, X and Y are as defined above are in each case
reacted with chloroformic esters or chloroformic thioesters of the formula (XV)
R²-M-CO-Cl (XV)
in which
R² and M are as defined above,
if appropriate in the presence of a diluent and if appropriate in the presence of an acid binder,
(P) compounds of the formula (I-1-a) shown above in which A, B, D, J, X and Y are as defined above, compounds of the formula (I-1-a') in which A, B, D, X and Y are as defined above and W' represents bromine 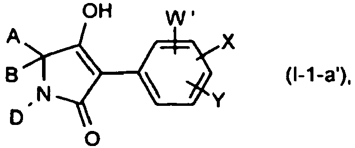
α) are reacted with metal iodides, if appropriate in the presence of a diluent, a Cu(I) salt and a base, or
β) are subjected to a halogen/metal exchange with metal organyls and the anion formed is quenched with iodinating agents.

3. The use of compounds of the formula (I) as claimed in claim 1 for preparing pesticides and/or herbicides.

4. A pesticide and/or herbicide which comprises at least one compound of the formula (I) as claimed in claim 1.

5. A method for controlling animal pests and/or unwanted vegetation which comprises allowing compounds of the formula (I) as claimed in claim 1 to act on pests and/or their habitats, with the exception of therapeutic methods.

6. The use of compounds of the formula (I) as claimed in claim 1 for controlling animal pests and/or unwanted vegetation, with the exception of the use in therapeutic methods.

7. A process for preparing pesticides and/or herbicides which comprises mixing compounds of the formula (I) as claimed in claim 1 with extenders and/or surfactants.

8. A composition comprising an effective amount of an active compound combination comprising, as components,
(a') at least one substituted cyclic ketoenol of the formula (I) in which A, B, D, G, J, X and Y are as defined above
and
(b') at least one crop plant compatibility-improving compound from the following group of compounds:
4-dichloroacetyl-1-oxa-4-azaspiro[4.5]decane (AD-67, MON-4660), 1-dichloroacetylhexahydro-3,3,8a-trimethylpyrrolo[1,2-a]pyrimidin-6(2H)-one (dicyclonon, BAS-145138), 4-dichloroacetyl-3,4-dihydro-3-methyl-2H-1,4-benzoxazine (benoxacor), 1-methylhexyl 5-chloroquinoline-8-oxyacetate (cloquintocet-mexyl - cf. also related compounds in EP-A-86750, EP-A-94349, EP-A-191736, EP-A-492366), 3-(2-chlorobenzyl)-1-(1-methyl-1-phenylethyl)urea (cumyluron), α-(cyanomethoximino)phenylacetonitrile (cyometrinil), 2,4-dichlorophenoxyacetic acid (2,4-D), 4-(2,4-dichlorophenoxy)butyric acid (2,4-DB), 1-(1-methyl-1-phenylethyl)-3-(4-methylphenyl)urea (daimuron, dymron), 3,6-dichloro-2-methoxybenzoic acid (dicamba), S-1-methyl-1-phenylethyl piperidine-1-thiocarboxylate (dimepiperate), 2,2-dichloro-N-(2-oxo-2-(2-propenylamino)ethyl)-N-(2-propenyl)-acetamide (DKA-24), 2,2-dichloro-N,N-di-2-propenylacetamide (dichlormid), 4,6-dichloro-2-phenylpyrimidine (fenclorim), ethyl 1-(2,4-dichlorophenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethylthiazole-5-carboxylate (flurazole), 4-chloro-N-(1,3-dioxolan-2-ylmethoxy)-α-trifluoroacetophenone oxime (fluxofenim), 3-dichloroacetyl-5-(2-furanyl)-2,2-dimethyloxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)ethyl 3,6-dichloro-2-methoxybenzoate (lactidichlor), (4-chloro-o-tolyloxy)acetic acid (MCPA), 2-(4-chloro-o-tolyloxy)propionic acid (mecoprop), diethyl 1-(2,4-dichorophenyl)-4,5-dihydro-5-methyl-1H-pyrazole-3,5-dicarboxylate (mefenpyr-diethyl - cf. also related compounds in WO-A-91/07874), 2-dichloromethyl-2-methyl-1,3-dioxolane (MG-191), 2-propenyl-1-oxa-4-azaspiro[4.5]decane-4-carbodithioate (MG-838), 1,8-naphthalic anhydride, α-(1,3-dioxolan-2-ylmethoximino)phenylacetonitrile (oxabetrinil), 2,2-dichloro-N-(1,3-dioxolan-2-ylmethyl)-N-(2-propenyl)acetamide (PPG-1292), 3-dichloroacetyl-2,2-dimethyloxazolidine (R-28725), 3-dichloroacetyl-2,2,5-trimethyloxazolidine (R-29148), 4-(4-chloro-o-tolyl)butyric acid, 4-(4-chlorophenoxy)butyric acid, diphenylmethoxyacetic acid, methyl diphenylmethoxyacetate, ethyl diphenylmethoxyacetate, methyl 1-(2-chlorophenyl)-5-phenyl-1H-pyrazole-3-carboxylate, ethyl 1-(2,4-dichlorophenyl)-5-methyl-1H-pyrazole-3-carboxylate, ethyl 1-(2,4-dichlorophenyl)-5-isopropyl-1H-pyrazole-3-carboxylate, ethyl 1-(2,4-dichlorophenyl)-5-(1,1-dimethylethyl)-1H-pyrazole-3-carboxylate, ethyl 1-(2,4-dichlorophenyl)-5-phenyl-1H-pyrazole-3-carboxylate (cf. also related compounds in EP-A-269806 and EP-A-333131), ethyl 5-(2,4-dichlorobenzyl)-2-isoxazoline-3-carboxylate, ethyl 5-phenyl-2-isoxazoline-3-carboxylate, ethyl 5-(4-fluorophenyl)-5-phenyl-2-isoxazoline-3-carboxylate (cf. also related compounds in WO-A-91/08202), 1,3-dimethylbut-1-yl 5-chloroquinoline-8-oxyacetate, 4-allyloxybutyl 5-chloroquinoline-8-oxyacetate, 1-allyloxyprop-2-yl 5-chloroquinoline-8-oxyacetate, methyl 5-chloroquinoxaline-8-oxyacetate, ethyl 5-chloroquinoline-8-oxyacetate, allyl 5-chloroquinoxaline-8-oxyacetate, 2-oxoprop-1-yl 5-chloroquinoline-8-oxyacetate, diethyl 5-chloroquinoline-8-oxymalonate, diallyl 5-chloroquinoxaline-8-oxymalonate, diethyl 5-chloroquinoline-8-oxymalonate (cf. also related compounds in EP-A-582198), 4-carboxychroman-4-ylacetic acid (AC-304415, cf. EP-A-613618), 4-chlorophenoxyacetic acid, 3,3'-dimethyl-4-methoxybenzophenone, 1-bromo-4-chloromethylsulfonylbenzene, 1-[4-(N-2-methoxybenzoylsulfamoyl)phenyl]-3-methylurea (also known as N-(2-methoxybenzoyl)-4-[(methylaminocarbonyl)amino]benzenesulfonamide), 1-[4-(N-2-methoxybenzoylsulfamoyl)phenyl]-3,3-dimethylurea, 1-[4-(N-4,5-dimethylbenzoylsulfamoyl)phenyl]-3-methylurea, 1-[4-(N-naphthylsulfamoyl)-phenyl]-3,3-dimethylurea, N-(2-methoxy-5-methylbenzoyl)-4-(cyclopropylaminocarbonyl)benzenesulfonamide,
and/or one of the following compounds, defined by general formulae,
of the general formula (IIa) 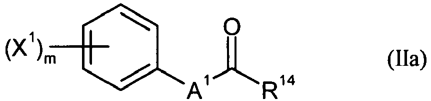 or of the general formula (IIb) 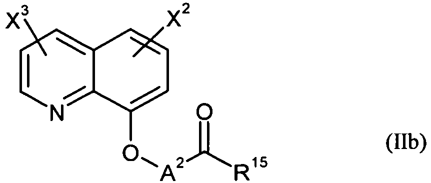 or of the formula (IIc) 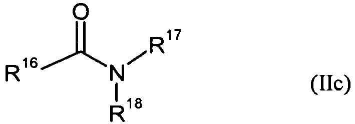 where
m represents a number 0, 1, 2, 3, 4 or 5,
A¹ represents one of the divalent heterocyclic groupings shown below 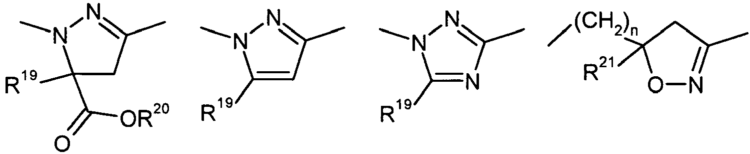
n represents a number 0, 1, 2, 3, 4 or 5,
A² represents optionally C₁-C₄-alkyl- and/or C₁-C₄-alkoxy-carbonyl- and/or C₁-C₄-alkenyloxy-carbonyl-substituted alkanediyl having 1 or 2 carbon atoms,
R¹⁴ represents hydroxyl, mercapto, amino, C₁-C₆-alkoxy, C₁-C₆-alkylthio, C₁-C₆-alkylamino or di(C₁-C₄-alkyl)-amino,
R¹⁵ represents hydroxyl, mercapto, amino, C₁-C₇-alkoxy, C₁-C₆-alkenyloxy, C₁-C₆-alkenyloxy-C₁-C₆-alkoxy, C₁-C₆-alkylthio, C₁-C₆-alkylamino or di(C₁-C₄-alkyl)-amino,
R¹⁶ represents in each case optionally fluorine-, chlorine- and/or bromine-substituted C₁-C₄-alkyl,
R¹⁷ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁸ represents hydrogen, in each case optionally fluorine-, chlorine- and/or bromine-substituted C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, or optionally fluorine-, chlorine- and/or bromine- or C₁-C₄-alkyl-substituted phenyl,
R¹⁷ and R¹⁸ also together represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are attached, form a 5- or 6-membered carbocycle,
R¹⁹ represents hydrogen, cyano, halogen, or represents in each case optionally fluorine-, chlorine- and/or bromine-substituted C₁-C₄-alkyl, C₃-C₆-cycloalkyl or phenyl,
R²⁰ represents hydrogen, in each case optionally hydroxyl-, cyano-, halogen- or C₁-C₄-alkoxy-substituted C₁-C₆-alkyl, C₃-C₆-cycloalkyl or tri-(C₁-C₄-alkyl)-silyl,
R²¹ represents hydrogen, cyano, halogen, or represents in each case optionally fluorine-, chlorine- and/or bromine-substituted C₁-C₄-alkyl, C₃-C₆-cycloalkyl or phenyl,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds, defined by general formulae,
of the general formula (IId) 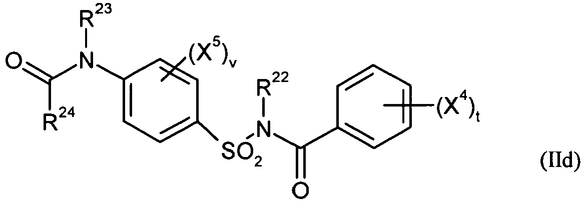 or of the general formula (IIe) 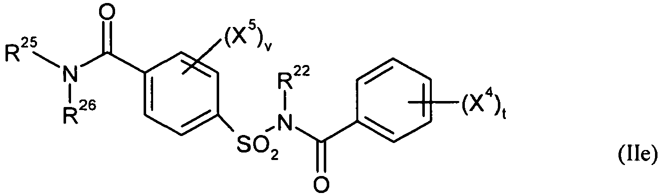 where
t represents a number 0, 1, 2, 3, 4 or 5,
v represents a number 0, 1, 2, 3, 4 or 5,
R²² represents hydrogen or C₁-C₄-alkyl,
R²³ represents hydrogen or C₁-C₄-alkyl,
R²⁴ represents hydrogen, in each case optionally cyano-, halogen- or C₁-C₄-alkoxy-substituted C₁-C₆-alkyl, C₁-C₆-alkoxy, C₁-C₆-alkylthio, C₁-C₆-alkylamino or di(C₁-C₄-alkyl)-amino, or in each case optionally cyano-, halogen- or C₁-C₄-alkyl-substituted C₃-C₆-cycloalkyl, C₃-C₆-cycloalkyloxy, C₃-C₆-cycloalkylthio or C₃-C₆-cycloalkylamino,
R²⁵ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, or optionally cyano-, halogen- or C₁-C₄-alkyl-substituted C₃-C₆-cycloalkyl,
R²⁶ represents hydrogen, optionally cyano-, hydroxyl-, halogen- or C₁-C₄-alkoxy-substituted C₁-C₆-alkyl, in each case optionally cyano- or halogen-substituted C₃-C₆-alkenyl or C₃-C₆-alkynyl, optionally cyano-, halogen- or C₁-C₄-alkyl-substituted C₃-C₆-cycloalkyl, or optionally nitro-, cyano-, halogen-, C₁-C₄-alkyl-, C₁-C₄-haloalkyl-, C₁-C₄-alkoxy- or C₁-C₄-haloalkoxy-substituted phenyl, or together with R²⁵ represents in each case optionally C₁-C₄-alkyl-substituted C₂-C₆-alkanediyl or C₂-C₅-oxaalkanediyl,
X⁴ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulfamoyl, hydroxyl, amino, halogen, C₁-C₄-alkyl, C₁-C₄-haloalkyl, C₁-C₄-alkoxy or C₁-C₄-haloalkoxy.

9. The composition as claimed in claim 8 in which the crop plant compatibility-improving compound is selected from the following group of compounds:
cloquintocet-mexyl, fenchlorazole-ethyl, isoxadifen-ethyl, mefenpyr-diethyl, furilazole, fenclorim, cumyluron, dymron or the compounds IIe-5 or IIe-11.

10. The composition as claimed in claim 8 in which the crop plant compatibility-improving compound is mefenpyr-diethyl.

11. A method for controlling unwanted vegetation which comprises allowing a composition as claimed in claim 8 to act on the plants or their habitats.

12. The use of a composition as claimed in claim 8 for controlling unwanted vegetation.

13. A method for controlling unwanted vegetation which comprises allowing a compound of the formula (I) as claimed in claim 1 and a crop plant compatibility-improving compound as claimed in claim 8 to act, separately in close temporal succession, or as a mixture, on the plants or their habitats.

14. A compound of the formula (II) 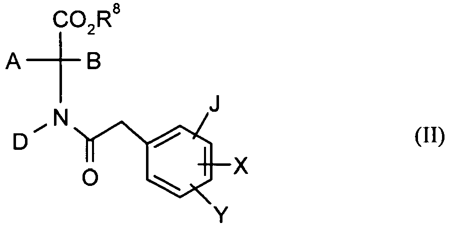 in which
A, B, D, J, X and Y are as defined above and
R⁸ represents alkyl.

15. A compound of the formula (XXV) 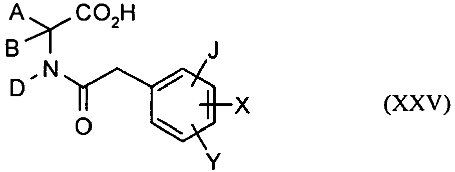 in which
A, B, D, J, X and Y are as defined above.

16. A compound of the formula (XXIV) 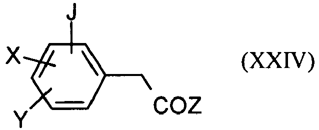 in which
J, X and Y are as defined above,
and Z representd a leaving group introduced by carboxylic acid activation agents, phosphorylating agents, halogenating agents, phosgene or chloroformic esters, with the exception of 2-iodo-5-methylphenyl acetyl chloride.

17. A compound of the formula (XXIX) 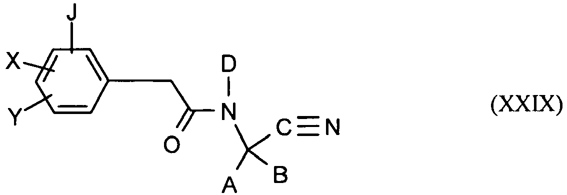 in which
A, B, D, J, X and Y are as defined above.

18. A compound of the formula (XXVII) 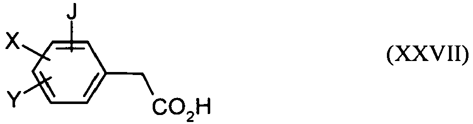 in which
J, X and Y have the meanings given in the table:
| **J** | **X** | **Y** |
|---|---|---|
| 4-1 | 2-C₂H₅ | 6-CH₃ |
| 4-1 | 2-CH₃ | 6-CH₃ |
| 2-1 | 4-Cl | 6-CH₃ |
| 2-1 | 4-Cl | 6-C₂H₅ |
| 4-1 | 2-Cl | 6-CH₃ |
| 4-1 | 2-Cl | 6-C₂H₅ |

19. A compound of the formula (XXXI) 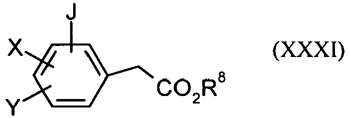 in which
J, X and Y have the meanings given in the table:
| **J** | **X** | **Y** | **R⁸** |
|---|---|---|---|
| 4-1 | 2-C₂H₅ | 6-CH₃ | CH₃ |
| 4-1 | 2-CH₃ | 6-CH₃ | CH₃ |
| 2-1 | 4-Cl | 6-CH₃ | CH₃ |
| 2-1 | 4-Cl | 6-C₂H₅ | CH₃ |
| 4-1 | 2-Cl | 6-CH₃ | CH₃ |
| 4-1 | 2-Cl | 6-C₂H₅ | CH₃ |
and R⁸ represents C₁-C₄-alkyl.

## Revendications

1. Composés de formule (I) 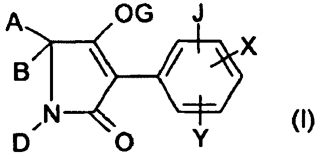 dans laquelle G représente l'hydrogène et J, X, Y, D, A et B ont les significations données dans le tableau :
| J | X | Y | D | A | B |
|---|---|---|---|---|---|
| 4-J | 2-C₂H₅ | 6-CH₃ | H | -(CH₂)₂-CHOCH₃-(CH₂)₂- | |
| 2-J | 4-CH₃ | 6-CH₃ | H | (CH₂)₂-CHCH₃-(CH₂)₂- | |
| 2-J | 4-Cl | 6-CH₃ | H | (CH₂)₂-CHOCH₃- (CH₂)₂ - | |
| 2-J | 5-CH₃ | H | H | -(CH₂)₂-CHOCH₃-(CH₂)₂- | |
| 2-J | 5-CH₃ | H | H | - (CH₂)₂-O- (CH₂)₂- | |
| 2-J | 4-Cl | 6-C₂H₅ | H | - (CH₂)₂-O- (CH₂)₂- | |
| 2-J | 4-Cl | 6-C₂H₅ | H | - (CH₂)₂-CHOCH₃- (CH₂)₂- | |
| 2-J | H | H | H | -(CH₂)₂-CHOCH₃-(CH₂)₂- | |
| 2-J | 4-CH₃ | 6-CH₃ | H | -(CH₂)₂-CHCH₃-(CH₂)₂- | |
| 2-J | 4-Cl | 6-CH₃ | H | - (CH₂)₂-O- (CH₂)₂- | |
| 3-J | 6-CH₃ | H | H | -(CH₂)₂-CHOCH₃-(CH₂)₂- | |
| 4-J | 2-C₂H₅ | 6-CH₃ | H | - (CH₂)₂-O- (CH₂)₂- | |
| 4-J | 2-C₂H₅ | 6-CH₃ | H | CH₃ | CH₃ |
| 4-J | 2-C₂H₅ | 6-CH₃ | H | 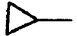 | CH₃ |
| 4-J | 2-C₂H₅ | 6-CH₃ | H | n-C₃H₇ | CH₃ |
| 4-J | 2-C₂H₅ | 6-CH₃ | 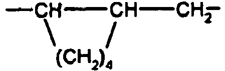 | | H |
| 4-J | 2-C₂H₅ | 6-CH₃ | H | -(CH₂)₅- | |
| 4-J | 2-C₂H₅ | 6-CH₃ | H | - (CH₂)₂-CHCH₃- (CH₂)₂- | |
| 4-J | 2-CH₃ | 6-CH₃ | H | (CH₂)₂-CHOCH₃-(CH₂)₂ | |
| 4-J | 2-CH₃ | 6-CH₃ | H | - (CH₂)₂-O- (CH₂)₂- | |
| 4-J | 2-C₂H₅ | 6-Cl | H | - (CH₂)₂-O- (CH₂)₂- | |
| 4-J | 2-CH₃ | 6-CH₃ | H | -CH₂-CHOC₄H₉-(CH₂)₃- | |
| 4-J | 2-CH₃ | 6-Cl | H | - (CH₂)₂-O- (CH₂)₂- | |
| 4-J | 2-C₂H₅ | 6-CH₃ | H | -CH₂-CHOC₄H₉-(CH₂)₃- | |
| 4-J | 2-C₂H₅ | 6-Cl | H | - (CH₂)₂-CHOCH₃- (CH₂)₂- | |
| 4-J | 2-CH₃ | 6-Cl | H | - (CH₂)₂-CHOCH₃- (CH₂)₂- | |
| 4-J | 2-C₂H₅ | 6-CH₃ | H | i-C₃H₇ | CH₃ |
| 4-J | 2-C₂H₅ | 6-CH₃ | H | i-C₄H₉ | CH₃ |
G représente 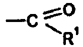 et J, X, Y, D, A, B et R¹ ont les significations données dans le tableau :
| J | X | Y | D | A | B | R¹ |
|---|---|---|---|---|---|---|
| 2-J | 5-CH₃ | H | H | -(CH₂)₂-CHOCH₃-(CH₂)₂- | | i-C₃H₇ |
| 2-J | 4-Cl | 6-C₂H₅ | H | -(CH₂)₂-CHOCH₃-(CH₂)₂- | | H₃C-O-CH₂- |
| 2-J | 4-Cl | 6- C₂H₅ | H | -(CH₂)₂-O-(CH₂)₂- | | H₃C-O-CH₂- |
| 4-J | 2-C₂H₅ | 6-CH₃ | H | -(CH₂)₂-O-(CH₂)₂- | | i-C₃H₇ |
| 4-J | 2-C₂H₅ | 6-CH₃ | H | C₃H₇ | CH₃ | i-C₃H₇ |
| 4-J | 2-CH₃ | 6-CH₃ | H | -CH₂-CH-OC₄H₉- (CH₂)₃- | | H₃C-O-CH₂- |
| 4-J | 2-CH₃ | 6-CH₃ | H | -CH₂-CHOC₄H₉- (CH₂) ₃- | | 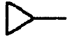 |
| 4-J | 2-CH₃ | 6-CH₃ | H | -CH₂-CHOC₄H₉- (CH₂)₃- | | Cl-CH₂- |
| 4-J | 2-CH₃ | 6-CH₃ | H | -CH₂-CHOC₄H₉- (CH₂)₃- | | 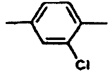 |
| 4-J | 2-C₂H₅ | 6-CH₃ | H | -(CH₂)₂-O-(CH₂)₂- | | H₃C-O-CH₂- |
| 4-J | 2-C₂H₅ | 6-Cl | H | -(CH₂)₂-O-(CH₂)₂- | | H₃C-O-CH₂- |
| 4-J | 2-C₂H₅ | 6-CH₃ | H | -CH₂-CHOC₄H₉- (CH₂) ₃- | | i-C₃H₇ |
| 4-J | 2-C₂H₅ | 6-CH₃ | H | i-C₃H₇ | CH₃ | i-C₃H₇ |
| 4-J | 2-C₂H₅ | 6-CH₃ | H | -CH₂-CHOC₄H₉-(CH₂)₃- | | H₃C-O-CH₂- |
| 4-J | 2-C₂H₅ | 6-CH₃ | H | -(CH₂)₅- | | H₃C-O-CH₂- |
| 4-J | 2-C₂H₅ | 6-CH₃ | H | -(CH₂)₅- | | i-C₃H₇ |
| 4-J | 2-C₂H₅ | 6-CH₃ | H | CH₃ | CH₃ | i-C₃H₇ |
| 4-J | 2-C₂H₅ | 6-CH₃ | H | - (CH₂)₅ - | | t-C₄H₉ |
| 4-J | 2-C₂H₅ | 6-CH₃ | H | 1-C₃H₇ | CH₃ | H₃C-O-CH₂- |
| 4-J | 2-C₂H₅ | 6-CH₃ | H | CH₃ | CH₃ | H₃C-O-CH₂- |
| 4-J | 2-C₂H₅ | 6-CH₃ | H | i-C₃H₇ | CH₃ | i-C₃H₇ |
| 2-J | 5-CH₃ | H | H | - (CH₂)₂-CHOCH₃-(CH₂)₂- | | 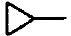 |
G représente 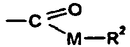 et J, X, Y, D, A, B, M et R² ont les significations données dans le tableau :
| J | X | Y | D | A | B | M | R² |
|---|---|---|---|---|---|---|---|
| 2-J | 5-CH₃ | H | H | - (CH₂)₂-CHOCH₃-(-CH₂)₂- | | O | C₆H₅-CH₂- |
| 2-J | 4-Cl | 6-CH₃ | H | - (CH₂)-CHOCH₃-(CH₂)₂- | | O | C₂H₅ |
| 2-J | 5-CH₃ | H | H | - (CH₂)₂-CHOCH₃-(CH₂)₂- | | O | C₂H₅ |
| 2-J | 4-Cl | 6-C₂H₅ | H | -(CH₂)₂-O-(CH₂)₂- | | O | C₂H₅ |
| 2-J | 4-Cl | 6- C₂H₅ | H | - (CH₂)₂-CHOCH₃-(CH₂)₂- | | O | C₂H₅ |
| 3-J | 6-CH₃ | H | H | - (CH₂)₂-CHOCH₃-(CH₂)₂- | | O | C₂H₅ |
| 4-J | 2-CH₃ | 6-CH₃ | H | - (CH₂)₂-CHOCH₃-(CH₂)₂- | | O | C₂H₅ |
| 4-J | 2-CH₃ | 6-Cl | H | -(CH₂)₂-O-(CH₂)₂- | | O | C₂H₅ |
| 4-J | 2-C₂H₅ | 6-CH₃ | H | n-C₃H₇ | CH₃ | O | C₂H₅ |
| 4-J | 2-C₂H₅ | 6-CH₃ | H | 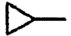 | CH₃ | O | C₂H₅ |
| 4-J | 2-C₂H₅ | 6-CH₃ | H | -(CH₂)₂-O-(CH₂)₂- | | O | C₂H₅ |
| 4-J | 2-C₂H₅ | 6-Cl | H | -(CH₂)₂-O-(CH₂)₂- | | O | C₂H₅ |
| 4-J | 2-CH₃ | 6-CH₃ | H | -CH₂-CHOC₄H₉-(CH₂)₃- | | O | C₆H₅-CH₂- |
| 4-J | 2-CH₃ | 6-CH₃ | H | -CH₂-CHOC₄H₉-(CH₂)₃- | | O | CH₂=CH-CH₂- |
| 4-J | 2-CH₃ | 6-CH₃ | H | -CH₂-CHOC₄H₉-(CH₂)₃- | | O | C₂H₅ |
| 4-J | 2-C₂H₅ | 6-CH₃ | H | -(CH₂)₅- | | O | C₂H₅ |
| 4-J | 2-C₂H₅ | 6-CH₃ | H | CH₃ | CH₃ | O | C₂H₅ |
| 4-J | 2-C₂H₅ | 6-CH₃ | H | i-C₄H₉ | CH₃ | O | C₂H₅ |
| 4-J | 2-CH₃ | 6-CH₃ | H | -(CH₂)₂-O-(CH₂)₂- | | O | C₂H₅ |
| 4-J | 2-C₂H₅ | 6-CH₃ | H | -(CH₂)₂-CHOCH₃-(CH₂)₂- | | O | C₂H₅ |

2. Procédé de fabrication de composés de formule (I) selon la revendication 1, **caractérisé en ce que**, pour l'obtention de
A) des composés de formule (I-1-a) 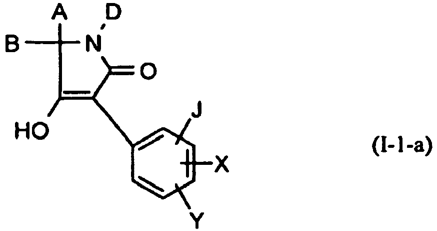 dans laquelle
A, B, D, J, X et Y ont les significations données précédemment,
des composés de formule (II) 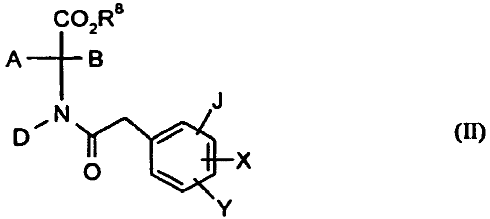 dans laquelle
A, B, D, J, X et Y ont les significations données précédemment,
et
R⁸ représente alkyle,
sont condensés intramoléculairement en présence d'un diluant et en présence d'une base,
(I) des composés de la formule (I-1-b) représentée précédemment, dans laquelle A, B, D, J, R¹, X et Y ont les significations données précédemment, des composés de la formule (I-1-a) représentée précédemment, dans laquelle A, B, D, J, X et Y ont les significations données précédemment, sont mis en réaction avec
(α) des halogénures d'acides de formule (XIII) 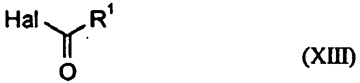 dans laquelle
R¹ a la signification donnée précédemment et
Hal représente un halogène
ou
(β) des anhydrides d'acides carboxyliques de formule (XIV)
R¹-CO-O-CO-R¹ (XIV)
dans laquelle
R¹ a la signification donnée précédemment,
éventuellement en présence d'un diluant et éventuellement en présence d'un liant acide,
(J) des composés de la formule (I-1-c) représentée précédemment, dans laquelle A, B, D, J, R², M, X et Y ont les significations données précédemment et L représente l'oxygène, des composés de la formule (I-1-a) représentée précédemment, dans laquelle A, B, D, J, X et Y ont les significations données précédemment, sont mis en réaction avec
des esters de l'acide chloroformique ou des thioesters de l'acide chloroformique de formule (XV)
R²-M-CO-Cl (XV)
dans laquelle
R² et M ont les significations données précédemment,
éventuellement en présence d'un diluant et éventuellement en présence d'un liant acide,
(P) des composés de la formule (I-1-a) représentée précédemment, dans laquelle A, B, D, J, X et Y ont les significations données précédemment, des composés de formule (I-1-a'), dans laquelle A, B, D, X et Y ont les significations données précédemment et W' représente le brome 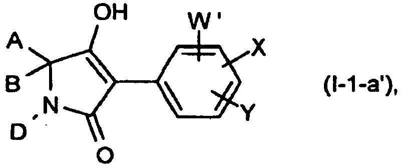
α) sont mis en réaction avec des iodures métalliques éventuellement en présence d'un diluant, d'un sel de Cu (I) et d'une base, ou
β) réalisent un échange halogène-métal avec des organyles métalliques et l'anion formé est désactivé avec des réactifs d'iodation.

3. Utilisation de composés de formule (I) selon la revendication 1 pour la fabrication d'agents de lutte contre les animaux nuisibles et/ou d'herbicides.

4. Agents de lutte contre les animaux nuisibles et/ou herbicides, **caractérisés par** une teneur en au moins un composé de formule (I) selon la revendication 1.

5. Procédé de lutte contre les animaux nuisibles et/ou la végétation indésirable, **caractérisé en ce que** des composés de formule (I) selon la revendication 1 sont laissés agir sur les animaux nuisibles et/ou leur habitat, à l'exception des procédés thérapeutiques.

6. Utilisation de composés de formule (I) selon la revendication 1 pour la lutte contre les animaux nuisibles et/ou la végétation indésirable, à l'exception de l'utilisation dans des procédés thérapeutiques.

7. Procédé de fabrication d'agents de lutte contre les animaux nuisibles et/ou d'herbicides, **caractérisé en ce que** des composés de formule (I) selon la revendication 1 sont mélangés avec des diluants et/ou des tensioactifs.

8. Agent contenant une teneur efficace en une combinaison d'agents actifs comprenant en tant que composants :
(a') au moins un céto-énol cyclique substitué de formule (I), dans laquelle A, B, D, G, J, X et Y ont la signification donnée précédemment,
et
(b') au moins un composé améliorant la compatibilité avec les plantes cultivées du groupe suivant de composés :
le 4-dichloroacétyl-1-oxa-4-aza-spiro[4.5]-décane (AD-67, MON-4660), la 1-dichloroacétyl-hexa-hydro-3,3,8a-triméthylpyrrolo[1,2-a]-pyrimidin-6(2H)-one (dicyclonon, BAS-145138), la 4-dichloroacétyl-3,4-dihydro-3-méthyl-2H-1,4-benzoxazine (benoxacor), l'ester 1-méthyl-hexylique de l'acide 5-chloroquinoline-8-oxy-acétique (cloquintocet-mexyl - voir également composés apparentés dans EP-A-86750, EP-A-94349, EP-A-191736, EP-A-492366), la 3-(2-chlorobenzyl)-1-(1-méthyl-1-phényl-éthyl)-urée (cumyluron), l'α-(cyanométhoximino)-phénylacétonitrile (cyométrinil), l'acide 2,4-dichloro-phénoxyacétique (2,4-D), l'acide 4-(2,4-dichloro-phénoxy)-butyrique (2,4-DB), la 1-(1-méthyl-1-phényl-éthyl)-3-(4-méthylphényl)-urée (daimuron, dymron), l'acide 3,6-dichloro-2-méthoxy-benzoïque (dicamba), l'ester S-1-méthyl-1-phényl-éthylique de l'acide pipéridine-1-thiocarboxylique (dimépipérate), le 2,2-dichloro-N-(2-oxo-2-(2-propénylamino)-éthyl)-N-(2-propényl)-acétamide (DKA-24), le 2,2-dichloro-N,N-di-2-propényl-acétamide (dichlormid), la 4,6-dichloro-2-phényl-pyrimidine (fenclorim), l'ester éthylique de l'acide 1-(2,4-dichloro-phényl)-5-trichlorométhyl-1H-1,2,4-triazol-3-carboxylique (fenchlorazole-éthyle - voir également composés apparentés dans EP-A-174562 et EP-A-346620), l'ester phénylméthylique de l'acide 2-chloro-4-trifluorométhyl-thiazol-5-carboxylique (flurazole), le 4-chloro-N-(1,3-di-oxolan-2-yl-méthoxy)-α-trifluoroacétophénonoxime (fluxofenim), la 3-dichloroacétyl-5-(2-furanyl)-2,2-diméthyl-oxazolidine (furilazole, MON-13900), le carboxylate d'éthyl-4,5-dihydro-5,5-diphényl-3-isoxazole (isoxadifen-éthyle - voir également composés apparentés dans WO-A-95/07897), le 3,6-dichloro-2-méthoxybenzoate de 1-(éthoxycarbonyl)-éthyle (lactidichlor), l'acide (4-chloro-o-tolyloxy)-acétique (MCPA), l'acide 2-(4-chloro-o-tolyloxy)-propionique (méco-prop), le 3,5-dicarboxylate de diéthyl-1-(2,4-dichloro-phényl)-4,5-dihydro-5-méthyl-1H-pyrazole (méfenpyr-diéthyle - voir également composés apparentés dans WO-A-91/07874), le 2-dichlorométhyl-2-méthyl-1,3-dioxolane (MG-191), le 4-carbodithioate de 2-propényl-1-oxa-4-azaspiro[4.5]décane (MG-838), l'anhydride de l'acide 1,8-naphtoïque, l'α-(1,3-dioxolan-2-yl-méthoximino)-phénylacétonitrile (oxabétrinil), le 2,2-dichloro-N-(1,3-dioxolan-2-yl-méthyl)-N-(2-propényl)-acétamide (PPG-1292), la 3-dichloroacétyl-2,2-diméthyl-oxazolidine (R-28725), la 3-dichloroacétyl-2,2,5-triméthyl-oxazolidine (R-29148), l'acide 4-(4-chloro-o-tolyl)-butyrique, l'acide 4-(4-chloro-phénoxy)-butyrique, l'acide diphénylméthoxyacétique, l'ester méthylique de l'acide diphénylméthoxyacétique, l'ester éthylique de l'acide diphénylméthoxyacétique, l'ester méthylique de l'acide 1-(2-chloro-phényl)-5-phényl-1H-pyrazol-3-carboxylique, l'ester éthylique de l'acide 1-(2,4-dichloro-phényl)-5-méthyl-1H-pyrazol-3-carboxylique, l'ester éthylique de l'acide 1-(2,4-dichloro-phényl)-5-isopropyl-1H-pyrazol-3-carboxylique, l'ester éthylique de l'acide 1-(2,4-dichloro-phényl)-5-(1,1-diméthyl-éthyl)-1H-pyrazol-3-carboxylique, l'ester éthylique de l'acide 1-(2,4-dichloro-phényl)-5-phényl-1H-pyrazol-3-carboxylique (voir également composés apparentés dans EP-A-269806 et EP-A-333131), l'ester éthylique de l'acide 5-(2,4-dichloro-benzyl)-2-isoxazoline-3-carboxylique, l'ester éthylique de l'acide 5-phényl-2-isoxazoline-3-carboxylique, l'ester éthylique de l'acide 5-(4-fluoro-phényl)-5-phényl-2-isoxazoline-3-carboxylique (voir également composés apparentés dans WO-A-91/08202), l'ester 1,3-diméthyl-but-1-ylique de l'acide 5-chloro-quinoline-8-oxy-acétique, l'ester 4-allyloxy-butylique de l'acide 5-chloro-quinoline-8-oxy-acétique, l'ester 1-allyloxy-prop-2-ylique de l'acide 5-chloro-quinoline-8-oxy-acétique, l'ester méthylique de l'acide 5-chloro-quinoxaline-8-oxy-acétique, l'ester éthylique de l'acide 5-chloro-quinoline-8-oxy-acétique, l'ester allylique de l'acide 5-chloro-quinoxaline-8-oxy-acétique, l'ester 2-oxoprop-1-ylique de l'acide 5-chloro-quinoline-8-oxy-acétique, l'ester diéthylique de l'acide 5-chloro-quinoline-8-oxy-malonique, l'ester diallylique de l'acide 5-chloro-quinoxaline-8-oxy-malonique, l'ester diéthylique de l'acide 5-chloroquinoline-8-oxy-malonique (voir également composés apparentés dans EP-A-582198), l'acide 4-carboxychroman-4-yl-acétique (AC-304415, voir EP-A-613618), l'acide 4-chloro-phénoxy-acétique, la 3,3'-diméthyl-4-méthoxy-benzophénone, le 1-bromo-4-chlorométhylsulfonyl-benzène, la 1-[4-(N-2-méthoxybenzoylsulfamoyl)-phényl]-3-méthyl-urée (alias N-(2-méthoxy-benzoyl)-4-[(méthylamino-carbonyl)-amino]-benzènesulfonamide), la 1-[4-(N-2-méthoxybenzoylsulfamoyl)-phényl]-3,3-diméthyl-urée, la 1-[4-(N-4,5-diméthylbenzoyl-sulfamoyl)-phényl]-3-méthyl-urée, la 1-[4-(N-naphtylsulfamoyl)-phényl]-3,3-diméthyl-urée, le N-(2-méthoxy-5-méthyl-benzoyl)-4-(cyclopropylaminocarbonyl)-benzènesulfonamide,
et/ou un des composés suivants définis par les formules générales
la formule générale (IIa) 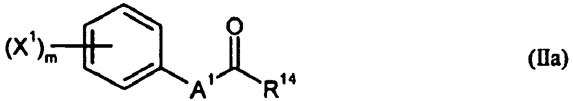
ou la formule générale (IIb) 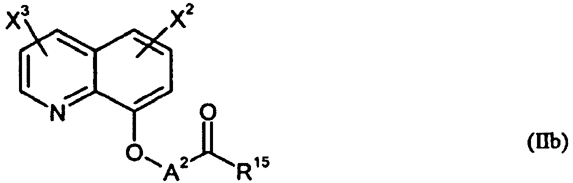
ou la formule (IIc) 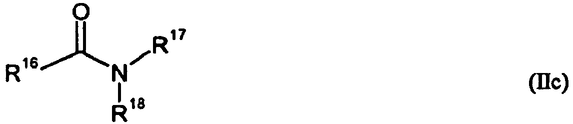
dans lesquelles
m représente un nombre 0, 1, 2, 3, 4 ou 5,
A¹ représente un des groupes hétérocycliques bivalents représentés ci-dessous 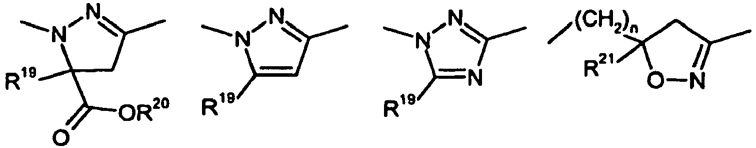
n représente un nombre 0, 1, 2, 3, 4 ou 5,
A² représente un alcanediyle de 1 ou 2 atomes de carbone éventuellement substitué par alkyle en C₁-C₄ et/ou alcoxycarbonyle en C₁-C₄ et/ou alcényloxycarbonyle en C₁-C₄,
R¹⁴ représente hydroxy, mercapto, amino, alcoxy en C₁-C₆, alkylthio en C₁-C₆, alkylamino en C₁-C₆ ou di(alkyl en C₁-C₄) -amino,
R¹⁵ représente hydroxy, mercapto, amino, alcoxy en C₁-C₇, alcényloxy en C₁-C₆, alcényloxy en C₁-C₆-alcoxy en C₁-C₆, alkylthio en C₁-C₆, alkylamino en C₁-C₆ ou di(alkyl en C₁-C₄)-amino,
R¹⁶ représente respectivement alkyle en C₁-C₄ éventuellement substitué par fluor, chlore et/ou brome,
R¹⁷ représente hydrogène, alkyle en C₁-C₆, alcényle en C₂-C₆ ou alcynyle en C₂-C₆ chacun éventuellement substitué par fluor, chlore et/ou brome, alcoxy en C₁-C₄-alkyle en C₁-C₄, dioxolanyl-alkyle en C₁-C₄, furyle, furyl-alkyle en C₁-C₄, thiényle, thiazolyle, pipéridinyle ou phényle éventuellement substitué par fluor, chlore et/ou brome ou alkyle en C₁-C₄,
R¹⁸ représente hydrogène, alkyle en C₁-C₆, alcényle en C₂-C₆ ou alcynyle en C₂-C₆ éventuellement substitué par fluor, chlore et/ou brome, alcoxy en C₁-C₄-alkyle en C₁-C₄, dioxolanyl-alkyle en C₁-C₄, furyle, furyl-alkyle en C₁-C₄, thiényle, thiazolyle, pipéridinyle ou phényle éventuellement substitué par fluor, chlore et/ou brome ou alkyle en C₁-C₄,
R¹⁷ et R¹⁸ représentent également ensemble un alcanediyle en C₃-C₆ ou un oxaalcanediyle en C₂-C₅ chacun éventuellement substitué par alkyle en C₁-C₄, phényle, furyle, un cycle benzène annelé ou par deux substituants qui forment ensemble avec l'atome C auquel ils sont reliés un carbocycle de 5 ou 6 éléments,
R¹⁹ représente hydrogène, cyano, halogène, ou alkyle en C₁-C₄, cycloalkyle en C₃-C₆ ou phényle chacun éventuellement substitué par fluor, chlore et/ou brome,
R²⁰ représente hydrogène, alkyle en C₁-C₆ éventuellement substitué par hydroxy, cyano, halogène ou alcoxy en C₁-C₄, cycloalkyle en C₃-C₆ ou tri(alkyle en C₁-C₄)-silyle,
R²¹ représente hydrogène, cyano, halogène, ou alkyle en C₁-C₄, cycloalkyle en C₃-C₆ ou phényle chacun éventuellement substitué par fluor, chlore et/ou brome,
X¹ représente nitro, cyano, halogène, alkyle en C₁-C₄, halogénoalkyle en C₁-C₄, alcoxy en C₁-C₄ ou halogénoalcoxy en C₁-C₄,
X² représente hydrogène, cyano, nitro, halogène, alkyle en C₁-C₄, halogénoalkyle en C₁-C₄, alcoxy en C₁-C₄ ou halogénoalcoxy en C₁-C₄,
X³ représente hydrogène, cyano, nitro, halogène, alkyle en C₁-C₄, halogénoalkyle en C₁-C₄, alcoxy en C₁-C₄ ou halogénoalcoxy en C₁-C₄,
et/ou les composés suivants définis par les formules générales
la formule générale (IId) 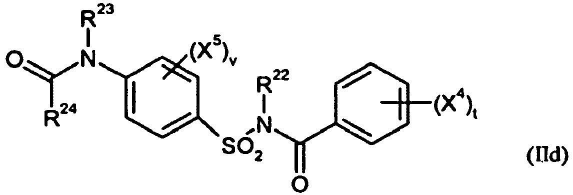 ou la formule générale (IIe) 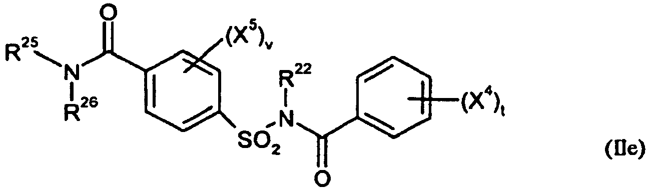 dans lesquelles
t représente un nombre 0, 1, 2, 3, 4 ou 5,
v représente un nombre 0, 1, 2, 3, 4 ou 5,
R²² représente hydrogène ou alkyle en C₁-C₄,
R²³ représente hydrogène ou alkyle en C₁-C₄,
R²⁴ représente hydrogène, alkyle en C₁-C₆, alcoxy en C₁-C₆, alkylthio en C₁-C₆, alkylamino en C₁-C₆ ou di(alkyle en C₁-C₄)amino chacun éventuellement substitué par cyano, halogène ou alcoxy en C₁-C₄, ou cycloalkyle en C₃-C₆, cycloalkyloxy en C₃-C₆, cycloalkylthio en C₃-C₆ ou cycloalkylamino en C₃-C₆ chacun éventuellement substitué par cyano, halogène ou alkyle en C₁-C₄,
R²⁵ représente hydrogène, alkyle en C₁-C₆ éventuellement substitué par cyano, hydroxy, halogène ou alcoxy en C₁-C₄, alcényle en C₃-C₆ ou alcynyle en C₃-C₆ chacun éventuellement substitué par cyano ou halogène, ou cycloalkyle en C₃-C₆ éventuellement substitué par cyano, halogène ou alkyle en C₁-C₄,
R²⁶ représente hydrogène, alkyle en C₁-C₆ éventuellement substitué par cyano, hydroxy, halogène ou alcoxy en C₁-C₄, alcényle en C₃-C₆ ou alcynyle en C₃-C₆ chacun éventuellement substitué par cyano ou halogène, cycloalkyle en C₃-C₆ éventuellement substitué par cyano, halogène ou alkyle en C₁-C₄, ou phényle éventuellement substitué par nitro, cyano, halogène, alkyle en C₁-C₄, halogénoalkyle en C₁-C₄, alcoxy en C₁-C₄ ou halogénoalcoxy en C₁-C₄, ou représente ensemble avec R²⁵ un alcanediyle en C₂-C₆ ou oxaalcanediyle en C₂-C₅ chacun éventuellement substitué par alkyle en C₁-C₄,
X⁴ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄, et
X⁵ représente nitro, cyano, carboxy, carbamoyle, formyle, sulfamoyle, hydroxy, amino, halogène, alkyle en C₁-C₄, halogénoalkyle en C₁-C₄, alcoxy en C₁-C₄ ou halogénoalcoxy en C₁-C₄.

9. Agent selon la revendication 8, dans lequel le composé améliorant la compatibilité avec les plantes cultivées est choisi dans le groupe suivant de composés :
cloquintocet-mexyl, fenchlorazole-éthyle, isoxadifen-éthyle, méfenpyr-diéthyle, furilazole, fenclorim, cumyluron, dymron ou les composés IIe-5 ou IIe-11.

10. Agent selon la revendication 8, dans lequel le composé améliorant la compatibilité avec les plantes cultivées est le méfenpyr-diéthyle.

11. Procédé de lutte contre la végétation indésirable, **caractérisé en ce qu'**un agent selon la revendication 8 est laissé agir sur les plantes ou leur environnement.

12. Utilisation d'un agent selon la revendication 8 pour lutter contre la végétation indésirable.

13. Procédé de lutte contre la végétation indésirable, **caractérisé en ce qu'**un composé de formule (I) selon la revendication 1 et un composé améliorant la compatibilité avec les plantes cultivées selon la revendication 8 sont laissés agir en succession temporelle proche ou en mélange sur les plantes ou leur environnement.

14. Composés de formule (II) 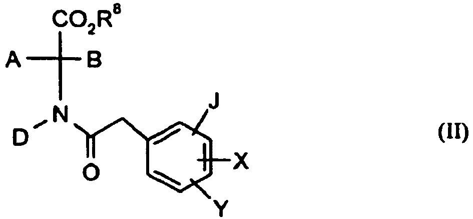 dans laquelle
A, B, D, J, X et Y ont les significations données précédemment,
et
R⁸ représente alkyle.

15. Composés de formule (XXV) 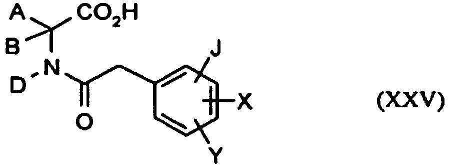 dans laquelle
A, B, D, J, X et Y ont les significations données précédemment.

16. Composés de formule (XXIV) 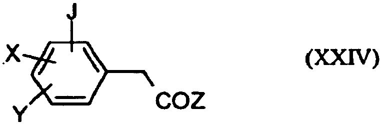 dans laquelle
J, X et Y ont les significations données précédemment, et Z représente un groupe partant introduit par des réactifs d'activation d'acides carboxyliques, des réactifs de phosphorylation, des agents d'halogénation, du phosgène ou des esters de l'acide chloroformique,
à l'exception du chlorure de l'acide 2-iodo-5-méthylphénylacétique.

17. Composés de formule (XXIX) 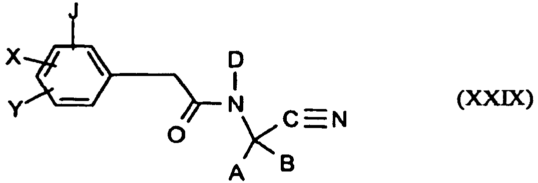 dans laquelle
A, B, D, J, X et Y ont les significations données précédemment.

18. Composés de formule (XXVII) 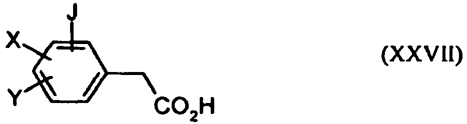 dans laquelle
J, X et Y ont les significations données dans le tableau :
| J | X | Y |
|---|---|---|
| 4-J | 2-C₂H₅ | 6-CH₃ |
| 4-J | 2-CH₃ | 6-CH₃ |
| 2-J | 4-Cl | 6-CH₃ |
| 2-J | 4-Cl | 6-C₂H₅ |
| 4-J | 2-Cl | 6-CH₃ |
| 4-J | 2-Cl | 6-C₂H₅ |

19. Composés de formule (XXXI) 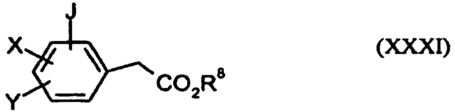 dans laquelle
J, X et Y ont les significations données dans le tableau :
| J | X | Y | R⁸ |
|---|---|---|---|
| 4-J | 2-C₂H₅ | 6-CH₃ | CH₃ |
| 4-J | 2-CH₃ | 6-CH₃ | CH₃ |
| 2-J | 4-Cl | 6-CH₃ | CH₃ |
| 2-J | 4-Cl | 6-C₂H₅ | CH₃ |
| 4-J | 2-Cl | 6-CH₃ | CH₃ |
| 4-J | 2-Cl | 6-C₂H₅ | CH₃ |
et R⁸ représente alkyle en C₁-C₄.
